(19) Europäisches Patentamt
European Patent Office
Office européen des brevets

(11) EP 3 845 669 A1

(12) EUROPEAN PATENT APPLICATION

(43) Date of publication:
**07.07.2021 Bulletin 2021/27**

(51) Int Cl.:
***C12Q 1/6886*** *(2018.01)*

(21) Application number: **21152410.3**

(22) Date of filing: **07.07.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2016 PCT/JP2016/070330**
**19.01.2017 JP 2017007725**

(62) Document number(s) of the earlier application(s) in accordance with Art. 76 EPC:
**17824343.2 / 3 483 282**

(71) Applicants:
- **Hanumat Co., Ltd.**
  **Kobe-shi, Hyogo 657-0025 (JP)**
- **EA Pharma Co., Ltd.**
  **Tokyo 104-0042 (JP)**

(72) Inventors:
- **KUSUNOKI, Masato**
  **Kobe-shi, Hyogo 657-0025 (JP)**
- **TOIYAMA, Yuji**
  **Tsu-shi, Mie 514-8507 (JP)**
- **TANAKA, Koji**
  **Tsu-shi, Mie 514-8507 (JP)**
- **ARAKI, Toshimitsu**
  **Tsu-shi, Mie 514-8507 (JP)**
- **MITSUI, Akira**
  **Kawasaki-shi, Kanagawa 210-8681 (JP)**
- **TAKEHANA, Kenji**
  **Chuo-ku, Tokyo 104-0042 (JP)**
- **UMEZAWA, Tsutomu**
  **Chuo-ku, Tokyo 104-0042 (JP)**

(74) Representative: **Mewburn Ellis LLP**
**Aurora Building**
**Counterslip**
**Bristol BS1 6BX (GB)**

Remarks:
•This application was filed on 19-01-2021 as a divisional application to the application mentioned under INID code 62.
•Claims filed after the date of filing of the application / after the date of receipt of the divisional application (Rule 68(4) EPC).

# (54) METHOD FOR JUDGING ONSET POSSIBILITY OF LARGE BOWEL CANCER

(57) The present invention provides a method for determining the likelihood of colorectal cancer development in a human ulcerative colitis patient, the method including: a measurement step of measuring methylation rates of one or more CpG sites present in specific differentially methylated regions in DNA recovered from a biological sample collected from the human ulcerative colitis patient; and a determination step of determining the likelihood of colorectal cancer development in the human ulcerative colitis patient based on average methylation rates of the differentially methylated regions which are calculated based on the methylation rates measured in the measurement step and a preset reference value or a preset multivariate discrimination expression, in which the reference value is a value for identifying a cancerous ulcerative colitis patient and a non-cancerous ulcerative colitis patient, which is set for the methylation rate of each differentially methylated region, and the multivariate discrimination expression includes, as variables, average methylation rates of one or more differentially methylated regions among the specific differentially methylated regions.

EP 3 845 669 A1

**Description**

Technical Field

The present invention relates to a method for determining the likelihood of colorectal cancer development in a human ulcerative colitis patient, and a kit for collecting a rectal mucosa specimen to be subjected to the method.

Priority is claimed on PCT International Application No. PCT/JP2016/70330, filed on July 8, 2016, and Japanese Patent Application No. 2017-007725, filed on January 19, 2017, the contents of which are incorporated herein by reference.

Background Art

Ulcerative colitis is an inflammatory bowel disease of unknown origin which can cause ulcers and erosion mainly in large intestinal mucosa. It is very difficult to achieve a complete cure therefor, and remission and recurrence repeatedly occur. Symptoms include local symptoms of the large intestine such as diarrhea, abdominal pain, and mucous and bloody stool, and systemic symptoms such as fever, vomiting, tachycardia, and anemia. Ulcerative colitis patients are more likely to develop colorectal cancer. For this reason, early detection and treatment of colorectal cancer are important in ulcerative colitis patients.

In general, an examination for early detection of colorectal cancer is usually performed by an endoscopic examination. However, detecting colorectal cancer at an early stage by visual recognition depends largely on an operator's skill and it is generally difficult to do so. Particularly in ulcerative colitis patients, it is very difficult to detect colorectal cancer at an early stage due to inherent severe inflammation of the intestinal mucosa. In addition, the endoscopic examination has problems of being highly invasive and of also being a heavy burden on a patient.

On the other hand, PTL 1 reports that in ulcerative colitis patients, a methylation rate of five miRNA genes of miR-1, miR-9, miR-124, miR-137, and miR-34b/c in tumorous tissue is significantly higher than non-tumorous ulcerative colitis tissue, and therefore the methylation rate of the five miRNA genes in a biological sample collected from colonic mucosa which is a non-cancerous part can be used as a marker for colorectal cancer development in ulcerative colitis patients.

Citation List

Patent Literature

[PTL 1] PCT International Publication No. WO 2014/151551

Summary of Invention

Problems to be Solved by the Invention

An object of the present invention is to provide a method for determining the likelihood of colorectal cancer development in a human ulcerative colitis patient by a method which is less invasive than an endoscopic examination and places a less burden on a patient, and a kit for collecting a rectal mucosa specimen to be subjected to the method.

Means to Solve the Problems

As a result of intensive studies to solve the above problems, the present inventors comprehensively investigated methylation rates of CpG (cytosine-phosphodiester bond-guanine) sites in genomic DNAs of ulcerative colitis patients, and found 80 CpG sites with markedly different methylation rates in patients who had developed colorectal cancer and patients who had not developed colorectal cancer. In addition, the present inventors separately found 112 differentially methylated regions (referred to as "DMR" in some cases), and completed the present invention.

That is, the present invention provides the following [1] to [34], namely a method for determining the likelihood of colorectal cancer development, a marker for analyzing a DNA methylation rate, and a kit for collecting large intestinal mucosa.

[1] A method for determining the likelihood of colorectal cancer development in a human ulcerative colitis patient, the method including:

a measurement step of measuring a methylation rate of one or more CpG sites present in the respective differentially methylated regions represented by differentially methylated region numbers 1 to 112 listed in Tables 1 to 4, in DNA recovered from a biological sample collected from the human ulcerative colitis patient; and
a determination step of determining the likelihood of colorectal cancer development in the human ulcerative colitis patient, based on average methylation rates of the differentially methylated regions which are calculated based on the methylation rates measured in the measurement step and a preset reference value or a preset multivariate discrimination expression,
in which the average methylation rate of the differentially methylated region is an average value of methylation rates of all CpG sites, for which the methylation rate is measured in the measurement step, among the CpG sites in the differentially methylated region,
the reference value is a value for identifying a cancerous ulcerative colitis patient and a non-cancerous ulcerative colitis patient, which is set for the average methylation rate of each differentially methylated region, and
the multivariate discrimination expression includes, as variables, average methylation rates of one or more differentially methylated regions among the differentially methylated regions represented by the differentially methylated region numbers 1 to 112.

[Table 1]

| DMR no. | Gene Symbol | Ensembl ID | Chromosome no. | DMR start | DMR end | Width | ± |
|---|---|---|---|---|---|---|---|
| 1 | MTMR11 | ENSG00000014914 | 1 | 149907598 | 149909051 | 1454 | − |
| 2 | SIX2 | ENSG00000170577 | 2 | 45233485 | 45233784 | 300 | + |
| 3 | COL3A1 | ENSG00000168542 | 2 | 189838986 | 189839961 | 976 | − |
| 4 | ARL14 | ENSG00000179674 | 3 | 160393670 | 160397766 | 4097 | − |
| 5 | S100P | ENSG00000163993 | 4 | 6695204 | 6695433 | 230 | − |
| 6 | VTRNA1-2 | ENSG00000202111 | 5 | 140098089 | 140099064 | 976 | − |
| 7 | PDGFA | ENSG00000197461 | 7 | 544037 | 545463 | 1427 | − |
| 8 | C9orf152 | ENSG00000188959 | 9 | 112970134 | 112970675 | 542 | − |
| 9 | TMPRSS4 | ENSG00000137648 | 11 | 117947606 | 117948147 | 542 | − |
| 10 | CEP112 | ENSG00000154240 | 17 | 63623628 | 63625636 | 2009 | − |
| 11 | ZMYND8 | ENSG00000101040 | 20 | 45946538 | 45947713 | 1176 | − |
| 12 | CASZ1 | ENSG00000130940 | 1 | 10839179 | 10839844 | 666 | − |
| 13 | KAZN | ENSG00000189337 | 1 | 15271343 | 15272595 | 1253 | − |
| 14 | RNF186; RP11-91K11.2 | ENSG00000178828; ENSG00000235434 | 1 | 20138780 | 20142876 | 4097 | − |
| 15 | SELENBP1 | ENSG00000143416 | 1 | 151344319 | 151345394 | 1076 | − |
| 16 | C1orf106 | ENSG00000163362 | 1 | 200862559 | 200865970 | 3412 | − |
| 17 | C4BPB | ENSG00000123843 | 1 | 207262158 | 207262699 | 542 | − |
| 18 |  | ENSG00000224037 | 1 | 234851858 | 234853830 | 1973 | − |
| 19 | MALL | ENSG00000144063 | 2 | 110872470 | 110872878 | 409 | − |
| 20 | NOSTRIN | ENSG00000163072 | 2 | 169658610 | 169659453 | 844 | − |
| 21 | SATB2; SATB2-AS1 | ENSG00000119042; ENSG00000225953 | 2 | 200334655 | 200335051 | 397 | + |
| 22 | HDAC4 | ENSG00000068024 | 2 | 240174125 | 240175146 | 1022 | + |
| 23 | HRH1 | ENSG00000196639 | 3 | 11266750 | 11267368 | 619 | − |
| 24 | ATP13A4-AS1; ATP13A4 | ENSG00000225473; ENSG00000127249 | 3 | 193272384 | 193272925 | 542 | − |
| 25 | ARHGAP24 | ENSG00000138639 | 4 | 86748456 | 86749527 | 1072 | − |
| 26 | RP11-335O4.3; TRIM2 | ENSG00000235872; ENSG00000109654 | 4 | 154125233 | 154126208 | 976 | − |
| 27 | PDLIM3 | ENSG00000154553 | 4 | 186425209 | 186426241 | 1033 | − |
| 28 | FAM134B | ENSG00000154153 | 5 | 16508433 | 16509611 | 1179 | − |
| 29 |  | ENSG00000222366 | 6 | 28944243 | 28946445 | 2203 | + |
| 30 | OR2I1P | ENSG00000237988 | 6 | 29520800 | 29521885 | 1086 | + |

[Table 2]

| DMR no. | Gene Symbol | Ensembl ID | Chromosome no. | DMR start | DMR end | Width | ± |
|---|---|---|---|---|---|---|---|
| 31 | FRK | ENSG00000111816 | 6 | 116381823 | 116382002 | 180 | − |
| 32 | IYD | ENSG00000009765 | 6 | 150689855 | 150690414 | 560 | − |
| 33 | SNX9 | ENSG00000130340 | 6 | 158374746 | 158376752 | 2007 | − |
| 34 | HOXA3 | ENSG00000243394; ENSG00000105997; ENSG00000240154 | 7 | 27154541 | 27155088 | 548 | − |
| 35 | DIP2C; PRR26 | ENSG00000151240; ENSG00000180525 | 10 | 695357 | 696843 | 1487 | − |
| 36 | TNKS1BP1 | ENSG00000149115 | 11 | 57087702 | 57091030 | 3329 | − |
| 37 | LRP5 | ENSG00000162337 | 11 | 68173589 | 68174773 | 1185 | − |
| 38 | LINC00940 | ENSG00000235049 | 12 | 2044784 | 2046983 | 2200 | − |
| 39 | DOCK9 | ENSG00000088387 | 13 | 99629723 | 99631071 | 1349 | − |
| 40 | IFI27 | ENSG00000165949 | 14 | 94576831 | 94577488 | 658 | − |
| 41 | TNFAIP2 | ENSG00000185215 | 14 | 103593425 | 103593599 | 175 | − |
| 42 | C14orf2 | ENSG00000156411 | 14 | 104354891 | 104357110 | 2220 | − |
| 43 | PRSS8 | ENSG00000052344 | 16 | 31146195 | 31147170 | 976 | − |
| 44 | | ENSG00000213472 | 16 | 57653646 | 57654187 | 542 | − |
| 45 | C16orf47 | ENSG00000197445 | 16 | 73205055 | 73208273 | 3219 | − |
| 46 | NOS2 | ENSG00000007171 | 17 | 26127399 | 26127624 | 226 | − |
| 47 | TTLL6 | ENSG00000170703 | 17 | 46827430 | 46827674 | 245 | + |
| 48 | SOX9-AS1 | ENSG00000234899 | 17 | 70214796 | 70217271 | 2476 | + |
| 49 | MISP | ENSG00000099812 | 19 | 750971 | 751512 | 542 | − |
| 50 | FXYD3 | ENSG00000089356 | 19 | 35606461 | 35607002 | 542 | − |
| 51 | LGALS4 | ENSG00000171747 | 19 | 39303428 | 39303969 | 542 | − |
| 52 | SULT2B1 | ENSG00000088002 | 19 | 49054848 | 49055525 | 678 | − |
| 53 | RIN2 | ENSG00000132669 | 20 | 19865804 | 19868083 | 2280 | − |
| 54 | SGK2 | ENSG00000101049 | 20 | 42187567 | 42188108 | 542 | − |
| 55 | HNF4A | ENSG00000101076 | 20 | 42984091 | 42985366 | 1276 | − |
| 56 | HNF4A | ENSG00000101076 | 20 | 43029911 | 43030079 | 169 | − |
| 57 | TFF1 | ENSG00000160182 | 21 | 43786546 | 43786709 | 164 | − |
| 58 | BAIAP2L2; PLA2G6 | ENSG00000128298; ENSG00000184381 | 22 | 38505808 | 38510180 | 4373 | − |
| 59 | RP3-395M20.3; PLCH2 | ENSG00000229393; ENSG00000149527 | 1 | 2425373 | 2426522 | 1150 | − |
| 60 | | ENSG00000184157 | 1 | 43751338 | 43751678 | 341 | − |

[Table 3]

| DMR no. | Gene Symbol | Ensembl ID | Chromosome no. | DMR start | DMR end | Width | ± |
|---|---|---|---|---|---|---|---|
| 61 | RP11-543D5.1 | ENSG00000227947 | 1 | 48190866 | 48191292 | 427 | + |
| 62 | B3GALT2; CDC73 | ENSG00000162630; ENSG00000134371 | 1 | 193154938 | 193155661 | 724 | − |
| 63 | AC016747.3; KIAA1841; C2orf74 | ENSG00000212978; ENSG00000162929; ENSG00000237651 | 2 | 61371986 | 61372587 | 602 | + |
| 64 | AC007392.3 | ENSG00000232046 | 2 | 66809757 | 66810771 | 1015 | + |
| 65 | KCNE4 | ENSG00000152049 | 2 | 223916558 | 223916687 | 130 | − |
| 66 | AGAP1 | ENSG00000157985 | 2 | 236444053 | 236444434 | 382 | − |
| 67 | PPP2R3A | ENSG00000073711 | 3 | 135684043 | 135684227 | 185 | − |
| 68 | APOD | ENSG00000189058 | 3 | 195310802 | 195311018 | 217 | − |
| 69 | MUC4 | ENSG00000145113 | 3 | 195536032 | 195537321 | 1290 | − |
| 70 | MCIDAS | ENSG00000234602 | 5 | 54518579 | 54519189 | 611 | + |
| 71 | OCLN | ENSG00000197822 | 5 | 68787631 | 68787825 | 195 | − |
| 72 | PCDHGA2; NA | ENSG00000081853; ENSG00000241325 | 5 | 140797155 | 140797364 | 210 | + |
| 73 | C6orf195 | ENSG00000164385 | 6 | 2514359 | 2516276 | 1918 | − |
| 74 | | ENSG00000196333 | 6 | 19179779 | 19182021 | 2243 | − |
| 75 | HCG16 | ENSG00000244349 | 6 | 28956144 | 28956970 | 827 | + |
| 76 | HCG9 | ENSG00000204625 | 6 | 29943251 | 29943629 | 379 | + |
| 77 | RNF39 | ENSG00000204618 | 6 | 30039051 | 30039749 | 699 | + |
| 78 | SLC22A16 | ENSG00000004809 | 6 | 110797397 | 110797584 | 188 | + |
| 79 | PARK2 | ENSG00000185345 | 6 | 161796297 | 161797341 | 1045 | − |
| 80 | WBSCR17 | ENSG00000185274 | 7 | 70597038 | 70597093 | 56 | + |
| 81 | RN7SL76P | ENSG00000241959 | 7 | 151156201 | 151158179 | 1979 | − |
| 82 | SPIDR | ENSG00000164808 | 8 | 48571960 | 48573044 | 1085 | − |
| 83 | CA3 | ENSG00000164879 | 8 | 86350503 | 86350656 | 154 | + |
| 84 | PPP1R16A; GPT | ENSG00000160972; ENSG00000167701 | 8 | 145728374 | 145729865 | 1492 | − |
| 85 | NPY4R | ENSG00000204174 | 10 | 47083219 | 47083381 | 163 | + |
| 86 | C10orf107 | ENSG00000183346 | 10 | 63422447 | 63422576 | 130 | − |
| 87 | LINC00857 | ENSG00000237523 | 10 | 81967370 | 81967832 | 463 | − |
| 88 | VAX1 | ENSG00000148704 | 10 | 118891415 | 118891890 | 476 | + |
| 89 | TACC2 | ENSG00000138162 | 10 | 123922971 | 123923178 | 208 | + |
| 90 | MUC2 | ENSG00000198788 | 11 | 1058891 | 1062477 | 3587 | − |

[Table 4]

| DMR no. | Gene Symbol | Ensembl ID | Chromosome no. | DMR start | DMR end | Width | ± |
|---|---|---|---|---|---|---|---|
| 91 | MUC2 | ENSG00000198788 | 11 | 1074614 | 1075155 | 542 | − |
| 92 | TEAD1 | ENSG00000187079 | 11 | 12697507 | 12701324 | 3818 | − |
| 93 | RP11-121M22.1 | ENSG00000175773 | 11 | 130270828 | 130272842 | 2015 | + |
| 94 | KCNC2 | ENSG00000166006 | 12 | 75601683 | 75601943 | 261 | + |
| 95 | NCOR2 | ENSG00000196498 | 12 | 124906454 | 124908279 | 1826 | − |
| 96 | PDX1 | ENSG00000139515 | 13 | 28498306 | 28498463 | 158 | + |
| 97 | PDX1 | ENSG00000139515 | 13 | 28500855 | 28501186 | 332 | + |
| 98 | | ENSG00000198348 | 14 | 101922989 | 101923532 | 544 | + |
| 99 | MEIS2 | ENSG00000134138 | 15 | 37387445 | 37387655 | 211 | + |
| 100 | CCDC64B | ENSG00000162069 | 16 | 3079798 | 3080032 | 235 | + |
| 101 | ADCY9 | ENSG00000162104 | 16 | 3999535 | 4001924 | 2390 | − |
| 102 | | ENSG00000227093 | 16 | 54407005 | 54408952 | 1948 | + |
| 103 | GRB7 | ENSG00000141738 | 17 | 37895616 | 37896445 | 830 | − |
| 104 | RAPGEFL1 | ENSG00000108352 | 17 | 38347581 | 38347738 | 158 | + |
| 105 | WNK4 | ENSG00000126562 | 17 | 40936617 | 40936916 | 300 | + |
| 106 | HOXB6; HOXB-AS3 | ENSG00000239558; ENSG00000108511; ENSG00000233101 | 17 | 46674245 | 46674664 | 420 | + |
| 107 | CHAD; ACSF2 | ENSG00000136457; ENSG00000167107 | 17 | 48546115 | 48546272 | 158 | + |
| 108 | | ENSG00000230792 | 17 | 55212625 | 55214595 | 1971 | + |
| 109 | | ENSG00000171282 | 17 | 79393453 | 79393610 | 158 | − |
| 110 | TPM4 | ENSG00000167460 | 19 | 16178026 | 16178163 | 138 | − |
| 111 | | ENSG00000248094 | 19 | 21646440 | 21646771 | 332 | + |
| 112 | RP6-109B7.4; MIRLET7BHG | ENSG00000235159; ENSG00000197182; ENSG00000245020 | 22 | 46461776 | 46465514 | 3739 | − |

[2] The method for determining the likelihood of colorectal cancer development according to [1], in which in the determination step, in a case where one or more among the differentially methylated regions represented by differentially methylated region numbers 1, 3 to 20, 23 to 28, 31 to 46, 49 to 60, 62, 65 to 69, 71, 73, 74, 79, 81, 82, 84, 86, 87, 90 to 92, 95, 101, 103, 109, 110, and 112 have an average methylation rate of equal to or lower than the preset reference value, or one or more among the differentially methylated regions represented by differentially methylated region numbers 2, 21, 22, 29, 30, 47, 48, 61, 63, 64, 70, 72, 75 to 78, 80, 83, 85, 88, 89, 93, 94, 96 to 100, 102, 104 to 108, and 111 have an average methylation rate of equal to or higher than the preset reference value, it is determined that there is a high likelihood of colorectal cancer development in the human ulcerative colitis patient.

[3] The method for determining the likelihood of colorectal cancer development according to [1], in which in the measurement step, the methylation rates of the one or more CpG sites present in the differentially methylated region, of which an average methylation rate is included as a variable in the multivariate discrimination expression, are measured, and
in the determination step, in a case where based on an average methylation rate of the differentially methylated region calculated based on the methylation rates measured in the measurement step and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.
[4] The method for determining the likelihood of colorectal cancer development according to [3],
in which the multivariate discrimination expression includes, as variables, average methylation rates of two or more differentially methylated regions selected from the differentially methylated regions represented by the differentially methylated region numbers 1 to 112.
[5] The method for determining the likelihood of colorectal cancer development according to [3],
in which the multivariate discrimination expression includes, as variables, average methylation rates of three or more differentially methylated regions selected from the differentially methylated regions represented by the differentially methylated region numbers 1 to 112.
[6] The method for determining the likelihood of colorectal cancer development according to [3],
in which the multivariate discrimination expression includes, as variables, average methylation rates of one or more differentially methylated regions selected from the group consisting of the differentially methylated regions represented by the differentially methylated region numbers 1 to 58.
[7] The method for determining the likelihood of colorectal cancer development according to [3],
in which the multivariate discrimination expression includes, as variables, average methylation rates of one or more differentially methylated regions selected from the group consisting of the differentially methylated regions represented by the differentially methylated region numbers 1 to 11.
[8] A method for determining the likelihood of colorectal cancer development in a human ulcerative colitis patient, the method including:

a measurement step of measuring methylation rates of one or more CpG sites selected from the group consisting of CpG sites in base sequences represented by SEQ ID NOs: 1 to 80, in DNA recovered from a biological sample collected from the human ulcerative colitis patient; and
a determination step of determining the likelihood of colorectal cancer development in the human ulcerative colitis patient, based on the methylation rates measured in the measurement step and a preset reference value or a preset multivariate discrimination expression,
in which the reference value is a value for identifying a cancerous ulcerative colitis patient and a non-cancerous ulcerative colitis patient, which is set for the methylation rate of each CpG site, and
the multivariate discrimination expression includes, as a variable, the methylation rate of at least one CpG site among the CpG sites in the base sequences represented by SEQ ID NOs: I to 80.

[9] The method for determining the likelihood of colorectal cancer development according to [8],
in which in the measurement step, methylation rates of 2 to 10 CpG sites are measured.
[10] The method for determining the likelihood of colorectal cancer development according to [8] or [9],
in which in the determination step, in a case where one or more among CpG sites in the base sequences represented by SEQ ID NOs: 1, 2, 11, 12, 14 to 18, 21 to 24, 26, 27, 29, 31, 45, 64, 65, 67, 77, 79, and 80 have a methylation rate of equal to or lower than the preset reference value, or one or more among CpG sites in the base sequences represented by SEQ ID NOs: 3 to 10, 13, 19, 20, 25, 28, 30, 32 to 44, 46 to 63, 66, 68 to 76, and 78 have a methylation rate of equal to or higher than the preset reference value, it is determined that there is a high likelihood of colorectal cancer development in the human ulcerative colitis patient.
[11] The method for determining the likelihood of colorectal cancer development according to any one of [8] to [10],
in which in the measurement step, methylation rates of CpG sites in the base sequences represented by SEQ ID NOs: 1 to 32 are measured, and
in the determination step, in a case where one or more among CpG sites in the base sequences represented by SEQ ID NOs: 1, 2, 11, 12, 14 to 18, 21 to 24, 26, 27, 29, and 31 have a methylation rate of equal to or lower than the preset reference value, or one or more among CpG sites in the base sequences represented by SEQ ID NOs: 3 to 10, 13, 19, 20, 25, 28, 30, and 32 have a methylation rate of equal to or higher than the preset reference value, it is determined that there is a high likelihood of colorectal cancer development in the human ulcerative colitis patient.
[12] The method for determining the likelihood of colorectal cancer development according to any one of [8] to [11],
in which in the determination step, in a case where a sum of the number of CpG sites having a methylation rate equal to or lower than the preset reference value among CpG sites in the base sequences represented by SEQ ID NOs: 1, 2, 11, 12, 14 to 18, 21 to 24, 26, 27, 29 and 31, and the number of CpG sites having a methylation rate equal to or higher than the preset reference value among CpG sites in the base sequences represented by SEQ ID NOs: 3 to 10, 13, 19, 20, 25, 28, 30, and 32 is three or more, it is determined that there is a high likelihood of colorectal cancer development in the human ulcerative colitis patient.

[13] The method for determining the likelihood of colorectal cancer development according to any one of [8] to [10], in which in the measurement step, methylation rates of CpG sites in the base sequences represented by SEQ ID NOs: 1 to 16 are measured, and

in the determination step, in a case where one or more among CpG sites in the base sequences represented by SEQ ID NOs: 1, 2, 11, 12, and 14 to 16 have a methylation rate of equal to or lower than the preset reference value, or one or more among CpG sites in the base sequences represented by SEQ ID NOs: 3 to 10 and 13 have a methylation rate of equal to or higher than the preset reference value, it is determined that there is a high likelihood of colorectal cancer development in the human ulcerative colitis patient.

[14] The method for determining the likelihood of colorectal cancer development according to any one of [8] to [10] and [13],

in which in the determination step, in a case where a sum of the number of CpG sites having a methylation rate equal to or lower than the preset reference value among CpG sites in the base sequences represented by SEQ ID NOs: 1, 2, 11, 12, and 14 to 16, and the number of CpG sites having a methylation rate equal to or higher than the preset reference value among CpG sites in the base sequences represented by SEQ ID NOs: 3 to 10 and 13 is three or more, it is determined that there is a high likelihood of colorectal cancer development in the human ulcerative colitis patient.

[15] The method for determining the likelihood of colorectal cancer development according to any one of [8] to [10], in which in the measurement step, methylation rates of CpG sites in the base sequences represented by SEQ ID NOs: 1 to 9 are measured, and

in the determination step, in a case where one or more among CpG sites in the base sequences represented by SEQ ID NOs: 1 and 2 have a methylation rate of equal to or lower than the preset reference value, or one or more among CpG sites in the base sequences represented by SEQ ID NOs: 3 to 9 have a methylation rate of equal to or higher than the preset reference value, it is determined that there is a high likelihood of colorectal cancer development in the human ulcerative colitis patient.

[16] The method for determining the likelihood of colorectal cancer development according to any one of [8] to [10] and [15],

in which in the determination step, in a case where a sum of the number of CpG sites having a methylation rate equal to or lower than the preset reference value among CpG sites in the base sequences represented by SEQ ID NOs: 1 and 2, and the number of CpG sites having a methylation rate equal to or higher than the preset reference value among CpG sites in the base sequences represented by SEQ ID NOs: 3 to 9 is three or more, it is determined that there is a high likelihood of colorectal cancer development in the human ulcerative colitis patient.

[17] The method for determining the likelihood of colorectal cancer development according to any one of [8] to [10], in which in the measurement step, methylation rates of one or more CpG sites selected from the group consisting of CpG sites in the base sequences represented by SEQ ID NOs: 33 to 66 are measured, and

in the determination step, in a case where one or more among CpG sites in the base sequences represented by SEQ ID NOs: 45, 64, and 65 have a methylation rate of equal to or lower than the preset reference value, or one or more among CpG sites in the base sequences represented by SEQ ID NOs: 33 to 44, 46 to 63, and 66 have a methylation rate of equal to or higher than the preset reference value, it is determined that there is a high likelihood of colorectal cancer development in the human ulcerative colitis patient.

[18] The method for determining the likelihood of colorectal cancer development according to any one of [8] to [10] and [17],

in which in the determination step, in a case where a sum of the number of CpG sites having a methylation rate equal to or lower than the preset reference value among CpG sites in the base sequences represented by SEQ ID NOs: 45, 64, and 65, and the number of CpG sites having a methylation rate equal to or higher than the preset reference value among CpG sites in the base sequences represented by SEQ ID NOs: 33 to 44, 46 to 63, and 66 is two or more, it is determined that there is a high likelihood of colorectal cancer development in the human ulcerative colitis patient.

[19] The method for determining the likelihood of colorectal cancer development according to any one of [8] to [10], in which in the measurement step, methylation rates of one or more CpG sites selected from the group consisting of CpG sites in the base sequences represented by SEQ ID NOs: 33, 35, 36, 43, and 67 to 80 are measured, and in the determination step, in a case where one or more among CpG sites in the base sequences represented by SEQ ID NOs: 67, 77, 79, and 80 have a methylation rate of equal to or lower than the preset reference value, or one or more among CpG sites in the base sequences represented by SEQ ID NOs: 33, 35, 36, 43, 68 to 76, and 78 have a methylation rate of equal to or higher than the preset reference value, it is determined that there is a high likelihood of colorectal cancer development in the human ulcerative colitis patient.

[20] The method for determining the likelihood of colorectal cancer development according to any one of [8] to [10] and [19],
in which in the determination step, in a case where a sum of the number of CpG sites having a methylation rate equal to or lower than the preset reference value among CpG sites in the base sequences represented by SEQ ID NOs: 67, 77, 79, and 80, and the number of CpG sites having a methylation rate equal to or higher than the preset reference value among CpG sites in the base sequences represented by SEQ ID NOs: 33, 35, 36, 43, 68 to 76, and 78 is two or more, it is determined that there is a high likelihood of colorectal cancer development in the human ulcerative colitis patient.
[21] The method for determining the likelihood of colorectal cancer development according to [12], [14], [16], [18], or [20],
in which in a case where the sum is five or more, it is determined that there is a high likelihood of colorectal cancer development in the human ulcerative colitis patient.
[22] The method for determining the likelihood of colorectal cancer development according to [8] or [9],
in which the multivariate discrimination expression includes, as variables, methylation rates of one or more CpG sites selected from the group consisting of CpG sites in the base sequences represented by SEQ ID NOs: 33 to 66,
in the measurement step, a methylation rate of the CpG site which is included as a variable in the multivariate discrimination expression is measured, and
in the determination step, in a case where based on the methylation rates measured in the measurement step and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.
[23] The method for determining the likelihood of colorectal cancer development according to [8] or [9],
in which the multivariate discrimination expression includes, as variables, methylation rates of one or more CpG sites selected from the group consisting of CpG sites in the base sequences represented by SEQ ID NOs: 33, 35, 36, 43, and 67 to 80,
in the measurement step, a methylation rate of the CpG site which is included as a variable in the multivariate discrimination expression is measured, and
in the determination step, in a case where based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.
[24] The method for determining the likelihood of colorectal cancer development according to any one of [1] to [23],
in which the multivariate discrimination expression is a logistic regression expression, a linear discrimination expression, an expression created by Naive Bayes classifier, or an expression created by Support Vector Machine.
[25] The method for determining the likelihood of colorectal cancer development according to any one of [1] to [24],
in which the biological sample is intestinal tract tissue.
[26] The method for determining the likelihood of colorectal cancer development according to any one of [1] to [25],
in which the biological sample is rectal mucosal tissue.
[27] The method for determining the likelihood of colorectal cancer development according to [26],
in which the rectal mucosal tissue is collected by a kit for collecting large intestinal mucosa which includes a collection tool and a collection auxiliary tool,
the collection tool has

a first plate-like clamping piece with a first clamping surface for clamping large intestinal mucosa formed at one end thereof,
a second plate-like clamping piece with a second clamping surface for clamping large intestinal mucosa formed at one end thereof, and
a connection portion that connects the first clamping piece and the second clamping piece in a mutually opposed state at an end portion where the first clamping surface and the second clamping surface are not formed,

at least one of the first clamping surface and the second clamping surface is cup-shaped,
the collection auxiliary tool has

a truncated cone-shaped collection tool introduction portion having a slit on a side wall, and
a rod-like gripping portion,

one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width of one end portion of the first clamping piece and one end portion of the second clamping piece, and
the collection tool introduction portion has a larger outer diameter of 30 to 70 mm and a length in a rotation axis direction of 50 to 150 mm.
[28] The method for determining the likelihood of colorectal cancer development according to [27],
in which the collection tool has
a first bending portion on a side of an end portion where the first clamping surface is formed, rather than a center portion of the first clamping piece, and
a second bending portion on a side of an end portion where the second clamping surface is formed, rather than a center portion of the second clamping piece.
[29] A kit for collecting large intestinal mucosa, including:

a collection tool; and
a collection auxiliary tool,
in which the collection tool has

a first plate-like clamping piece with a first clamping surface for clamping large intestinal mucosa formed at one end thereof,
a second plate-like clamping piece with a second clamping surface for clamping large intestinal mucosa formed at one end thereof, and
a connection portion that connects the first clamping piece and the second clamping piece in a mutually opposed state at an end portion where the first clamping surface and the second clamping surface are not formed,

at least one of the first clamping surface and the second clamping surface is cup-shaped,
the collection auxiliary tool has
a truncated cone-shaped collection tool introduction portion having a slit on a side wall, and
a rod-like gripping portion,
one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width of one end portion of the first clamping piece and one end portion of the second clamping piece, and
the collection tool introduction portion has a larger outer diameter of 30 to 70 mm and a length in a rotation axis direction of 50 to 150 mm.

[30] The kit for collecting large intestinal mucosa according to [29],
in which the collection tool has
a first bending portion on a side of an end portion where the first clamping surface is formed, rather than a center portion of the first clamping piece, and
a second bending portion on a side of an end portion where the second clamping surface is formed, rather than a center portion of the second clamping piece.
[31] The kit for collecting large intestinal mucosa according to [29] or [30],
in which both the first clamping surface and the second clamping surface are cup-shaped.
[32] The kit for collecting large intestinal mucosa according to any one of [29] to [31],
in which the collection auxiliary tool has a through-hole in a rotation axis direction, and the collection tool introduction portion has a larger outer diameter of 30 to 70 mm and a length in a rotation axis direction of 50 to 150 mm, and the cup-shaped side edge portion has an inner diameter of 2 to 3 mm.
[33] The kit for collecting large intestinal mucosa according to any one of [29] to [32],
in which the first clamping surface and the second clamping surface have serrated side edge portions.
[34] A marker for analyzing a DNA methylation rate, including:

a DNA fragment having a partial base sequence containing one or more CpG sites selected from the group consisting of CpG sites in the base sequences represented by SEQ ID NOs: I to 80,

in which the marker is used to determine the likelihood of colorectal cancer development in an ulcerative colitis patient.

Advantageous Effects of the Invention

According to the method for determining the likelihood of colorectal cancer development according to the present invention, for a biological sample collected from an ulcerative colitis patient, it is possible to determine the likelihood of colorectal cancer development by investigating a methylation rate of a specific CpG site or an average methylation rate of a specific DMR in a genomic DNA. In addition, according to the kit for collecting rectal mucosa according to the present invention, it is possible to collect rectal mucosa from a patient's anus in a relatively safe and convenient manner.

Brief Description of the Drawings

FIG. 1 is an explanatory view of a collection tool 2A which is an embodiment of the collection tool and a collection tool 2B which is a modification example of the collection tool 2A.
FIG. 2 is an explanatory view of a collection tool 2C which is a modification example of the collection tool 2A.
FIG. 3 is an explanatory view of a collection auxiliary tool 11A which is an embodiment of a collection auxiliary tool 11.
FIG. 4 is an explanatory view of a collection auxiliary tool 11B which is a modification example of the collection auxiliary tool 11A.
FIG. 5 is an explanatory view of a use mode of a kit for collecting rectal mucosa.
FIG. 6A is a result of cluster analysis based on methylation levels of CpG sites in 32 CpG sets chosen as a result of comprehensive DNA methylation analysis in Example 1.
FIG. 6B is a result of principal component analysis based on methylation levels of CpG sites in 32 CpG sets chosen as a result of comprehensive DNA methylation analysis in Example 1.
FIG. 6C is a result of cluster analysis based on methylation levels of CpG sites in 16 CpG sets chosen as a result of comprehensive DNA methylation analysis in Example 1.
FIG. 6D is a result of principal component analysis based on methylation levels of CpG sites in 16 CpG sets chosen as a result of comprehensive DNA methylation analysis in Example 1.
FIG. 6E is a result of cluster analysis based on methylation levels of CpG sites in 9 CpG sets chosen as a result of comprehensive DNA methylation analysis in Example 1.
FIG. 6F is a result of principal component analysis based on methylation levels of CpG sites in 9 CpG sets chosen as a result of comprehensive DNA methylation analysis in Example 1.
FIG. 7A is a result of cluster analysis based on methylation levels of 27 CpG sites with an absolute value of DiffScore higher than 30 among CpG sites in the five miRNA genes of miR-1, miR-9, miR-124, miR-137, and miR-34b/c in Example 1.
FIG. 7B is a result of principal component analysis based on methylation levels of 27 CpG sites with an absolute value of DiffScore higher than 30 among CpG sites in the five miRNA genes of miR-1, miR-9, miR-124, miR-137, and miR-34b/c in Example 1.
FIG. 8A is a result of cluster analysis based on methylation levels of CpG sites in 34 CpG sets chosen as a result of comprehensive DNA methylation analysis in Example 2.
FIG. 8B is a result of principal component analysis based on methylation levels of CpG sites in 34 CpG sets chosen as a result of comprehensive DNA methylation analysis in Example 2.
FIG. 9 is a ROC curve of examination for the presence or absence of colorectal cancer development in ulcerative colitis patients in a case where methylation rates of the three CpG sites of a CpG site (cg10931190) in the base sequence represented by SEQ ID NO: 34, a CpG site (cg13677149) in the base sequence represented by SEQ ID NO: 37, and a CpG site (cg14516100) in the base sequence represented by SEQ ID NO: 56 are used as markers in Example 2.
FIG. 10A is a result of cluster analysis based on methylation levels of CpG sites in 18 CpG sets chosen as a result of comprehensive DNA methylation analysis in Example 3.
FIG. 10B is a result of principal component analysis based on methylation levels of CpG sites in 18 CpG sets chosen as a result of comprehensive DNA methylation analysis in Example 3.
FIG. 11 is a result of cluster analysis based on methylation rates of 112 DMR's (112 DMR sets) chosen as a result of comprehensive DNA methylation analysis in Example 4.
FIG. 12 is a result of principal component analysis based on methylation rates of 112 DMR's (112 DMR sets) chosen as a result of comprehensive DNA methylation analysis in Example 4.

FIG. 13 is a ROC curve of examination for the presence or absence of colorectal cancer development in ulcerative colitis patients in a case where average methylation rates of the three DMR's of DMR represented by DMR no. 2, DMR represented by DMR no. 10, and DMR represented by DMR no. 55 in Example 4 are used as markers in Example 4.

Description of Embodiments

<Method for determining the likelihood of colorectal cancer development

A cytosine base of a CpG site in a genomic DNA can undergo a methylation modification at a C5 position thereof. In the present invention and the present specification, in a case where a methylated cytosine base (methylated cytosine) amount and a non-methylated cytosine base (non-methylated cytosine) amount among CpG sites in a biological sample collected from an individual organism are measured, a methylation rate of a CpG site means a proportion (%) of the methylated cytosine amount with respect to a sum of both amounts. In addition, in the present invention and the present specification, an average methylation rate of DMR means an additive average value (arithmetic average value) or synergistic average value (geometric average value) of methylation rates of a plurality of CpG sites present in DMR. However, an average value other than these may be used.

The method for determining the likelihood of colorectal cancer development according to the present invention (hereinafter referred to as "determination method according to the present invention" in some cases) is a method for determining the likelihood of colorectal cancer development in a human ulcerative colitis patient in which the difference in methylation rate of CpG sites or DMR's in a genomic DNA between a group of ulcerative colitis patients (non-cancerous ulcerative colitis patients) who have not developed colorectal cancer and a group of ulcerative colitis patients (cancerous ulcerative colitis patients) who have developed colorectal cancer is used as a marker. Using a methylation rate of a CpG site or an average methylation rate of DMR, both of which become these markers, as an index, it is determined whether the likelihood of colorectal cancer development in a human ulcerative colitis patient is high or low. By using a methylation rate of a specific CpG site or an average methylation rate of a specific DMR as a marker used for determining the likelihood of colorectal cancer development in an ulcerative colitis patient, it is possible to detect colorectal cancer at an early stage in an ulcerative colitis patient, in whom it is very difficult to make a visual discrimination, in a more objective and sensitive manner, and it is possible to expect early detection.

Determination of the likelihood of colorectal cancer development in a human ulcerative colitis patient based on a methylation rate of a CpG site used as a marker may be made based on the measured methylation rate value itself of the CpG site, or in a case where a multivariate discrimination expression that includes the methylation rate of the CpG site as a variable is used, the determination may be made based on a discrimination value obtained from the multivariate discrimination expression.

Determination of the likelihood of colorectal cancer development in a human ulcerative colitis patient based on the average methylation rate of DMR used as a marker may be made based on an average methylation rate value itself of the DMR calculated from methylation rates of two or more CpG sites in the DMR, or in a case where a multivariate discrimination expression that includes the average methylation rate of the DMR as a variable is used, the determination may be made based on a discrimination value obtained from the multivariate discrimination expression.

For a CpG site and DMR which are used as markers in the present invention, it is preferable that a methylation rate thereof be largely different between a non-cancerous ulcerative colitis patient group and a cancerous ulcerative colitis patient group. A larger difference between the two groups allows the presence or absence of colorectal cancer development to be detected in a more reliable manner. For the CpG site and the DMR which are used as markers in the present invention, a methylation rate thereof in cancerous ulcerative colitis patients may be significantly higher than non-cancerous ulcerative colitis patients, that is, a higher methylation rate may be exhibited due to colorectal cancer development, or a methylation rate thereof in cancerous ulcerative colitis patients may be significantly lower than non-cancerous ulcerative colitis patients, that is, a lower methylation rate may be exhibited due to colorectal cancer development.

For the CpG site and the DMR which are used as markers in the present invention, it is more preferable that the same cancerous ulcerative colitis patient have a small difference in methylation rate between a non-cancerous site and a cancerous site of the large intestine. By using such a methylation rate of a CpG site or such an average methylation rate of DMR as an index, even in a case where a biological sample collected from a non-cancerous site of a cancerous ulcerative colitis patient is used, it is possible to determine the presence or absence of colorectal cancer development in a highly sensitive manner similar to a case where a biological sample collected from a cancerous site is used. For example, mucosa deep in the large intestine needs to be collected using an endoscope or the like, which places a heavy burden on a patient. However, rectal mucosa in the vicinity of the anus can be collected in a comparatively easy manner. By using a CpG site or DMR having a small difference in methylation rate between a non-cancerous site and a cancerous site of the large intestine as a marker, irrespective of a location where the cancerous site is formed, it is possible to detect a patient who has developed colorectal cancer using rectal mucosa in the vicinity of the anus as a biological sample without omission.

Among determination methods according to the present invention, the method for making a determination based on the measured methylation rate value itself of the CpG site is specifically a method for determining the likelihood of colorectal cancer development in a human ulcerative colitis patient, the method including a measurement step of measuring methylation rates of a plurality of specific CpG sites to be used as markers in DNA recovered from a biological sample collected from the human ulcerative colitis patient, and a determination step of determining the likelihood of colorectal cancer development in the human ulcerative colitis patient based on the methylation rates measured in the measurement step and a reference value set previously with respect to each CpG site.

Specifically, a CpG site used as a marker in the present invention is one or more CpG sites selected from the group consisting of CpG sites in the base sequences represented by SEQ ID NOs: 1 to 80. The respective base sequences are shown in Tables 5 to 12. In the base sequences of the tables, CG in brackets is a CpG site detected by comprehensive DNA methylation analysis shown in Examples 1 to 3. A DNA fragment having a base sequence containing these CpG sites can be used as a DNA methylation rate analysis marker for determining the likelihood of colorectal cancer development in an ulcerative colitis patient.

[Table 5]

| CpG ID | Base sequence | UCSC_REFGENE_NAME | ± | SEQ ID NO |
|---|---|---|---|---|
| cg05795005 | CCATCAGGGTAAGGGTACCTGGACTTGCGGCTTTTTAGGTCGGCCTGGCTCCGCTCCTTC[CG]CGGTGACGAGGTCCCCCGGCCTCCTAGGGTTGGGAAGAGCTGCTTTCCTGACTCTCGTTC | LIN7C;BDNFOS | − | 1 |
| cg05208607 | GGCTTGACTTCTCCCACGCCCCATAGACCCGGCACCGTGTAATAACTGGGCCCGTGTCCT[CG]CCTGAAAACTGGGGGTCACACGGCCTGTCCTGAAGAACTCTGATGTGATAAACACCATAG | KIAA1609 | − | 2 |
| cg20795417 | GAGGCCAAGACGGGAGGATCACTTGAACTCAGGAGTTCGAGACCAGCCTGGGTAACACAG[CG]AGACACTGTGTGAAAAAAATGTAAAAATTAACTGGGTGTGGTGGTGTGCGCCTGTAGTCC | TK1 | + | 3 |
| cg10528424 | GGGCAGCCCCTGCAGCACTGGGCAGACATGCTGGCCCACGCCCGGCGGCCCATTGCCCAG[CG]GCACCCCCTGCGGCCAGCCAGGGAGGTGGACCGCATGCTGGCCCTGCAGCCCCGCCTTCG | SYT8 | + | 4 |
| cg05876883 | CAAGCTGGAAAAGGGTGGAACTCATGGCTGGGCAGACAGGACAGTTCTCCAGGGATCTGG[CG]GTAGATCTGTGTCTGGAACCCAGGTTCCCTGATGTCTGTGTCAGGGTGCCACCCCAGACC | SLC38A7 | + | 5 |
| cg03978067 | GCGCCGGCAGGAGGGCCCTGAGCAGACCCGGCCCGGGGGCCCGGCCAAGGCCGCCTGCCC[CG]AGACCCCACTCCCAGCACCCACAGCAGAGCCACTGGGCCAGGGTGCCTCTGCCTTCCTGG | EEF1D | + | 6 |
| cg10772532 | CACATATGTCTGCCTCCTATCATTTCTTCATGAGGTTCAGGGCAAAGGGCCTAGTCAAGC[CG]ATGATCTTTGGTTGCCCCTACACTTTCCCCAAACCACCTACAAATAAACAAAACAAGGGG | C14orf145 | + | 7 |
| cg25287257 | CTGGGCCGCGGGGCTCCTACTGGGGCGCGGGCTGGTGGCTGGGCCGCGGGGGCGGCGAGT[CG]TCCTCCGAGGAGCAGTCGGAGGAGGCGGCGTGGACGCTGGCGCCGTTGCTGTAGGGGAAA | MNX1 | + | 8 |
| cg19848924 | TTGCGGGCCAGCGCGAGTTCCGGGTGGGTGGGGGATGGGCGGACCCCGCACTCGGAGCTG[CG]AGCAGGCCCCACCGGCCCCAGGCAGTGAAGGGCTTAGCACCTGGGCCAGCAGCTGCTGTG | | + | 9 |
| cg05161773 | GGCTCAGGAGAAGGGGTAGAACGGGAGGGCTTCCTGGAGGAAGGCTTCCTAACCAGAGAC[CG]GGGTAGGAGTTTGCCAGGCAGGTGATGCTGGCCAGCTTCTCTTGCCATTTTCCTTTTCTT | SEPT9 | + | 10 |
| cg07216619 | ACCTTTGCAGCGAGCGTTACAGCTCTTAAAGGTAGCGTATCCCGAGTTTTTCGTTCCTCC[CG]GTGGGTTCGTGGGCTGGTTACTCTAGCCGACTTCAGAAGTAAACCCACAGACCTCTGCAG | | − | 11 |

[Table 6]

| CpG ID | Base sequence | UCSC_REFGENE_NAME | ± | SEQ ID NO |
|---|---|---|---|---|
| cg11476907 | CTGGACACAGCCAGCTTGACTCTGGAAGAACCGCCTGGCACAAAGCAATCAGGCAGTGGG[CG]TTCCCTTTGACAGGCTGGCTGTCTTTACATAGAACCTACTGGAAACATCACATCTGCCTG | | − | 12 |
| cg09084244 | GCATTTTAATTCAGACTAGCCACGTTTCAGCGCTCAGTAGCCACCATAGCTAGGGGTCAC[CG]TATTGAACAGTGCAGGGCTGCAGCTACTAGCGGAGGGCTCCTGCGACGGACACACCGGGT | CDK2AP1 | + | 13 |
| cg00921266 | ACCGACTTGGGTATGTTTCTTATGAATATTACACGCGGAGCAGCGTCTGGTCCGGGGGTG[CG]GTGGGGGTGTTGGGGCGGGCGGGAGGGGAGACCAAGGCGGCTGGGGAAGCGCGGGCTGG | HOXA3 | − | 14 |
| cg01493009 | AGAAAACAGAAGAGACTTGTGTGTGTGTTACACATATGTACGTATACACACACGTGCGTT[CG]CAAGCATGCCTAAGGAGATTTCTTTCAAAAAGAAGGCTGGCCCAACAATTTCAGTGGCCA | FOXO1 | − | 15 |
| cg08101036 | CTAGTGGCACCGACTTGGGTATGTTTCTTATGAATATTACACGCGGAGCAGCGTCTGGTC[CG]GGGGTGCGGTGGGGGGTGTTGGGGCGGGCGGGAGGGGAGACCAAGGCGGCTGGGGAAGCG | HOXA3 | − | 16 |
| cg20106077 | GAATCCCATGAGTGATGGCCAATTCAGGAGGCGAAGCACCCAGCAAGTTCCCCACCACAG[CG]GACATGGAACACGCACGAGAGGCAGAGACATGAAGGACAGAAGGATGGAAGGAAGTACGG | WDR27 | − | 17 |
| cg12908908 | GGGTTGAGAACCACTGATTTAGACATTGCTGTCCCAATTAATATTTAAATAGTCACAGCC[CG]TTAGCTCCACTAATCCAGTTGCATTACCACCGGCATACAAAAGATTATTTTTTAAATACC | | − | 18 |
| cg04515524 | CACTTAGATGCTCAGTAAATGCTCCAGGAAACTGCAGCACAAGGAATAATGAACTTGGAG[CG]GGGAAGAGCTGGCTTTGTCCCGGGAGAGCTCGGGCAAGAGGCCTCGCATGTCTGTGCCTC | PLVAP | + | 19 |
| cg05380919 | AATGCAGTGATTAAAGGACACAAGGCCTCAGTGTGCATCATTCTCATTGTGGCTTTCAGG[CG]GCTGTGGAAGACAGGGTGGGGATGGTGGCTTCGGGAGGTGAGGTGCTCTGGGACTTGGGC | GSTT1 | + | 20 |
| cg15360451 | AGGGACCTTCCTTGGACACTCGGCTCCCTGGGCCTGACGGTGGACTCATCCTTTACAAGG[CG]GCTGGAGACGACCTGATTCTTCCATCCCTTTCCCCTGTGTGCAGGTTTTACTGGGCTGCG | | − | 21 |
| cg19775763 | GTCAGTGGGCTGGGGTGTGATCTGTGGGCGGGCTGGGGTGCCTGTGCAGTGATCTGTCGG[CG]GGCCGGGGTGTGATCTGTGGGCGGGCTGGGGTGCCTGTGCAGTGATCTGTCCGCAGGCTG | | − | 22 |

[Table 7]

| CpG ID | Base sequence | UCSC _REFGENE _NAME | ± | SEQ ID NO |
|---|---|---|---|---|
| cg01871025 | CCAGGATGCGTTGTCACCATAAGTTACAGTACAAGTTGGTTCCCTCTCTTCTCTCTCCCC[CG]CACCTCGACCTTCTGCCCTGTCTCAGACACACACACACACACACACACACACACACACAC | | − | 23 |
| cg05008296 | ATTGGGTTTTATAACTTTATAAAAGCCTTTCATTTGTTTTGTTCCTTATTCAGTCATTCA[CG]CATTTGACAAACATTTATGGCATTCCTATAGTGTACTAGGCACTGTGCTGATGTCCAGCA | RDH11 | − | 24 |
| cg08708231 | GCTTAGATTTCTCACATTCCAGCACATGCACATGGTCTGACAGTGGTTCTTCATGAGGAG[CG]GAGGTGGGGAGCATGGAGAGTGTGTGAGAGCCACCTGGGCACCTTTTTGTCAAAATATAC | OPCML | + | 25 |
| cg27024127 | TCACTCATTCATTCATCCAGAGACAGGCACAGACAGGCTGTGACACAGGAGCTGGCAATG[CG]GTCTCCACGTGGCCGGAACTGAGCGGCTATCTGGAATAAAGGGAGGGATTGCAGCGGCTG | SCARA3 | − | 26 |
| cg22274196 | GCAATATACAAATTAAAGGATGGGGGTTTTTTCCCATTCATTCAATAAATCGTTAGTGAA[CG]CCTTCTGGATACATGACAGCTAGGCCAGGGAATGAGCCTGCAAAGACGAGGAAGATGTCT | | − | 27 |
| cg11844537 | GAGACGAGCTAGTAATGGAGGGTGGGCCGTGGGGTGAGGAAGGTGCCCAAATTTGCCGAG[CG]GTAACCTTACCAAGGACTGGGAAGCAGGGTTTTCACCTACTGACCCCCGTCCCTCCTCGG | TCERG1L | + | 28 |
| cg09908042 | GGGAGAGTTCTTCCAGGATATGTCTGGCTGTGGACTAGCAAGTCCAGCCTCACCGTGTAT[CG]CCAAATTGCTCTCCAAACGATACCAATCTCCACCAGCAGCATCTGAAAGTTCCCATTGCT | PCSK6 | − | 29 |
| cg15828613 | ACCAAAGAAAATAGTTGCAGCTTAATGCCTCACTTGGGAGTTTGCAAAGTCTCTGCTCTC[CG]AAGGCCTTGGTGGGTGAAAAGCCTAAATCGTCCTTATTTCCCACCTTGCTTCTCTCCTTC | | + | 30 |
| cg06461588 | TGGTGGTTGATAGTGTTGTTCAGAACATCGATGTTTTTCCTGATTTTTGGTCTGTTCTGT[CG]ATTTCTGAGAAAGTATTAAAATTAAAGTTGGGTCTTGCATTTTTATCCATTCTGTCAGTC | DNAJC5 | − | 31 |
| cg08299859 | AGGGACTACCTTTCTGCGTATTCCTTTCTGTTCTTTAAAAATGTTAAACCATGGGGTGCT[CG]CTTCGGCAGCACATATACTAAAATTGGAACGATACAGAGAAGATTAGCATGGCCCCTGCG | | + | 32 |

32 CpG sites in brackets in the base sequences represented by SEQ ID NOs: 1 to 32 (hereinafter collectively referred to as "32 CpG sets" in some cases) have a largely different methylation rate between a non-cancerous ulcerative colitis patient group and a cancerous ulcerative colitis patient group in comprehensive DNA methylation analysis in Example 1 as described later. Among these, cancerous ulcerative colitis patients have a much lower methylation rate than non-cancerous ulcerative colitis patients at the CpG sites ("-" in the tables) in the base sequences represented by SEQ ID NOs: 1, 2, 11, 12, 14 to 18, 21 to 24, 26, 27, 29, and 31, and cancerous ulcerative colitis patients have a much higher methylation rate than non-cancerous ulcerative colitis patients at the CpG sites ("+" in the tables) in the base sequences represented by SEQ ID NOs: 3 to 10, 13, 19, 20, 25, 28, 30, and 32. The CpG site used as a marker is not limited to these 32 CpG sites and also includes other CpG sites in the base sequences represented by SEQ ID NOs: 1 to 32.

[Table 8]

| CpG ID | Base sequence | UCSC REFGENE _NAME | ± | SEQ ID NO |
|---|---|---|---|---|
| cg24887265 | CCCAGGGGGCGGCGGGCTGAGGAGCAGTGCGGGGCTGGATGATGAGTGGTCTGGCGTCCC[CG]ATGGAGTCTTCTCATCCTCCGAGCTGCCTAACACCGACTTGCCGCTGCCATTCAGCGGGT | SIX2 | + | 33 |
| cg10931190 | GGGCTGAGGCCCCAGGTGACAGACGTTTTCCAGTCTACGCTGCGCGGGGCTAAGCCTCTG[CG]AGGCAGAGCGCACTAAAGCGTGCGCCGCCTCCGGGAGAGCTGAGCTCAGGACAGCATCGT | TSLP | + | 34 |
| cg22797031 | TAACACGAATGACAAGTGGGTGATTTTCAAGAAGCGCCCGGTCCCTCTAGAGAATGCGTC[CG]AATATCAGCGGAGCCGACTGCGTATGCCTCCGGATGCCCATCTATAAACTCTCTTGCTTG | | + | 35 |
| cg22158650 | CGAGGAACAGCGAGCCCCCGGACGCTGACTGCAGGACGTCCCAGTTTGTGCCCGGGTCTC[CG]TCCCTCCCCGTACGGGGCTCGTACCCCCGGGCCTGGGTCTGACCCACAGGGCGCTGAGGC | | + | 36 |
| cg13677149 | GGCGGCAGTGGTGGGGGCGGCTCGCAAGGCACCCTGGCGTGCAGCGCCAGTGACCAGATG[CG]TCGTTACCGCACCGCCTTCACCCGAGAGCAGATTGCGCGGCTGGAGAAGGAATTCTACCG | EVX1 | + | 37 |
| cg22795586 | GCCTTAGCGCTCTGGTGACCTCCGCGGGATTCTGAGAAAAGCACTGCGGAACGGCGGGAG[CG]GGCCCTGCTGCTTGCTTCGCGCCCCCCACCCGCCCGGGGACCGCGACTAAGTCCCCGACG | | + | 38 |
| cg04389897 | AGCAGTAGCAGCAGCAGGAAGGGTTGCTGATCCCGGAGCTGTCACCCGCCGGAGGGTGGG[CG]CGCGGGGGGCTGGTGAGGCGTGGGAGGGGCGGGGCGGGAGGAGAGCCTCACTTTCTGTGC | TFAP2A | + | 39 |
| cg27651243 | CTCAGACCGCCCGTGGGTCACAAGTGCAAAGGTAACAGTGTCCCCTGGGAGGCCGGGATG[CG]TCGGGGGCGGGGAGGGCGCGCACCTGGGTCTCGGTGAGCATGAGCGAGGTGGCCACCTCG | MNX1 | + | 40 |
| cg09765089 | CTCGCGCAGGCAGCGGGCGCGTGTGGCCCGGGCTGGGCAAGCCGAGGAACAGCGAGCCCC[CG]GACGCTGACTGCAGGACGTCCCAGTTTGTGCCCGGGTCTCCGTCCCTCCCCGTACGGGGC | | + | 41 |
| cg17542408 | GCCCGCGGAGCCACGTCAGGCCCCCAGCTCCCCCGGATCCCACCACGCACCAGGCCCCTC[CG]CCCGGCAAGTGGCCCAAGCAGGCATCCGCAACGGAAGGACAATTTTAAAAACAAACCCTC | ODZ4 | + | 42 |
| cg21229570 | CCTCTCCCACACCAACCTCCAGCGCGCGAAGCAGAGAACGAGAGGAAAGTTTGCGGGGTT[CG]AATCGAAAATGTCGACATCTTGCTAATGGTCTGCAAACTTCCGCCAATTATGACTGACCT | | + | 43 |
| cg14394550 | GAGCGGTGTCTTGCTAGGCCGGTTGGGGTACTTGCGGGGCCGGATGGGCTTGAGGGTGAG[CG]GCGGCTGGGGCAGGCTGCCAAAGCCCGGGTGGATCTGCTTGTCTTTGAATGCCTTGATGG | EGR3 | + | 44 |

[Table 9]

| CpG ID | Base sequence | UCSC _REFGENE _NAME | ± | SEQ ID NO |
|---|---|---|---|---|
| cg20326647 | AGGGAGTTTATAGGGACTCCACGGCGCGGTGGCTCGCCTGGGCTGAGAGGCTGACTAACG[CG]CTGACACGGCGGCACGGGGCTTTACAGGCCACGGGCCCTGCCGGCGAGACTGGGAGGGAG | | − | 45 |
| cg20373036 | CACCGCACACTAACCACCCCAACGCCTGGGGGGCCAGCCCGGCACCGAACCCGTCTATCA[CG]TCAAGCGGCCAACCCCTCAACGTGTACTCGCAGCCTGGCTTCACCGTGAGCGGCATGCTG | POU3F4 | + | 46 |
| cg19968840 | CTCACAGCGGGTCCCCCCACTCCCCGGCAGGGTGGCGTTCTGCTTCTGGCTCCTCTCCAA[CG]TGCTGCTCTCCACGCCGGCCCCGCTCTACGGAGGCCTGGCACTGCTGACCACCGGAGCCT | DUOXA2 | + | 47 |
| cg12162138 | CCGCGGGGCAAGAGCGGGGCTGCCTGAGCCCGCGGAGCCACGTCAGGCCCCCAGCTCCCC[CG]GATCCCACCACGCACCAGGCCCCTCCGCCCGGCAAGTGGCCCAAGCAGGCATCCGCAACG | ODZ4 | + | 48 |
| cg01307130 | GAGGGTCTTTCCTGCCCGGGTTTCGGACACTGTTGGAGTTTCAGGGAGCTTGGGCGCAGG[CG]GCGATCTCAAAGCGCAGCAGGCTCCGCAGAAGAGGCGGGCTCCGGGCAGAGACCGCTAGC | | + | 49 |
| cg24960947 | CCCGCCCGCCTCTCGGCCCCCATCCCGGTCTGGTCCACTCCCACCCCTCCAACCCCATGC[CG]GCCACTGCAGTACTCACACCGCACGCCTGGGCTCTGCCTCTGGCCCGGGTTGGGGGCGGC | GAL3ST3 | + | 50 |
| cg26074603 | GTGGTTCTGCTTTGGTTTCCGAGTGGACGAGGTTCTCTGGGCAGCGGGACTGAGTCTTGG[CG]CCCAGGTGAGCCGCCCTTCTCCGACGAGAAACTACTTGTTGGCGTTTTCCGGATTCAGGT | KCNC2 | + | 51 |
| cg05575614 | GACGAATTCCCTTTTTCCCTCTACAGCAATCCCTCAGATTTCTGGGGGAAAATGGGGCCC[CG]TTTTCCAGTACACAGGCCACCCCAGGAAGACCGCGTCGGGCGCTGTGTGATCTGGAGAGT | | + | 52 |
| cg08309529 | CAGAATGGCGGCTCCAGAGGCGGTTTCAAGTTTCATAAGTCAGGTAACACTGTGGGTTTC[CG]CCTTCTCGGACGCGGGGAAAGGGGAGACAGGAGGCTTCCCCTTGCGCGGGGTGGGTCGGT | MNX1 | + | 53 |
| cg24879782 | CAGCCTAGAAGAAGGGTCCCCTCAGTAGAGACCAGGCCTCCAGCTCTCCGTCCGGCGCTC[CG]CTCCACAACCCGCCAGTCGATGTGAGGTCCGTCAAGGGAGCGATCCCTCCGTCTGCCCGG | | + | 54 |
| cg17538572 | GGGCTGCGAACCCCAACTGGCGGGCGACGGGGACTCCGAGCAGCAGCTTGTGGAGGCCTT[CG]AGGAAATGACCCGCAATCTCTTCTCGCTGCCCATCGACGTGCCCTTCAGCGGGCTGTACC | CYP26A1 | + | 55 |
| cg14516100 | GAGCTCACCCGGGTGGGAGACAGAGCCGGGGCGCGCGAGCTTGGTGTGGGGGCGCCACTC[CG]GGGCGGAGGGGAGGGGCTACCAGTGACTTCTCCGAGTCGGGAGCTAGAAAGAGGCTTCCG | SORBS2 | + | 56 |

[Table 10]

| CpG ID | Base sequence | UCSC _REFGENE _NAME | ± | SEQ ID NO |
|---|---|---|---|---|
| cg25740565 | TCTTTACCCCCGACTCCCTGGAGCTTGGTCTCGGGATGCCAACTTGGGGCACGGAGGCGA[CG]GGCTGCTCCGAAGCTGGAGGGTTTCTGCTTGGGTCAGAGGGATCACGACCTCAGCAGAGC | FLJ32063 | + | 57 |
| cg21045464 | GCTGATCGATGAAGGAGACAAGCTGGCCCACGGGGAGGTCAATACAATCGATGCGGACCT[CG]ACGAAACGGAAGAATCTCGCAGGTTCCTGCGTGCTGGGTTCCACTCAAAATGTTTCAGGA | | + | 58 |
| cg23955842 | GTGGCAGCGACGGCGGCGGCAGCGGAGATCCCAAGGTCCGTAAGCGGGGAACTGGGGGGT[CG]CAGGGCGGGCCGGCCAAGAGGCTTGGGAGCTGGGCGTTGCTGGGGGTGGAGGGATAGAAG | GPR50 | + | 59 |
| cg22964918 | CAGAGGGAGGAGGTGCCCCTCACTAGATAAGGGGCCGCCGGCTGGCTGCCGGCTCCATGA[CG]CCCGTGGGGTCACCCCCCGGCCCCGGGACTCAGCCAGCCTCGCTCCTCGCTCCTCGCTCC | EVX1 | + | 60 |
| cg00061551 | AAACCTCTTTCTTATGTAAAGTGCTCAGTCTCGGGTATGTCTTTATCAGCAGCATGAAAA[CG]GACTAATACAGGCCATCGCAGAGACACACATTAAACTCTCACTATGGCTACTTTGGGAGG | ALG1 | + | 61 |
| cg04610028 | CTTGCCCCAGCTGGGACAGCCCTGCTCTGAGGACCAGACACAGGCAGGTGTTGTGCTATC[CG]CAGTGGCTGTTTCTGGAAGGCAGGAGCCTGCCTTCACTTCTGCACCACTTAGCACAGTGC | RAB11B | + | 62 |
| cg20139683 | CAGCAGGGGGAGCCGGGATGTGGCTCACATGCCTGGGGCTGCTCCGTGGCCATCTGGATG[CG]TGCACACGGCAGCAGGGGCAGCCGGGATGTGGCTTACGTGCCTGGGGCTGCTCCGTGGCC | POLE | + | 63 |
| cg09549987 | GTGAGCATGGGTGATTGGGTGGGGGAGTTGGGAGGGGTGCTAGTGTTCCGTGTGTGTGCA[CG]TTTGTGCACATGCGTTGTATGCACCTATGTGTAGAGAGAGAAGGTGAATGAAGTGTAAGA | SPAG11B | − | 64 |
| cg02299007 | ACCCGTCCCGTTCGACGCCTCTGGCCGCCCCGTCCTTGCTTCTCATCTCACAGGGCACTG[CG]AGCCGCCTGTCGCAATCAGCATTGAGAGCCAAAACAGCTGTTTGGTGACTGTGCGAGGTT | | − | 65 |
| cg17917970 | ACCCTGCACCCCCAAAGTCCTGACAACGCACACCCCACGAAGCCGGCGCACGCGCCCCTA[CG]ACACCCATTCGGTGCTGCTCCGCACACCCCCGCACGCCGCCCGTGCACCTCCCGTGTCTC | DUSP9 | + | 66 |

34 CpG sites in brackets in the base sequences represented by SEQ ID NOs: 33 to 66 (hereinafter collectively referred to as "34 CpG sets" in some cases) have a largely different methylation rate between a non-cancerous ulcerative colitis patient group and a cancerous ulcerative colitis patient group in comprehensive DNA methylation analysis in Example 2 as described later. Among these, cancerous ulcerative colitis patients have a much lower methylation rate than non-cancerous ulcerative colitis patients at the CpG sites ("-" in the tables) in the base sequences represented by SEQ ID NOs: 45, 64, and 65, and cancerous ulcerative colitis patients have a much higher methylation rate than non-cancerous ulcerative colitis patients at the CpG sites ("+" in the tables) in the base sequences represented by SEQ ID NOs: 33 to 44, 46 to 63, and 66. The CpG site used as a marker is not limited to these 34 CpG sites and also includes other CpG sites in the base sequences represented by SEQ ID NOs: 33 to 66.

[Table 11]

| CpG ID | Base sequence | UCSC _REFGENE _NAME | ± | SEQ ID NO |
|---|---|---|---|---|
| cg10339295 | CCCCAAGCCTTGCCAGATTACATTGTCAAGGCCAGCACTTTGGAGATATTTCCTTGGTTT[CG]CAATTCACACAGTGACTAACACATGTTACATTTTGAAAACTTCTCTGGGTAAAAATTTAA | | − | 67 |
| cg24887265 | CCCAGGGGGCGGCGGGCTGAGGAGCAGTGCGGGGCTGGATGATGAGTGGTCTGGCGTCCC[CG]ATGGAGTCTTCTCATCCTCCGAGCTGCCTAACACCGACTTGCCGCTGCCATTCAGCGGGT | SIX2 | + | 33 |
| cg22797031 | TAACACGAATGACAAGTGGGTGATTTTCAAGAAGCGCCCGGTCCCTCTAGAGAATGCGTC[CG]AATATCAGCGGAGCCGACTGCGTATGCCTCCGGATGCCCATCTATAAACTCTCTTGCTTG | | + | 35 |
| cg01736784 | TAAAGCGCGGCGGGGAGTCCGGGGGGCTCCCGCCTGGAGGGCTGTGTGAGCGGCGGGCCG[CG]GGGCGGCGCGGGGGGCGCTCTCCACTCTGCGGAAGCTGCCCCCTCTGCCCTCCGGTCCGC | DDX25;PUS3 | + | 68 |
| cg22158650 | CGAGGAACAGCGAGCCCCCGGACGCTGACTGCAGGACGTCCCAGTTTGTGCCCGGGTCTC[CG]TCCCTCCCCGTACGGGGCTCGTACCCCCGGGCCTGGGTCTGACCCACAGGGCGCTGAGGC | | + | 36 |
| cg00723994 | GCCTCTGCCCGAGCGCGCCCTTCGGCCCCTGCAATTAGCGCCGGGAGGTCAGCAGGAACC[CG]GACGCCTTCACCCGCGGCTCAAAGCACAGCAAAAGGCGACCCCATCCCCTCCCCTCCGCG | | + | 69 |
| cg26315862 | GAAATCCCCCGCAGTTAGCGGTCAACAGAAAGGGCGACACGGAACGGGGTTCCTGGCACC[CG]AGCTCGCCGCACCGAAGTCTCCTGGTAACAGCGACACGGGACCGGGCTATGTGACCACAC | | + | 70 |
| cg19937061 | CGCGCCCGCAGGGCCCGCCCACCGCTTTGCTTACGCCGCTGCCCGTGGGCCACCCCGGCG[CG]CAGGGTCCCCAGCCCGCGCCTCCGCCACAGCCGGCTTTCCCGCGCAGCCACGGACTGCAC | | + | 71 |
| cg04004787 | AAAAGGACCAGCGGGATCCGGCCGCAAGAATTGGAAAGCCTAGGAAGTGGCGGTGGCTGG[CG]CGTTTGGGGAGCAGGAGTGGGGATAGGGAAGCAGAGCTTGAGAGACCTTCCTCCGGGGCA | | + | 72 |

[Table 12]

| CpG ID | Base sequence | UCSC _REFGENE _NAME | ± | SEQ ID NO |
|---|---|---|---|---|
| cg03409187 | GCGACGGAGACACTACCGAGAACCAGATGTTCGCCGCCCGCGTGGTCATCCTGCTGCTGC[CG]TTTGCCGTCATCCTGGCCTCCTACGGTGCCGTGGCCCGAGCTGTCTGTTGCATGCGGTTC | | + | 73 |
| cg00282249 | TTCCAGGAGCCCCCCGTATAAGGACCCCAGGGACTCCTCTCCCCACGCGGCCGGGCCGCC[CG]CCCGGCCCCCAGCCCGGAGAGCTGCCACCGACCCCCTCAACGTCCCAAGCCCCAGCTCTG | CCNA1 | + | 74 |
| cg20148575 | GGGGCCACCAGGTGGGCCGGGGGCGCGGTGGAAGCGGATGGTCTGGGTCGACGGGAGAAG[CG]AAGCGGGCGCGGGAGGCGGGCGCGGGAGGCGGGCGCGGGAGGCGGGCGCGGGAGGCGGGC | | + | 75 |
| cg21229570 | CCTCTCCCACACCAACCTCCAGCGCGCGAAGCAGAGAACGAGAGGAAAGTTTGCGGGGTT[CG]AATCGAAAATGTCGACATCTTGCTAATGGTCTGCAAACTTCCGCCAATTATGACTGACCT | | + | 43 |
| cg14416371 | GAGACACGAGTCCAGGGGCGCGGAGGGGCGGGCAGCGCGCGGAGTGGTGAGACTGAGCCG[CG]ATGGAACGCGCTGGGGAGACCCAGCCTGTTCGGCTCCAGGGTTCGGAGACATCCTGGGCT | MIR129-2 | + | 76 |
| cg26081900 | GCACACACACACACACGTGAATATATATATATATATATATATATATATATATGAAATC[CG]GATGGATCAAGATGTTTATAGAAATGCAAAGCTTTAAATCTGTGGAAGAAATGAGAGAAA | BTNL3 | − | 77 |
| cg10168149 | CGGAGTGCGCATTGCGCTAACACGCGCACGGGAATTGCACCCTTGCCGGAGCCTCCGCAC[CG]TGCGCCCTTCAAAGAGCTGGCGACCCCGCTCACGTGTAAGCAACCTCCCACTTTGAAACT | FLJ32063 | + | 78 |
| cg25366315 | CTGGTTCTGGGCCTTCCCAGACAAAAGCCAGAGACCCGGAGCCTCTTTCTGAGAAGGAAC[CG]GGCGTCCCCAAGATTTCCTCTAGCCGAGTCCCCTGGGTCCCCCGAGGACCGGGACAGCTC | | − | 79 |
| cg19850149 | CGGCGCGCTCTGCCAGGGACCCCCCCCCCCACCGCCGGTGCCCGAGTGGGCCGCGTAGG[CG]GGGCCCAGCCCATAGGCCGCCAGCTCCAGCCGCTGCAGCGTTCTACGCGGTCCGGGACGC | | − | 80 |

18 CpG sites in brackets in the base sequences represented by SEQ ID NOs: 33, 35, 36, 43, and 67 to 80 (hereinafter collectively referred to as " 18 CpG sets" in some cases) have a largely different methylation rate between a non-cancerous ulcerative colitis patient group and a cancerous ulcerative colitis patient group in comprehensive DNA methylation analysis in Example 3 as described later. Among these, cancerous ulcerative colitis patients have a much lower methylation rate than non-cancerous ulcerative colitis patients at the CpG sites ("-" in the tables) in the base sequences represented by SEQ ID NOs: 67, 77, 79, and 80, and cancerous ulcerative colitis patients have a much higher methylation rate than non-cancerous ulcerative colitis patients at the CpG sites ("+" in the tables) in the base sequences represented by SEQ ID NOs: 33, 35, 36, 43, 68 to 76, and 78. The CpG site used as a marker is not limited to these 18 CpG sites and also includes other CpG sites in the base sequences represented by SEQ ID NOs: 33, 35, 36, 43, and 67 to 80.

Regarding the respective CpG sites, reference values are previously set for identifying a cancerous ulcerative colitis patient and a non-cancerous ulcerative colitis patient. For the CpG sites marked with "+" in Tables 5 to 7 among the 32 CpG sets, and the CpG sites marked with "+" in Tables 8 to 12 among the 34 CpG sets and the 18 CpG sets, in a case where the measured methylation rate is equal to or higher than a preset reference value, it is determined that there is a high likelihood of colorectal cancer development in a human ulcerative colitis patient. For the CpG sites marked with "-" in Tables 5 to 7 among the 32 CpG sets, and the CpG sites marked with "-" in Tables 8 to 12 among the 34 CpG sets and the 18 CpG sets, in a case where the measured methylation rate is equal to or lower than a preset reference value, it is determined that there is a high likelihood of colorectal cancer development in a human ulcerative colitis patient.

The reference value for each CpG site can be experimentally obtained as a threshold value capable of distinguishing between a cancerous ulcerative colitis patient group and a non-cancerous ulcerative colitis patient group by measuring a methylation rate of the CpG site in both groups. Specifically, a reference value for methylation of any CpG site can be obtained by a general statistical technique. Examples thereof are shown below. However, ways of determining the reference value in the present invention are not limited to these.

As an example of a way of obtaining the reference value, for example, among ulcerative colitis patients, in patients (non-cancerous ulcerative colitis patients) who are not diagnosed with colorectal cancer by pathological examination using biopsy tissue in an endoscopic examination, DNA methylation of rectal mucosa is firstly measured for any CpG site. After performing measurement for a plurality of patients, a numerical value such as an average value or median value thereof which represents methylation of a group of these patients can be calculated and used as a reference value.

In addition, DNA methylation of rectal mucosa was measured for a plurality of non-cancerous ulcerative colitis patients and a plurality of cancerous ulcerative colitis patients, a numerical value such as an average value or a median value and a deviation which represent methylation of the cancerous ulcerative colitis patient group and the non-cancerous ulcerative colitis patient group were calculated, respectively, and then a threshold value that distinguishes between both numerical values is obtained taking the deviations also into consideration, so that the threshold value can be used a reference value.

As the CpG site used as a marker in the present invention, only the CpG sites in the base sequences represented by SEQ ID NOs: 1 to 16 may be used. Among the 32 CpG sets, these 16 CpG sites (hereinafter collectively referred to as "16 CpG sets" in some cases) have a small difference in methylation rate between a non-cancerous site and a cancerous site of the large intestine in cancerous ulcerative colitis patients. As the CpG site used as a marker in the present invention, it is also preferable to use only the CpG sites in the base sequences represented by SEQ ID NOs: 1 to 9. Among the 16 CpG sets, these 9 CpG sites (hereinafter collectively referred to as "9 CpG sets" in some cases) have a smaller difference in methylation rate between a non-cancerous site and a cancerous site of the large intestine in cancerous ulcerative colitis patients.

In the determination step, in a case where one or more among the CpG sites in the base sequences represented by SEQ ID NOs: 1, 2, 11, 12, 14 to 18, 21 to 24, 26, 27, 29, 31, 45, 64, 65, 67, 77, 79, and 80 have a methylation rate of equal to or lower than a preset reference value, or one or more among the CpG sites in the base sequences represented by SEQ ID NOs: 3 to 10, 13, 19, 20, 25, 28, 30, 32 to 44, 46 to 63, 66, 68 to 76, and 78 have a methylation rate of equal to or higher than a preset reference value, it is determined that there is a high likelihood of colorectal cancer development in the human ulcerative colitis patient. In the determination step according to the present invention, in a case where a sum of the number of CpG sites having a methylation rate equal to or lower than a preset reference value among the CpG sites in the base sequences represented by SEQ ID NOs: 1, 2, 11, 12, 14 to 18, 21 to 24, 26, 27, 29, 31, 45, 64, 65, 67, 77, 79, and 80, and the number of CpG sites having a methylation rate equal to or higher than a preset reference value among the CpG sites in the base sequences represented by SEQ ID NOs: 3 to 10, 13, 19, 20, 25, 28, 30, 32 to 44, 46 to 63, 66, 68 to 76, and 78 is 2 or more, preferably 3 or more, and more preferably five or more, it is determined that there is a high likelihood of colorectal cancer development in the human ulcerative colitis patient, which makes it possible to make a more accurate determination.

In a case of using the 32 CpG sets as markers in the present invention, that is, in a case where methylation rates of the 32 CpG sets are measured in the measurement step, in the determination step, in a case where one or more among the CpG sites in the base sequences represented by SEQ ID NOs: 1, 2, 11, 12, 14 to 18, 21 to 24, 26, 27, 29, and 31 have a methylation rate of equal to or lower than a preset reference value, or one or more among the CpG sites in the base sequences represented by SEQ ID NOs: 3 to 10, 13, 19, 20, 25, 28, 30, and 32 have a methylation rate of equal to or higher than a preset reference value, it is determined that there is a high likelihood of colorectal cancer development in the human ulcerative colitis patient. In the determination method according to the present invention, in a case where a sum of the number of CpG sites having a methylation rate equal to or lower than a preset reference value among the CpG sites in the base sequences represented by SEQ ID NOs: 1, 2, 11, 12, 14 to 18, 21 to 24, 26, 27, 29 and 31, and the number of CpG sites having a methylation rate equal to or higher than a preset reference value among the CpG sites in the base sequences represented by SEQ ID NOs: 3 to 10, 13, 19, 20, 25, 28, 30, and 32 is 3 or more, and preferably five or more, it is determined that there is a high likelihood of colorectal cancer development in the human ulcerative colitis patient, which makes it possible to make a more accurate determination.

In the case of using the 34 CpG sets as markers in the present invention, in the determination step, in a case where one or more among the CpG sites in the base sequences represented by SEQ ID NOs: 45, 64, and 65 have a methylation rate of equal to or lower than a preset reference value, or one or more among the CpG sites in the base sequences represented by SEQ ID NOs: 33 to 44, 46 to 63, and 66 have a methylation rate of equal to or higher than a preset reference value, it is determined that there is a high likelihood of colorectal cancer development in the human ulcerative colitis patient. In the determination method according to the present invention, in a case where a sum of the number of CpG sites having a methylation rate equal to or lower than a preset reference value among the CpG sites in the base sequences represented by SEQ ID NOs: 45, 64, and 65, and the number of CpG sites having a methylation rate equal to or higher than a preset reference value among the CpG sites in the base sequences represented by SEQ ID NOs: 33 to 44, 46 to 63, and 66 is two or more, preferably 3 or more, and more preferably five or more, it is determined that there is a high likelihood of colorectal cancer development in the human ulcerative colitis patient, which makes it possible to make a more accurate determination.

In a case of using the 18 CpG sets as markers in the present invention, in the determination step, in a case where one or more among the CpG sites in the base sequences represented by SEQ ID NOs: 67, 77, 79, and 80 have a methylation rate of equal to or lower than a preset reference value, or one or more among the CpG sites in the base sequences represented by SEQ ID NOs: 33, 35, 36, 43, 68 to 76, and 78 have a methylation rate of equal to or higher than a preset reference value, it is determined that there is a high likelihood of colorectal cancer development in the human ulcerative colitis patient. In the determination method according to the present invention, in a case where a sum of the number of CpG sites having a methylation rate equal to or lower than a preset reference value among the CpG sites in the base sequences represented by SEQ ID NOs: 67, 77, 79, and 80, and the number of CpG sites having a methylation rate equal to or higher than a preset reference value among the CpG sites in the base sequences represented by SEQ ID NOs: 33, 35, 36, 43, 68 to 76, and 78 is two or more, preferably three or more, and more preferably five or more, it is determined that there is a high likelihood of colorectal cancer development in the human ulcerative colitis patient, which makes it possible to make a more accurate determination.

In the present invention, one or more CpG sites selected from the group consisting of CpG sites in the base sequences represented by SEQ ID NOs: 1 to 80 can be used as markers. As the CpG site used as a marker in the present invention, all 80 CpG sites (hereinafter collectively referred to as "80 CpG sets" in some cases) in brackets in the base sequences represented by SEQ ID NOs: 1 to 80 may be used, or the 32 CpG sets, the 16 CpG sets, the 9 CpG sets, the 34 CpG sets, or the 18 CpG sets may be used. The CpG sites of the 32 CpG sets, the CpG sites of the 16 CpG sets, and the CpG sites of the 9 CpG sets are excellent in that all the sets show a small variance of methylation rate between a non-cancerous ulcerative colitis patient group and a cancerous ulcerative colitis patient group and have a high ability to identify the non-cancerous ulcerative colitis patient group and the cancerous ulcerative colitis patient group. On the other hand, the 34 CpG sets and the 18 CpG sets have somewhat lower specificity than the 32 CpG sets, the CpG sites of the 16 CpG sets, and the CpG sites of the 9 CpG sets. However, the 34 CpG sets and the 18 CpG sets have very high sensitivity, and, for example, are very suitable for primary screening examination of cancerous ulcerative colitis.

Among determination methods according to the present invention, the method for making a determination based on an average methylation rate value itself of a specific DMR is specifically a method for determining the likelihood of colorectal cancer development in a human ulcerative colitis patient, the method including a measurement step of measuring methylation rates of one or more CpG sites present in the specific DMR used as a marker in the present invention in DNA recovered from a biological sample collected from the human ulcerative colitis patient, and a determination step of determining the likelihood of colorectal cancer development in the human ulcerative colitis patient based on an average methylation rate of the DMR calculated based on the methylation rates measured in the measurement step and a reference value previously set with respect to the average methylation rate of each DMR. The average methylation rate of each DMR is calculated as an average value of methylation rates of all CpG sites, for which a methylation rate has been measured in the measurement step, among the CpG sites in the DMR.

Specifically, the DMR used as a marker in the present invention is one or more DMR's selected from the group consisting of DMR's represented by DMR numbers 1 to 112. Chromosomal positions and corresponding genes of the respective DMR's are shown in Tables 13 to 16. Base positions of start and end points of DMR's in the tables are based on a data set "GRCh37/hg19" of human genome sequence. A DNA fragment having a base sequence containing a CpG site present in these DMR's can be used as a DNA methylation rate analysis marker for determining the likelihood of colorectal cancer development in an ulcerative colitis patient.

[Table 13]

| DMR no. | Gene Symbol | Ensembl ID | Chromosome no. | DMR start | DMR end | Width | ± |
|---|---|---|---|---|---|---|---|
| 1 | MTMR11 | ENSG00000014914 | 1 | 149907598 | 149909051 | 1454 | − |
| 2 | SIX2 | ENSG00000170577 | 2 | 45233485 | 45233784 | 300 | + |
| 3 | COL3A1 | ENSG00000168542 | 2 | 189838986 | 189839961 | 976 | − |
| 4 | ARL14 | ENSG00000179674 | 3 | 160393670 | 160397766 | 4097 | − |
| 5 | S100P | ENSG00000163993 | 4 | 6695204 | 6695433 | 230 | − |
| 6 | VTRNA1-2 | ENSG00000202111 | 5 | 140098089 | 140099064 | 976 | − |
| 7 | PDGFA | ENSG00000197461 | 7 | 544037 | 545463 | 1427 | − |
| 8 | C9orf152 | ENSG00000188959 | 9 | 112970134 | 112970675 | 542 | − |
| 9 | TMPRSS4 | ENSG00000137648 | 11 | 117947606 | 117948147 | 542 | − |
| 10 | CEP112 | ENSG00000154240 | 17 | 63623628 | 63625636 | 2009 | − |
| 11 | ZMYND8 | ENSG00000101040 | 20 | 45946538 | 45947713 | 1176 | − |
| 12 | CASZ1 | ENSG00000130940 | 1 | 10839179 | 10839844 | 666 | − |
| 13 | KAZN | ENSG00000189337 | 1 | 15271343 | 15272595 | 1253 | − |
| 14 | RNF186; RP11-91K11.2 | ENSG00000178828; ENSG00000235434 | 1 | 20138780 | 20142876 | 4097 | − |
| 15 | SELENBP1 | ENSG00000143416 | 1 | 151344319 | 151345394 | 1076 | − |
| 16 | C1orf106 | ENSG00000163362 | 1 | 200862559 | 200865970 | 3412 | − |
| 17 | C4BPB | ENSG00000123843 | 1 | 207262158 | 207262699 | 542 | − |
| 18 | | ENSG00000224037 | 1 | 234851858 | 234853830 | 1973 | − |
| 19 | MALL | ENSG00000144063 | 2 | 110872470 | 110872878 | 409 | − |
| 20 | NOSTRIN | ENSG00000163072 | 2 | 169658610 | 169659453 | 844 | − |
| 21 | SATB2; SATB2-AS1 | ENSG00000119042; ENSG00000225953 | 2 | 200334655 | 200335051 | 397 | + |
| 22 | HDAC4 | ENSG00000068024 | 2 | 240174125 | 240175146 | 1022 | + |
| 23 | HRH1 | ENSG00000196639 | 3 | 11266750 | 11267368 | 619 | − |
| 24 | ATP13A4-AS1; ATP13A4 | ENSG00000225473; ENSG00000127249 | 3 | 193272384 | 193272925 | 542 | − |
| 25 | ARHGAP24 | ENSG00000138639 | 4 | 86748456 | 86749527 | 1072 | − |
| 26 | RP11-335O4.3; TRIM2 | ENSG00000235872; ENSG00000109654 | 4 | 154125233 | 154126208 | 976 | − |
| 27 | PDLIM3 | ENSG00000154553 | 4 | 186425209 | 186426241 | 1033 | − |
| 28 | FAM134B | ENSG00000154153 | 5 | 16508433 | 16509611 | 1179 | − |
| 29 | | ENSG00000222366 | 6 | 28944243 | 28946445 | 2203 | + |
| 30 | OR2I1P | ENSG00000237988 | 6 | 29520800 | 29521885 | 1086 | + |

[Table 14]

| DMR no. | Gene Symbol | Ensembl ID | Chromosome no. | DMR start | DMR end | Width | ± |
|---|---|---|---|---|---|---|---|
| 31 | FRK | ENSG00000111816 | 6 | 116381823 | 116382002 | 180 | − |
| 32 | IYD | ENSG00000009765 | 6 | 150689855 | 150690414 | 560 | − |
| 33 | SNX9 | ENSG00000130340 | 6 | 158374746 | 158376752 | 2007 | − |
| 34 | HOXA3 | ENSG00000243394; ENSG00000105997; ENSG00000240154 | 7 | 27154541 | 27155088 | 548 | − |
| 35 | DIP2C; PRR26 | ENSG00000151240; ENSG00000180525 | 10 | 695357 | 696843 | 1487 | − |
| 36 | TNKS1BP1 | ENSG00000149115 | 11 | 57087702 | 57091030 | 3329 | − |
| 37 | LRP5 | ENSG00000162337 | 11 | 68173589 | 68174773 | 1185 | − |
| 38 | LINC00940 | ENSG00000235049 | 12 | 2044784 | 2046983 | 2200 | − |
| 39 | DOCK9 | ENSG00000088387 | 13 | 99629723 | 99631071 | 1349 | − |
| 40 | IFI27 | ENSG00000165949 | 14 | 94576831 | 94577488 | 658 | − |
| 41 | TNFAIP2 | ENSG00000185215 | 14 | 103593425 | 103593599 | 175 | − |
| 42 | C14orf2 | ENSG00000156411 | 14 | 104354891 | 104357110 | 2220 | − |
| 43 | PRSS8 | ENSG00000052344 | 16 | 31146195 | 31147170 | 976 | − |
| 44 | | ENSG00000213472 | 16 | 57653646 | 57654187 | 542 | − |
| 45 | C16orf47 | ENSG00000197445 | 16 | 73205055 | 73208273 | 3219 | − |
| 46 | NOS2 | ENSG00000007171 | 17 | 26127399 | 26127624 | 226 | − |
| 47 | TTLL6 | ENSG00000170703 | 17 | 46827430 | 46827674 | 245 | + |
| 48 | SOX9-AS1 | ENSG00000234899 | 17 | 70214796 | 70217271 | 2476 | + |
| 49 | MISP | ENSG00000099812 | 19 | 750971 | 751512 | 542 | − |
| 50 | FXYD3 | ENSG00000089356 | 19 | 35606461 | 35607002 | 542 | − |
| 51 | LGALS4 | ENSG00000171747 | 19 | 39303428 | 39303969 | 542 | − |
| 52 | SULT2B1 | ENSG00000088002 | 19 | 49054848 | 49055525 | 678 | − |
| 53 | RIN2 | ENSG00000132669 | 20 | 19865804 | 19868083 | 2280 | − |
| 54 | SGK2 | ENSG00000101049 | 20 | 42187567 | 42188108 | 542 | − |
| 55 | HNF4A | ENSG00000101076 | 20 | 42984091 | 42985366 | 1276 | − |
| 56 | HNF4A | ENSG00000101076 | 20 | 43029911 | 43030079 | 169 | − |
| 57 | TFF1 | ENSG00000160182 | 21 | 43786546 | 43786709 | 164 | − |
| 58 | BAIAP2L2; PLA2G6 | ENSG00000128298; ENSG00000184381 | 22 | 38505808 | 38510180 | 4373 | − |
| 59 | RP3-395M20.3; PLCH2 | ENSG00000229393; ENSG00000149527 | 1 | 2425373 | 2426522 | 1150 | − |
| 60 | | ENSG00000184157 | 1 | 43751338 | 43751678 | 341 | − |

[Table 15]

| DMR no. | Gene Symbol | Ensembl ID | Chromosome no. | DMR start | DMR end | Width | ± |
|---|---|---|---|---|---|---|---|
| 61 | RP11-543D5.1 | ENSG00000227947 | 1 | 48190866 | 48191292 | 427 | + |
| 62 | B3GALT2;CDC73 | ENSG00000162630; ENSG00000134371 | 1 | 193154938 | 193155661 | 724 | − |
| 63 | AC016747.3; KIAA1841; C2orf74 | ENSG00000212978; ENSG00000162929; ENSG00000237651 | 2 | 61371986 | 61372587 | 602 | + |
| 64 | AC007392.3 | ENSG00000232046 | 2 | 66809757 | 66810771 | 1015 | + |
| 65 | KCNE4 | ENSG00000152049 | 2 | 223916558 | 223916687 | 130 | − |
| 66 | AGAP1 | ENSG00000157985 | 2 | 236444053 | 236444434 | 382 | − |
| 67 | PPP2R3A | ENSG00000073711 | 3 | 135684043 | 135684227 | 185 | − |
| 68 | APOD | ENSG00000189058 | 3 | 195310802 | 195311018 | 217 | − |
| 69 | MUC4 | ENSG00000145113 | 3 | 195536032 | 195537321 | 1290 | − |
| 70 | MCIDAS | ENSG00000234602 | 5 | 54518579 | 54519189 | 611 | + |
| 71 | OCLN | ENSG00000197822 | 5 | 68787631 | 68787825 | 195 | − |
| 72 | PCDHGA2; NA | ENSG00000081853; ENSG00000241325 | 5 | 140797155 | 140797364 | 210 | + |
| 73 | C6orf195 | ENSG00000164385 | 6 | 2514359 | 2516276 | 1918 | − |
| 74 | | ENSG00000196333 | 6 | 19179779 | 19182021 | 2243 | − |
| 75 | HCG16 | ENSG00000244349 | 6 | 28956144 | 28956970 | 827 | + |
| 76 | HCG9 | ENSG00000204625 | 6 | 29943251 | 29943629 | 379 | + |
| 77 | RNF39 | ENSG00000204618 | 6 | 30039051 | 30039749 | 699 | + |
| 78 | SLC22A16 | ENSG00000004809 | 6 | 110797397 | 110797584 | 188 | + |
| 79 | PARK2 | ENSG00000185345 | 6 | 161796297 | 161797341 | 1045 | − |
| 80 | WBSCR17 | ENSG00000185274 | 7 | 70597038 | 70597093 | 56 | + |
| 81 | RN7SL76P | ENSG00000241959 | 7 | 151156201 | 151158179 | 1979 | − |
| 82 | SPIDR | ENSG00000164808 | 8 | 48571960 | 48573044 | 1085 | − |
| 83 | CA3 | ENSG00000164879 | 8 | 86350503 | 86350656 | 154 | + |
| 84 | PPP1R16A; GPT | ENSG00000160972; ENSG00000167701 | 8 | 145728374 | 145729865 | 1492 | − |
| 85 | NPY4R | ENSG00000204174 | 10 | 47083219 | 47083381 | 163 | + |
| 86 | C10orf107 | ENSG00000183346 | 10 | 63422447 | 63422576 | 130 | − |
| 87 | LINC00857 | ENSG00000237523 | 10 | 81967370 | 81967832 | 463 | − |
| 88 | VAX1 | ENSG00000148704 | 10 | 118891415 | 118891890 | 476 | + |
| 89 | TACC2 | ENSG00000138162 | 10 | 123922971 | 123923178 | 208 | + |
| 90 | MUC2 | ENSG00000198788 | 11 | 1058891 | 1062477 | 3587 | − |

[Table 16]

| DMR no. | Gene Symbol | Ensembl ID | Chromosome no. | DMR start | DMR end | Width | ± |
|---|---|---|---|---|---|---|---|
| 91 | MUC2 | ENSG00000198788 | 11 | 1074614 | 1075155 | 542 | − |
| 92 | TEAD1 | ENSG00000187079 | 11 | 12697507 | 12701324 | 3818 | − |
| 93 | RP11-121M22.1 | ENSG00000175773 | 11 | 130270828 | 130272842 | 2015 | + |
| 94 | KCNC2 | ENSG00000166006 | 12 | 75601683 | 75601943 | 261 | + |
| 95 | NCOR2 | ENSG00000196498 | 12 | 124906454 | 124908279 | 1826 | − |
| 96 | PDX1 | ENSG00000139515 | 13 | 28498306 | 28498463 | 158 | + |
| 97 | PDX1 | ENSG00000139515 | 13 | 28500855 | 28501186 | 332 | + |
| 98 | | ENSG00000198348 | 14 | 101922989 | 101923532 | 544 | + |
| 99 | MEIS2 | ENSG00000134138 | 15 | 37387445 | 37387655 | 211 | + |
| 100 | CCDC64B | ENSG00000162069 | 16 | 3079798 | 3080032 | 235 | + |
| 101 | ADCY9 | ENSG00000162104 | 16 | 3999535 | 4001924 | 2390 | − |
| 102 | | ENSG00000227093 | 16 | 54407005 | 54408952 | 1948 | + |
| 103 | GRB7 | ENSG00000141738 | 17 | 37895616 | 37896445 | 830 | − |
| 104 | RAPGEFL1 | ENSG00000108352 | 17 | 38347581 | 38347738 | 158 | + |
| 105 | WNK4 | ENSG00000126562 | 17 | 40936617 | 40936916 | 300 | + |
| 106 | HOXB6; HOXB-AS3 | ENSG00000239558; ENSG00000108511; ENSG00000233101 | 17 | 46674245 | 46674664 | 420 | + |
| 107 | CHAD; ACSF2 | ENSG00000136457; ENSG00000167107 | 17 | 48546115 | 48546272 | 158 | + |
| 108 | | ENSG00000230792 | 17 | 55212625 | 55214595 | 1971 | + |
| 109 | | ENSG00000171282 | 17 | 79393453 | 79393610 | 158 | − |
| 110 | TPM4 | ENSG00000167460 | 19 | 16178026 | 16178163 | 138 | − |
| 111 | | ENSG00000248094 | 19 | 21646440 | 21646771 | 332 | + |
| 112 | RP6-109B7.4; MIRLET7BHG | ENSG00000235159; ENSG00000197182; ENSG00000245020 | 22 | 46461776 | 46465514 | 3739 | − |

DMR's represented by DMR numbers 1 to 112 (hereinafter collectively referred to as "112 DMR sets" in some cases) have a largely different methylation rate of a plurality of CpG sites contained in each region between a non-cancerous ulcerative colitis patient group and a cancerous ulcerative colitis patient group. Among these, cancerous ulcerative colitis patients have a much lower average methylation rate of DMR (average value of methylation rates of a plurality of CpG sites present in DMR) than non-cancerous ulcerative colitis patients at DMR's ("-" in the tables) represented by DMR numbers 1, 3 to 20, 23 to 28, 31 to 46, 49 to 60, 62, 65 to 69, 71, 73, 74, 79, 81, 82, 84, 86, 87, 90 to 92, 95, 101, 103, 109, 110, and 112, and cancerous ulcerative colitis patients have a much higher average methylation rate of DMR than non-cancerous ulcerative colitis patients at DMR's ("+" in the tables) represented by DMR numbers 2, 21, 22, 29, 30, 47, 48, 61, 63, 64, 70, 72, 75 to 78, 80, 83, 85, 88, 89, 93, 94, 96 to 100, 102, 104 to 108, and 111.

In the present invention, in a case where the average methyl ation rate of DMR is used as a marker, one of DMR's represented by DMR nos. 1 to 112 may be used as a marker, any two or more selected from the group consisting of DMR's represented by DMR numbers 1 to 112 may be used as markers, or all of the DMR's represented by DMR numbers 1 to 112 may be used as markers. In the present invention, from the viewpoint of further increasing determination accuracy, the number of DMR's used as a marker among DMR's represented by DMR nos. 1 to 112 is preferably two or more, more preferably three or more, even more preferably four or more, and still more preferably five or more.

From the viewpoint of obtaining further increased determination accuracy, the DMR whose methylation rate is used as a marker in the present invention is preferably one or more selected from the group consisting of DMR's represented by DMR numbers 1 to 58 (hereinafter collectively referred to as "58 DMR sets" in some cases), more preferably two or more selected from the 58 DMR sets, even more preferably three or more selected from the 58 DMR sets, still more preferably four or more selected from the 58 DMR sets, and particularly preferably five or more selected from the 58 DMR sets. Among these, one or more selected from the group consisting of DMR's represented by DMR nos. 1 to 11 (hereinafter collectively referred to as "11 DMR sets" in some cases) are preferable, 2 or more selected from 11 DMR sets are more preferable, 3 or more selected from the 11 DMR sets are even more preferable, 4 or more selected from the 11 DMR sets are still more preferable, and 5 or more selected from the 11 DMR sets are particularly preferable.

An average methylation rate of each DMR may be an average value of methylation rates of all CpG sites contained in each DMR or may be an average value obtained by optionally selecting, in a predetermined manner, at least one CpG site from all CpG sites contained in each DMR and averaging methylation rates of the selected CpG sites. A methylation rate of each CpG site can be measured in the same manner as the measurement of a methylation rate of a CpG site in the base sequence represented by SEQ ID NO: 1 or the like.

Regarding the average methylation rate of each DMR, a reference value is previously set for identifying a cancerous ulcerative colitis patient and a non-cancerous ulcerative colitis patient. For the DMR's marked with "+" in Tables 13 to 16 among the 112 DMR sets, in a case where the measured average methylation rate of the DMR is equal to or higher than a preset reference value, it is determined that there is a high likelihood of colorectal cancer development in a human ulcerative colitis patient. For the DMR's marked with "-" in Tables 13 to 16 among the 112 DMR sets, in a case where the measured average methylation rate of the DMR is equal to or lower than a preset reference value, it is determined that there is a high likelihood of colorectal cancer development in a human ulcerative colitis patient.

The reference value for the average methylation rate of each DMR can be experimentally obtained as a threshold value capable of distinguishing between a cancerous ulcerative colitis patient group and a non-cancerous ulcerative colitis patient group by measuring an average methylation rate of the DMR in both groups. Specifically, a reference value for an average methylation rate of DMR can be obtained by a general statistical technique.

In a case where methylation rates of CpG sites such as the 80 CpG sets are used as markers, in the determination method according to the present invention, it is possible to determine the likelihood of colorectal cancer development in the human ulcerative colitis patient based on the methylation rates measured in the measurement step and a preset multivariate discrimination expression, in the determination step. The multivariate discrimination expression includes, as variables, methylation rates of one or more CpG sites among CpG sites in the base sequences represented by SEQ ID NOs: 1 to 80.

In a case where average methylation rates of one or more DMR' s selected from the group consisting of the 112 DMR sets are used as markers, in the determination method according to the present invention, it is possible to determine the likelihood of colorectal cancer development in the human ulcerative colitis patient based on an average methylation rate of DMR calculated based on the methylation rates measured in the measurement step and a preset multivariate discrimination expression, in the determination step. The multivariate discrimination expression includes, as variables, methylation rates of one or more CpG sites among CpG sites in the 112 DMR sets.

The multivariate discrimination expression used in the present invention can be obtained by a general technique used for discriminating between two groups. As the multivariate discrimination expression, a logistic regression expression, a linear discrimination expression, an expression created by Naive Bayes classifier, or an expression created by Support Vector Machine are mentioned, but not limited thereto. For example, these multivariate discrimination expressions can be created using an ordinary method by measuring a methylation rate of one CpG site or two or more CpG sites among CpG sites in the base sequences represented by SEQ ID NOs: 1 to 80 with respect to a cancerous ulcerative colitis patient group and a non-cancerous ulcerative colitis patient group, and using the obtained methylation rate as a variable. In addition, these multivariate discrimination expressions can be created using an ordinary method by measuring an average methylation rate of one DMR or two or more DMR's among the DMR's in the 112 DMR sets with respect to the cancerous ulcerative colitis patient group and the non-cancerous ulcerative colitis patient group, and using the obtained methylation rate as a variable.

In the multivariate discrimination expression used in the present invention, a reference discrimination value for identifying a cancerous ulcerative colitis patient and a non-cancerous ulcerative colitis patient is previously set. The reference discrimination value can be experimentally obtained as a threshold value capable of distinguishing between a cancerous ulcerative colitis patient group and a non-cancerous ulcerative colitis patient group by obtaining a discrimination value which is a value of a multivariate discrimination expression used with respect to both groups and making a comparison for the discrimination value of the cancerous ulcerative colitis patient group and the discrimination value of the non-cancerous ulcerative colitis patient group.

In a case of making a determination using a multivariate discrimination expression, specifically, in the measurement step, a methylation rate of a CpG site or an average methylation rate of DMR which is included as a variable in the multivariate discrimination expression used is measured, and in the determination step, a discrimination value which is a value of the multivariate discrimination expression is calculated based on the methylation rate measured in the measurement step, and the multivariate discrimination expression, and, based on the discrimination value and a preset reference discrimination value, it is determined whether the likelihood of colorectal cancer development in a human ulcerative colitis patient in whom the methylation rate of the CpG site or the average methylation rate of the DMR is measured is high or low. In a case where the discrimination value is equal to or higher than the preset reference discrimination value, it is determined that the likelihood of colorectal cancer development in a human ulcerative colitis patient is high.

The multivariate discrimination expression used in the present invention is preferably an expression including, as variables, methylation rates of one or more CpG sites selected from the group consisting of the 34 CpG sites, more preferably an expression including, as variables, only methylation rates of one or more CpG sites selected from the group consisting of the 34 CpG sites, even more preferably an expression including, as variables, only methylation rates of two to ten CpG sites selected from the group consisting of the 34 CpG sites, and still more preferably an expression including, as variables, only methylation rates of two to five CpG sites selected from the group consisting of the 34 CpG sites.

The multivariate discrimination expression used in the present invention is preferably an expression including, as variables, methylation rates of one or more CpG sites selected from the group consisting of the 18 CpG sites, more preferably an expression including, as variables, only methylation rates of one or more CpG sites selected from the group consisting of the 18 CpG sites, even more preferably an expression including, as variables, only methylation rates of two to ten CpG sites selected from the group consisting of the 18 CpG sites, and still more preferably an expression including, as variables, only methylation rates of two to five CpG sites selected from the group consisting of the 18 CpG sites.

For CpG sites constituting the 34 CpG sets and the 18 CpG sets, even in a case where 2 to 10, and preferably two to five CpG sites are selected from these sets and only the selected CpG sites are used, it is possible to determine the likelihood of colorectal cancer development in a human ulcerative colitis patient with sufficient sensitivity and specificity. For example, as shown in Example 2 as described later, in a case where among the 34 CpG sets, the three CpG sites of the CpG site in the base sequence represented by SEQ ID NO: 34, the CpG site in the base sequence represented by SEQ ID NO: 37, and the CpG site in the base sequence represented by SEQ ID NO: 56 are used as markers, and a multivariate discrimination expression created by logistic regression using methylation rates of the three CpG sites as variables is used, it is possible to determine the likelihood of colorectal cancer development for a human ulcerative colitis patient with sensitivity of about 96% and specificity of about 92%. In a case where the number of CpG sites for which a methylation rate is measured is large in a clinical examination or the like, labor and cost may be excessive. By choosing a CpG site used as a marker from CpG sites constituting the 34 CpG sets and the 18 CpG sets, it is possible to accurately determine the likelihood of colorectal cancer development in a human ulcerative colitis patient using a reasonable number of CpG sites of two to ten which are measurable in a clinical examination.

The multivariate discrimination expression used in the present invention is preferably an expression including, as variables, average methylation rates of one or more DMR's selected from the group consisting of the 112 DMR sets as described above, more preferably an expression including, as variables, only average methylation rates of two or more DMR's selected from the group consisting of the 112 DMR sets as described above, even more preferably an expression including, as variables, only average methylation rates of three or more DMR's selected from the group consisting of the 112 DMR sets as described above, still more preferably an expression including, as variables, only average methylation rates of four or more DMR's selected from the group consisting of the 112 DMR sets as described above, and particularly preferably an expression including, as variables, only average methylation rates of five or more DMR's selected from the group consisting of the 112 DMR sets as described above. Among these, an expression including, as variables, average methylation rates of one or more DMR's selected from the group consisting of the 58 DMR sets as described above is preferable, an expression including, as variables, only average methylation rates of two or more DMR's selected from the group consisting of the 58 DMR sets as described above is more preferable, an expression including, as variables, only average methylation rates of two to ten DMR's selected from the group consisting of the 58 DMR sets as described above is even more preferable, an expression including, as variables, only average methylation rates of three to ten DMR's selected from the group consisting of the 58 DMR sets as described above is still more preferable, and an expression including, as variables, only average methylation rates of five to ten DMR's selected from the group consisting of the 58 DMR sets as described above is particularly preferable. More preferably, an expression including, as variables, average methylation rates of one or more DMR's selected from the group consisting of the 11 DMR sets as described above is preferable, an expression including, as variables, only average methylation rates of two or more DMR's selected from the group consisting of the 11 DMR sets as described above is more preferable, an expression including, as variables, only average methylation rates of two to ten DMR's selected from the group consisting of the 11 DMR sets as described above is even more preferable, an expression including, as variables, only average methylation rates of three to ten DMR's selected from the group consisting of the 11 DMR sets as described above is still more preferable, and an expression including, as variables, only average methylation rates of five to ten DMR's selected from the group consisting of the 11 DMR sets as described above is particularly preferable.

A biological sample to be subjected to the determination method according to the present invention is not particularly limited as long as the biological sample is collected from a human ulcerative colitis patient and contains a genomic DNA of the patient. The biological sample may be blood, plasma, serum, tears, saliva, or the like, or may be mucosa of gastrointestinal tract or a piece of tissue collected from other tissue such as liver. As the biological sample to be subjected to the determination method according to the present invention, large intestinal mucosa is preferable from the viewpoint of strongly reflecting a state of large intestine, and rectal mucosa is more preferable from the viewpoint of being collectible in a relatively less invasive manner. The rectal mucosa of the large intestine can be conveniently collected using, for example, a kit for collecting large intestinal mucosa as described later.

In addition, it is sufficient that the biological sample is in a state in which DNA can be extracted. The biological sample may be a biological sample that has been subjected to various pretreatments. For example, the biological sample may be formalin-fixed paraffin embedded (FFPE) tissue. Extraction of DNA from the biological sample can be carried out by an ordinary method, and various commercially available DNA extraction/purification kits can also be used.

A method for measuring a methylation rate of a CpG site is not particularly limited as long as the method is capable of distinguishing and quantifying a methylated cytosine base and a non-methylated cytosine base with respect to a specific CpG site. A methylation rate of a CpG site can be measured using a method known in the art as it is or with appropriate modification as necessary. As the method for measuring a methylation rate of a CpG site, for example, a bisulfite sequencing method, a combined bisulfite restriction analysis (COBRA) method, a quantitative analysis of DNA methylation using real-time PCR (qAMP) method, and the like are mentioned. Alternatively, the method may be performed using a microarray-based integrated analysis of methylation by isoschizomers (MIAM) method.

<Kit for collecting large intestinal mucosa>

A kit for collecting large intestinal mucosa according to the present invention includes a collection tool for clamping and collecting rectal mucosa and a collection auxiliary tool for expanding the anus and allowing the collection tool to reach a surface of large intestinal mucosa from the anus. Hereinafter, referring to FIGS. 1 to 5, the kit for collecting large intestinal mucosa according to the present invention will be described.

FIGS. 1(A) to 1(C) are explanatory views of a collection tool 2A which is an embodiment of a collection tool 2 of a kit 1 for collecting large intestinal mucosa. FIG. 1(A) is a perspective view showing a state in which force is not applied to a first clamping piece 3a and a second clamping piece 3b of the collection tool 2A, and FIG. 1(B) is a perspective view showing a state in which force is applied thereto. In addition, FIG. 1(C) is a partially enlarged view of a tip end having a clamping surface of the collection tool 2A. As shown in FIG. 1(A), the collection tool 2A has a first clamping piece 3a, a second clamping piece 3b, a connection portion 4, a first clamping surface 5a, and a second clamping surface 5b.

The first clamping piece 3a is a plate-like member with the first clamping surface 5a, which clamps large intestinal mucosa, formed at one end thereof, and the second clamping piece 3b is a plate-like member with the second clamping surface 5b, which clamps large intestinal mucosa, formed at one end thereof. In the connection portion 4, the first clamping piece 3a and the second clamping piece 3b are connected to each other in a mutually opposed state at an end portion where the first clamping surface 5a and the second clamping surface 5b are not formed. A shape of the first clamping piece 3a and the second clamping piece 3b may be a rod shape in addition to a plate shape, and there is no limitation on the shape as long as the shape has a certain length for clamping and collecting rectal mucosa.

Due to application of force to the first clamping piece 3a and the second clamping piece 3b, the two pieces come close to each other. Therefore, in a state in which the first clamping surface 5a and the second clamping surface 5b of the collection tool 2A are in contact with large intestinal mucosa, by applying force to the first clamping piece 3a and the second clamping piece 3b, it is possible to clamping large intestinal mucosa with the first clamping surface 5a and the second clamping surface 5b. More specifically, a side edge portion 6a of the first clamping surface 5a and a side edge portion 6b of the second clamping surface 5b come into contact with each other in a state in which the large intestinal mucosa is clamped therebetween. By separating the collection tool 2A from the large intestinal mucosa in this state, the large intestinal mucosa clamped between the first clamping surface 5a and the second clamping surface 5b is torn off and collected.

A length of the first clamping piece 3a and the second clamping piece 3b is preferably 50 to 250 mm, more preferably 100 to 200 mm, even more preferably 70 to 200 mm, and still more preferably 70 to 150 mm. By causing the first clamping piece 3a and the second clamping piece 3b to have a length in the above-mentioned range, it is easy to directly clamp and collect large intestinal mucosa from the anus.

At least one of the first clamping surface 5a and the second clamping surface 5b is preferably cup-shaped in order to collect large intestinal mucosa in a state in which damage of tissue is relatively small. Due to being a case where at least one of both surfaces is cup-shaped, a space is formed inside in a case where the side edge portion 6a of the first clamping surface 5a and the side edge portion 6b of the second clamping surface 5b come into contact with each other. Among the large intestinal mucosa clamped between the first clamping surface 5a and the second clamping surface 5b, a portion housed in the space is not subjected to much load in a case where the large intestinal mucosa is torn off, so that destruction of tissue can be suppressed. As shown in FIG. 1, both surfaces are cup-shaped, which makes it easier to collect the large intestinal mucosa and makes it possible to suppress destruction of tissue.

In a case where the first clamping surface 5a and the second clamping surface 5b are cup-shaped, an inner diameter of the side edge portion 6a and the side edge portion 6b may be set to such a size that a necessary amount of large intestinal mucosa can be collected. In a case of large intestinal mucosa to be subjected to the determination method according to the present invention, it is sufficient to have a size such that a small amount of mucosa can be collected. For example, by setting an inner diameter of the side edge portion 6a and the side edge portion 6b to 1 to 5 mm and preferably 2 to 3 mm, it is possible to collect a sufficient amount of large intestinal mucosa without excessively damaging the large intestinal mucosa.

It is sufficient that the side edge portion 6a and the side edge portion 6b can come into close contact with each other. The side edge portions may be flat, and are preferably serrated as shown in FIG. 1 (C). In a case of being serrated, the large intestinal mucosa can be cut and collected with a relatively weak force by being clamped between a side edge portion 6a' and a side edge portion 6b'.

A protrusion portion 8a may be formed on an inner side of either one of the first clamping piece 3a and the second clamping piece 3b, and a cylindrical portion 9a may be formed on an inner side of the other one, so that the protrusion portion 8a and the cylindrical portion 9a face each other. In a case where force is applied to the first clamping piece 3a and the second clamping piece 3b, a tip end of the protrusion portion 8a fits into the cylindrical portion 9a in a state in which the side edge portion 6a and the side edge portion 6b are in contact with each other. Due to the fact that the tip end of the protrusion portion 8a fits into the cylindrical portion 9a, it is possible to stably collect the large intestinal mucosa without misalignment of the side edge portion 6a and the side edge portion 6b in a case of separating the collection tool 2 from the large intestinal mucosa.

FIG. 1 (D) is an explanatory view of a collection tool 2B which is a modification example of the collection tool 2A, and more specifically, is a perspective view showing a state in which force is not applied to a first clamping piece 3a and a second clamping piece 3b of the collection tool 2B. The first clamping piece 3a may have a first bending portion 7a on a side of an end portion where the first clamping surface 5a is formed, rather than a center portion thereof. The second clamping piece 3b may have a second bending portion 7b on a side of an end portion where the second clamping surface 5b is formed, rather than a center portion thereof. Due to the fact that the first clamping piece 3a and the second clamping piece 3b are inclined while maintaining a mutually opposed state on a side of tip ends where the clamping surfaces are formed rather than central portions, it becomes easy to penetrate through a slit 13 of a collection auxiliary tool 11 and come into contact with large intestinal mucosa. Specifically, as shown in FIG. 1(D), bending is done to intersect a virtual plane P on which a side of the connection portion 4 from the center portion of the first clamping piece 3a and a side of the connection portion 4 from the center portion of the second clamping piece 3b are placed. A bending angle $\theta_1$ is preferably 10° to 50°, more preferably 20° to 40°, and even more preferably from 25° to 35°. In addition, a length from the first bending portion 7a to the tip end of the first clamping surface 5a and a length from the second bending portion 7b to the tip end of the second clamping surface 5b are preferably 20 to 60 mm, and more preferably 30 to 50 mm. By setting the length from the bending portion to the tip end of the clamping surface to be within the above-mentioned range, it becomes easier to collect mucosa in a state of penetrating the slit 13 of the collection auxiliary tool 11.

FIGS. 2(A) to 2(E) are explanatory views of a collection tool 2C which is another modification example of the collection tool 2A. FIG. 2(A) is a front view showing a state in which force is not applied to a first clamping piece 3a and a second clamping piece 3b of a collection tool 2, and FIG. 2(B) is a plan view of a collection tool 2C. FIG. 2(C) is an enlarged view of a protrusion portion 8b of the collection tool 2C. FIG. 2(D) is a plan view showing a state in which an engaging claw of the protrusion portion 8b on a tip end part of the collection tool 2C is engaged with an overhanging part of an opening edge portion of a cylindrical portion 9b. FIG. 2(E) is a plan view showing a state in which the first clamping surface 5a and the second clamping surface 5b on a tip end part of the collection tool 2C are bonded to each other.

In a case of collecting mucosal tissue from the rectum of a subject, the collection tool 2 is in a state in which a distance between the first clamping piece 3a and the second clamping piece 3b is closed rather than being open, which makes it easy to penetrate the slit 13 of the collection auxiliary tool 11. Therefore, as shown by the protrusion portion 8b in FIG. 2, a protrusion portion of the collection tool 2 may be an engaging claw. The number of engaging claws of the protrusion portion 8b may be one, or two or more, and any number thereof may be used as long as the protrusion portion 8b can be engaged with an overhanging part of an opening edge portion of the cylindrical portion 9b. In this case, the cylindrical portion 9b for engaging the protrusion portion 8b is provided with the overhanging part radially inward at the opening edge portion, which makes it possible to cause the engaging claw of the protrusion portion 8b to be engaged with the overhanging part of the opening edge portion of the cylindrical portion 9b (FIG. 2(D)). A height of the engaging claw of the protrusion portion 8b is preferably adjusted so that in a case of a state of being engaged with the overhanging part of the cylindrical portion 9b, a state in which tip ends of the first clamping surface 5a and the second clamping surface 5b are close to each other but not bonded to each other is caused, and in a case where force is further applied to the first clamping piece 3a and the second clamping piece 3b, it becomes possible to bond the first clamping surface 5a and the second clamping surface 5b to each other without causing the tip end of the protrusion portion 8b to go through a bottom part of the cylindrical portion 9b. As a result, the tip ends of the first clamping surface 5a and the second clamping surface 5b are stabilized in a state with close proximity, without applying force to the first clamping piece 3a and the second clamping piece 3b of the collection tool 2. The collection tool 2 penetrates through the slit 13 of the collection auxiliary tool 11 in a state of FIG. 2(D). In a case where the tip end comes into contact with rectal mucosal tissue, force is applied to the first clamping piece 3a and the second clamping piece 3b to be a state of FIG. 2(E), and a part of the mucosal tissue is caused to be clamped, so that mucosal tissue is collected.

In the collection tool 2, the first clamping piece 3a and the second clamping piece 3b may be provided with corresponding buffer portions 10a between the connection portion and the bending portion. The buffer portions 10a have elastic parts 10b at tip ends thereof, and in a state in which the engaging claw of the protrusion portion 8b is engaged with the overhanging part of the opening edge portion of the cylindrical portion 9b, the buffer portions 10a are bonded to each other at the elastic parts 10b (FIG. 2(D)). This buffer portion allows the collection tool 2 to more stably maintain a state in which the engaging claw of the protrusion portion 8b is engaged with the overhanging part of the opening edge portion of the cylindrical portion 9b. Even in a case where force is further applied to the first clamping piece 3a and the second clamping piece 3b, since the elastic parts 10b at the tip ends are deformed by pressing, it is possible to bond the first clamping surface 5a and the second clamping surface 5b to each other (FIG. 2(E)).

FIGS. 3(A) and 3(B) are explanatory views of a collection auxiliary tool 11A which is an embodiment of the collection auxiliary tool 11. FIG. 3(A) is a perspective view as seen from a lower side of the collection auxiliary tool 11A, and FIG. 3(B) is a bottom view as seen from a slit side of the collection auxiliary tool 11A. As shown in FIG. 3(A), the collection auxiliary tool 11A has a collection tool introduction portion 12, a slit 13, and a gripping portion 14.

The collection tool introduction portion 12 is a truncated cone-shaped member having a slit 13 on a side wall. In the collection tool introduction portion 12, insertion into the anus is done from a tip end side edge portion 15 having a smaller outer diameter, and the collection tool 2 is inserted from a proximal side edge portion 16 having a larger outer diameter. The collection tool introduction portion 12 may have a through-hole in a rotation axis direction. From the viewpoint of ease of insertion into the anus, an outer diameter of the proximal side edge portion 16 is preferably 30 to 70 mm, and more preferably 40 to 50 mm. In addition, from the viewpoint of ease of introduction of the collection tool 2 into a surface of large intestinal mucosa, an outer diameter of the tip end side edge portion 15 is preferably 10 to 30 mm, and more preferably 15 to 25 mm. Similarly, a length of the collection tool introduction portion 12 in a rotation axis direction is preferably 50 to 150 mm, more preferably 70 to 130 mm, and even more preferably 80 to 120 mm.

The slit 13 is provided from the tip end side edge portion 15 of the collection tool introduction portion 12 toward the proximal side edge portion 16. Presence of the slit 13 reaching the tip end side edge portion 15 on a part of a side wall of the collection tool introduction portion 12 increases a degree of freedom of movement of the tip end of the collection tool 2 in the intestinal tract, which makes it possible to more easily collect large intestinal mucosa in the rectum, the internal structure of which is complicated. The slit 13 may be set at any position of the collection tool introduction portion 12. For example, as shown in FIG. 3(A), the slit 13 is preferably located on a side close to the gripping portion 14. In addition, the number of the slit 13 provided in the collection tool introduction portion 12 may be one, or two or more.

In order to cause the collection tool 2 to penetrate the slit 13 and reach a surface of large intestinal mucosa, a width of the slit 13 is designed to be wider than a width of the first clamping surface 5a and the second clamping surface 5b of the collection tool 2 in a state in which the side edge portion 6a and the side edge portion 6b are in contact with each other. In addition, the width of the slit 13 may be constant. However, as shown in FIG. 3(B), the width of the slit 13 is preferably wider going from the tip end side edge portion 15 to a proximal side edge portion 16 side. For example, in a state in which the side edge portion 6a and the side edge portion 6b are in contact with each other, in a case where a width $L_1$ (see FIG. 1(B)) of the first clamping surface 5a and the second clamping surface 5b of the collection tool 2 is 3 to 8 mm, a width $L_2$ (see FIG. 3(B)) on a side of the tip end side edge portion 15 of the slit 13 is preferably 7 to 15 mm, and a width $L_3$ (see FIG. 3(B)) on a side of the proximal side edge portion 16 of the slit 13 is preferably 10 to 20 mm. Two or more slits 13 may be formed on a wall surface of the collection tool introduction portion 12.

One end of the gripping portion 14 is connected in the vicinity of the proximal side edge portion 16 of the collection tool introduction portion 12 in a direction away from the collection tool introduction portion 12. A length of the gripping portion 14 is preferably 50 to 150 mm, and more preferably 70 to 130 mm, from the viewpoint of ease of grasping by hand or the like. A shape of the gripping portion 14 may be any shape as long as the shape is easy to grasp, and may be, for example, a plate shape, a rod shape, or any other shape.

FIG. 4 is an explanatory view of a collection auxiliary tool 11B which is a modification example of the collection auxiliary tool 11A. FIG. 4(A) is a perspective view as seen from aN upper side of the collection auxiliary tool 11 B, and FIG. 4(B) is a perspective view as seen from a lower side thereof. In addition, FIGS. 4(C) to 4(G) are a front view, a plan view, a bottom view, a left side view, and a right side view of the collection auxiliary tool 11B, respectively. As shown in the gripping portion 14, the gripping portion of the collection auxiliary tool may be a hollow rod shape of which a lower side is open and which is reinforced by ribs.

FIG. 5 is an explanatory view showing a mode of use of the kit 1 for collecting large intestinal mucosa according to the present invention. First, the collection auxiliary tool 11 is inserted from the tip end side edge portion 15 into the anus of a subject whose large intestinal mucosa is to be collected. In a state in which the gripping portion 14 is held with one hand and is stabilized, the collection tool 2 is introduced from an opening part on a side of the proximal side edge portion 16. The introduced collection tool 2 is caused to penetrate through the slit 13 from the tip end and reach a surface of the large intestinal mucosa. The collection tool 2 is pulled out from the slit 13 in a state (clamping surfaces 5) where the large intestinal mucosa is clamped between the clamping surface 5a and the clamping surface 5b of the collection tool 2, so that the large intestinal mucosa can be collected.

Examples

Next, the present invention will be described in more detail by showing examples and the like. However, the present invention is not limited thereto.

[Example 1]

With respect to DNA in large intestinal mucosa collected from 8 patients (UC cancerous patients) (7 males and 1 female) who had been diagnosed as having colorectal cancer by pathological diagnosis using biopsy tissue in an endoscopic examination and had undergone surgery, and 8 patients with internal medicine treatment-refractory ulcerative colitis (non-cancerous UC patients) (7 males and 1 female) who had undergone surgery for other than cancer, among ulcerative colitis patients, comprehensive analysis for a methylation rate of a CpG site was conducted. An average age of the 8 UC cancerous patients was 47.1 $\pm$ 12.4 years old, and an average diseased-duration was 11.4 $\pm$ 7.3 years. An average age of the 8 non-cancerous UC patients was 44.3 $\pm$ 16.4 years old, and an average-diseased duration was 6.5 $\pm$ 5.2 years.

<Comprehensive analysis of methylation level of CpG site>

(1) Biopsy and DNA extraction

Mucosal tissue was collected from 3 locations in the large intestine of the same patient, and formalin fixed paraffin embedded (FFPE) samples were prepared according to an ordinary method. The collected sites were cecum, rectum, and cancerous part for the UC cancerous patients, and were cecum, transverse colon, and rectum for non-cancerous UC patients. A section was cut out from each of the FFPE samples and DNA was extracted using QIAmp DNA FFPE tissue kit (manufactured by Qiagen).

(2) Quality evaluation of DNA sample

A concentration of the obtained DNA was obtained as follows. That is, a fluorescence intensity of each sample was measured using Quant-iT PicoGreen ds DNA Assay Kit (manufactured by Life Technologies), and a concentration thereof was calculated using a calibration curve of λ-DNA attached to the kit.

Next, each sample was diluted to 1 ng/μL, with TE (pH 8.0), real-time PCR was carried out using Illumina FFPE QC Kit (manufactured by Illumina) and Fast SYBR Green Master Mix (manufactured by Life Technologies), so that a Ct value was obtained. A difference in Ct value (hereinafter referred to as ΔCt value) between the sample and a positive control was calculated for each sample, and quality was evaluated. Samples with a ΔCt value less than 5 were determined to have good quality and subjected to subsequent steps.

(3) Bisulfite treatment

Bisulfite treatment was performed on the DNA samples using EZ DNA Methylation Kit (manufactured by ZYMO RESEARCH).

(4) Restoration of degraded DNA and whole genome amplification

For each DNA after the bisulfite treatment, Infinium HD FFPE Restore Kit (manufactured by Illumina) was used to restore the degraded DNA. The restored DNA was alkali-denatured and neutralized. To the resultant were added enzymes and primers for amplification of the whole genome of Human Methylation 450 DNA Analysis Kit (manufactured by Illumina), and isothermal reaction was allowed to proceed in Incubation Oven (manufactured by Illumina) at 37°C for 20 hours or longer, so that the whole genome was amplified.

(5) Fragmentation and purification of whole genome-amplified DNA

To the whole genome-amplified DNA was added an enzyme for fragmentation of Human Methylation 450 DNA Analysis Kit (manufactured by Illumina Co.), and reaction was allowed to proceed in Microsample Incubator (SciGene) at 37°C for 1 hour. To the fragmented DNA were added a coprecipitant and 2-propanol, and the resultant was centrifuged to precipitate DNA.

(6) Hybridization

To the precipitated DNA was added a hybridization buffer, and reaction was allowed to proceed in Hybridization Oven (manufactured by Illumina) at 48°C for 1 hour, so that the DNA was dissolved. The dissolved DNA was incubated in Microsample Incubator (manufactured by SciGene) at 95 °C for 20 minutes to denature into single strands, and then dispensed onto the BeadChip of Human Methylation 450 DNA Analysis Kit (manufactured by Illumina). The resultant was allowed to react in Hybridization Oven at 48°C for 16 hours or longer to hybridize probes on the BeadChip with the single-stranded DNA.

(7) Labeling reaction and scanning

The probes on the BeadChip after the hybridization were subjected to elongation reaction to bind fluorescent dyes. Subsequently, the BeadChip was scanned with the iSCAN system (manufactured by Illumina), and methylated fluorescence intensity and non-methylated fluorescence intensity were measured. At the end of the experiment, it was confirmed that all of the scanned data was complete and that scanning was normally done.

(8) Quantification and comparative analysis of DNA methylation level

The scanned data was analyzed using the DNA methylation analysis software GenomeStudio (Version: V201 1.1). A DNA methylation level (β value) was calculated by the following expression.

$$[\beta \text{ value}] = [\text{Methylated fluorescence intensity}] \div ([\text{Methylated fluorescence intensity}] + [\text{Non-methylated fluorescence intensity}] + 100)$$

In a case where the methylation level is high, the β value approaches 1, and in a case where the methylation level is low, the β value approaches 0. DiffScore calculated by GenomeStudio was used for comparative analysis of the DNA methylation level of the UC cancer patient rectal sample group (n=8) for the non-cancerous UC patient rectal sample group (n=8). In a case where the DNA methylation levels of both groups are close to each other, DiffScore approaches 0. In a case where the level is higher in the UC cancerous patients, a positive value is exhibited, and in a case where the level is lower in the UC cancerous patients, a negative value is exhibited. The greater a difference in methylation level between both groups, the greater an absolute value of DiffScore. In addition, a value (Δβ value) obtained by subtracting an average β value of the non-cancerous UC patient rectal sample group (n=8) from an average β value of the UC cancer patient rectal sample group (n=8) was also used for the comparative analysis.

GenomeStudio and the software Methylation Module (Version: 1.9.0) were used for DNA methylation quantification and DNA methylation level comparative analysis. Setting conditions for GenomeStudio are as follows.

DNA methylation quantification;
Normalization: Yes (Controls)
Subtract Background: Yes
Content Descriptor: HumanMethytation450_15017482_v. 1.2. bpm

DNA methylation level comparative analysis;
Normalization: Yes (Controls)
Subtract Background: Yes
Content Descriptor: HumanMethylation450_15017482_v. 1.2. bpm

Ref Group: Comparative analysis 4. Group-3
Error Model: Illumina custom
Compute False Discovery Rate: No

(9) Multivariate analysis

Using the results obtained by the DNA methylation level quantification and comparative analysis, DiffScore was calculated with the statistical analysis software R (Version: 3.0.1, 64 bit, Windows (registered trademark)), and cluster analysis and principal component analysis were performed.

R script of cluster analysis:

> data.dist < -as.dist (1-cor (data. frame, use="pairwise.complete.obs",method="p"))> hclust (data.dist, method="complete")
# data. frame: data frame composed of CpG (row) x sample (column) # 1-Pearson correlation coefficient defined as distance, implemented by complete linkage method
R script of principal component analysis:

```
> prcomp(t(data.frame), scale = T)
# data.frame: data frame composed of CpG (row) x sample (column)
```

<Selection of CpG biomarker>

(1) Extraction of CpG biomarker candidates

As means for selecting GpG biomarker candidates from comprehensive DNA methylation analysis data, narrowing-down based on DiffScore and $\Delta\beta$ value has been reported (BMC Med genomics vol. 4, p. 50,2011; Sex Dev vol. 5, p. 70, 2011). Biomarker candidates are extracted by setting an absolute value of DiffScore to higher than 30 and an absolute value of $\Delta\beta$ value to higher than 0.2 for the former case, and by setting an absolute value of DiffScore to higher than 30 and an absolute value of $\Delta\beta$ value to higher than 0.3 for the latter case. According to these methods, biomarker candidates were extracted from 485,577 CpG sites loaded on the BeadChip.
Specifically, firstly, 72,905 CpG sites with an absolute value of DiffScore higher than 30 were selected from the 485,577 CpG sites. On the BeadChip, 86 CpG sites located in the respective gene regions of miR-1, miR-9, miR-124, miR-137, and miR-34 b/c described in PTL 1 were also loaded. However, among these, the number of the CpG sites with an absolute value of DiffScore higher than 30 was 27.
Next, from the 72,905 CpG sites, 32 CpG sites with an absolute value of $\Delta\beta$ value higher than 0.3 were extracted. Hereinafter, these 32 CpG sites are collectively referred to as "32 CpG sets". At this point, all CpG sites located in the respective gene regions described in PTL 1 were excluded.
Furthermore, for the purpose of discriminating cancerous patients without missing, in the cancer patient samples, narrowing-down to samples with less fluctuation in DNA methylation level was performed. That is, an unbiased variance var of $\beta$ values of 24 samples (3 sites $\times$ 8 samples per each site) of the UC cancerous patients was obtained, and 16 CpG sites with a value of unbiased variance var lower than 0.05 were chosen. Hereinafter, these 16 CpG sites are collectively referred to as "16 CpG sets". From the 16 CpG sets, further narrowing-down to 9 CpG sites with a value of unbiased variance var lower than 0.03 was performed. Hereinafter, the 9 CpG sites are collectively referred to as "9 CpG sets".
The results of the respective CpG sites of the 32 CpG sets are shown in Table 17. In the table, the CpG site with # in the "16 CpG" column shows a CpG site included in the 16 CpG sets, and the CpG site with the # in the "9 CpG" column shows a CpG site included in the 9 CpG sets.

[Table 17]

| CpG ID | Average β value (cancerous UC rectal) n=8 | Average β value (non-cancerous UC rectal) n=8 | DiffScore | Δβ value | β value unbiased variance (cancerous UC) n=24 | 32 CpG | 16 CpG | 9 CpG |
|---|---|---|---|---|---|---|---|---|
| cg05795005 | 0.03 ± 0.01 | 0.45 ± 0.35 | -371 | -0.41 | 0.000 | # | # | # |
| cg05208607 | 0.09 ± 0.10 | 0.50 ± 0.42 | -371 | -0.37 | 0.006 | # | # | # |
| cg20795417 | 0.82 ± 0.10 | 0.51 ± 0.40 | 374 | 0.31 | 0.014 | # | # | # |
| cg10528424 | 0.68 ± 0.18 | 0.38 ± 0.38 | 374 | 0.30 | 0.021 | # | # | # |
| cg05876883 | 0.62 ± 0.15 | 0.23 ± 0.26 | 374 | 0.39 | 0.023 | # | # | # |
| cg03978067 | 0.89 ± 0.15 | 0.53 ± 0.30 | 374 | 0.35 | 0.025 | # | # | # |
| cg10772532 | 0.62 ± 0.13 | 0.23 ± 0.06 | 374 | 0.38 | 0.026 | # | # | # |
| cg25287257 | 0.61 ± 0.15 | 0.31 ± 0.18 | 374 | 0.30 | 0.029 | # | # | # |
| cg19848924 | 0.76 ± 0.14 | 0.40 ± 0.24 | 374 | 0.36 | 0.030 | # | # | # |
| cg05161773 | 0.57 ± 0.20 | 0.24 ± 0.28 | 374 | 0.33 | 0.034 | # | # | |
| cg07216619 | 0.27 ± 0.20 | 0.58 ± 0.15 | -371 | -0.31 | 0.035 | # | # | |
| cg11476907 | 0.22 ± 0.20 | 0.59 ± 0.14 | -371 | -0.36 | 0.036 | # | # | |
| cg09084244 | 0.43 ± 0.21 | 0.12 ± 0.16 | 374 | 0.30 | 0.037 | # | # | |
| cg00921266 | 0.36 ± 0.15 | 0.70 ± 0.12 | -371 | -0.34 | 0.045 | # | # | |
| cg01493009 | 0.30 ± 0.24 | 0.64 ± 0.24 | -368 | -0.30 | 0.045 | # | # | |
| cg08101036 | 0.37 ± 0.16 | 0.67 ± 0.13 | -367 | -0.30 | 0.045 | # | # | |
| cg20106077 | 0.26 ± 0.25 | 0.64 ± 0.30 | -371 | -0.38 | 0.054 | # | | |
| cg12908908 | 0.13 ± 0.25 | 0.49 ± 0.31 | -371 | -0.35 | 0.058 | # | | |
| cg04515524 | 0.59 ± 0.25 | 0.17 ± 0.20 | 374 | 0.41 | 0.062 | # | | |
| cg05380919 | 0.78 ± 0.22 | 0.49 ± 0.37 | 374 | 0.31 | 0.062 | # | | |
| cg15360451 | 0.31 ± 0.27 | 0.62 ± 0.18 | -371 | -0.31 | 0.065 | # | | |
| cg19775763 | 0.20 ± 0.28 | 0.61 ± 0.32 | -371 | -0.40 | 0.072 | # | | |
| cg01871025 | 0.43 ± 0.27 | 0.79 ± 0.02 | -371 | -0.35 | 0.072 | # | | |
| cg05008296 | 0.45 ± 0.29 | 0.76 ± 0.11 | -371 | -0.30 | 0.089 | # | | |
| cg08708231 | 0.58 ± 0.27 | 0.25 ± 0.31 | 374 | 0.31 | 0.092 | # | | |
| cg27024127 | 0.38 ± 0.30 | 0.69 ± 0.27 | -365 | -0.30 | 0.094 | # | | |
| cg22274196 | 0.28 ± 0.31 | 0.63 ± 0.15 | -371 | -0.34 | 0.103 | # | | |
| cg11844537 | 0.80 ± 0.33 | 0.45 ± 0.48 | 374 | 0.31 | 0.104 | # | | |
| cg09908042 | 0.28 ± 0.32 | 0.61 ± 0.17 | -371 | -0.32 | 0.108 | # | | |
| cg15828613 | 0.57 ± 0.36 | 0.26 ± 0.32 | 374 | 0.31 | 0.111 | # | | |
| cg06461588 | 0.45 ± 0.36 | 0.89 ± 0.02 | -371 | -0.37 | 0.123 | # | | |
| cg08299859 | 0.68 ± 0.40 | 0.38 ± 0.44 | 374 | 0.36 | 0.139 | # | | |

(2) Multivariate analysis of clinical samples using CpG biomarker candidates

Cluster analysis and principal component analysis for all 48 samples were performed using the 32 CpG sets, 16 CpG sets, and 9 CpG sets, and as shown in FIGS. 6A, 6C, and 6E, in the cluster analysis, all UC cancer patient samples accumulated in the same cluster (within a frame, in the drawings) in any of the CpG sets. In addition, as shown in FIGS. 6B, 6D, and 6F, in the principal component analysis (the vertical axis is a second principal component), the UC cancer patient samples (●) and the non-cancerous UC patient samples **(▲)** each formed independent clusters, in a first principal component (horizontal axis) direction. That is, in any of the CpG sets, it was possible to clearly distinguish between the 24 UC cancer patient samples and the 24 non-cancerous UC patient samples. On the other hand, cluster analysis and principal component analysis were performed in the same manner using 27 CpG sites chosen from the CpG sites located in the respective gene regions described in PTL 1, and as shown in FIGS. 7A and 7B, it was not possible to clearly distinguish between the UC cancer patient samples and the non-cancerous UC patient samples. From these results, 32 CpG's listed in Table 17 are extremely useful as biomarkers of colorectal cancer development in an ulcerative colitis patient, and it is apparent that these CpG's can be used to determine the presence or absence of colorectal cancer development in an ulcerative colitis patient with high sensitivity and specificity.

[Example 2]

Apart from the ulcerative colitis patients of Example 1, with respect to DNA in large intestinal mucosa collected from 24 patients (UC cancerous patients) who had been diagnosed as having colorectal cancer by pathological diagnosis using biopsy tissue in an endoscopic examination and had undergone surgery, and 24 patients with internal medicine treatment-refractory ulcerative colitis (non-cancerous UC patients) who had undergone surgery for other than cancer, comprehensive analysis for a methylation rate of a CpG site was conducted.

For the DNA to be subjected to analysis of a methylation rate of a CpG site, DNA was extracted from an FFPE sample collected from mucosal tissue of the rectum of an ulcerative colitis patient in the same manner as in Example 1, the whole genome was amplified, and quantification and comparative analysis of DNA methylation level of the CpG site were performed. The results were used to calculate DiffScore, and cluster analysis and principal component analysis were performed.

(1) Extraction of CpG biomarker candidates

Subsequently, CpG biomarker candidates were extracted from comprehensive DNA methylation analysis data. Specifically, firstly, 324 CpG sites with an absolute value of $\Delta\beta$ higher than 0.2 were extracted from 485,577 CpG sites.

Next, the following two types of logistic regression models were created.

(1) 161,700 logistic regression models based on all combinations of 3 CpG sites, the 3 CpG sites obtained by selecting the top 100 CpG sites from 324 CpG sites in 2 groups of t-test assay and selecting three CpG's from the 100 CpG's.

(2) 52,326 logistic regression models based on all combinations of 2 CpG's selected from 324 CpG sites.

Regarding discrimination expressions of both logistic regression models, a CpG site that satisfies each of the following four criteria was selected, and a frequency of appearing CpG sites was calculated.

[Criterion 1] Sensitivity of higher than 90%, specificity of higher than 90%, and coefficient p value of discrimination expression of lower than 0.05. [Criterion 2] Sensitivity of higher than 90%, specificity of higher than 90%, coefficient p value of discrimination expression of lower than 0.05, and Akaike's information criterion (AIC) of lower than 30.

[Criterion 3] Sensitivity of higher than 95%, specificity of higher than 85%, and coefficient p value of discrimination expression of lower than 0.05. [Criterion 4] Sensitivity of higher than 95%, specificity of higher than 85%, coefficient p value of discrimination expression of lower than 0.05, and AIC of lower than 30.

The top 10 CpG sites were selected for each of the four criteria, and 34 CpG sites (34 CpG sets) listed in Tables 8 to 10 were chosen. The results of the respective CpG sites are shown in Table 18.

[Table 18]

| CpG ID | Δβ value | Average β value (cancerous UC) n=24 | Average β value (non-cancerous UC) n=24 | p value (t assay, cancerous UC vs non-cancerous UC) | β value unbiased variance (cancerous UC) n=24 |
|---|---|---|---|---|---|
| cg24887265 | 0.30 | 0.61 ± 0.12 | 0.32 ± 0.13 | 9.0E-11 | 0.013 |
| cg10931190 | 0.21 | 0.39 ± 0.11 | 0.18 ± 0.07 | 8.1E-10 | 0.011 |
| cg22797031 | 0.27 | 0.73 ± 0.14 | 0.46 ± 0.12 | 2.4E-09 | 0.018 |
| cg22158650 | 0.26 | 0.52±0.13 | 0.26 ± 0.12 | 2.4E-09 | 0.016 |
| cg13677149 | 0.24 | 0.58 ± 0.12 | 0.34 ± 0.12 | 5.7E-09 | 0.014 |
| cg22795586 | 0.21 | 0.66 ± 0.11 | 0.45 ± 0.10 | 5.9E-09 | 0.012 |
| cg04389897 | 0.24 | 0.44 ± 0.13 | 0.21 ± 0.10 | 9.4E-09 | 0.017 |
| cg27651243 | 0.24 | 0.42 ± 0.13 | 0.18 ± 0.09 | 1.0E-08 | 0.018 |
| cg09765089 | 0.22 | 0.54 ± 0.10 | 0.31 ± 0.12 | 1.0E-08 | 0.011 |
| cg17542408 | 0.28 | 0.47 ± 0.17 | 0.19 ± 0.08 | 1.5E-08 | 0.028 |
| cg21229570 | 0.30 | 0.55 ± 0.18 | 0.25 ± 0.09 | 2.1E-08 | 0.033 |
| cg14394550 | 0.23 | 0.73 ± 0.12 | 0.50 ± 0.12 | 3.1E-08 | 0.015 |
| cg20326647 | -0.21 | 0.61 ± 0.12 | 0.81 ± 0.09 | 4.1E-08 | 0.015 |
| cg20373036 | 0.30 | 0.60 ± 0.15 | 0.30 ± 0.17 | 5.9E-08 | 0.023 |
| cg19968840 | 0.24 | 0.46 ± 0.16 | 0.21 ± 0.08 | 6.7E-08 | 0.024 |
| cg12162138 | 0.20 | 0.30 ± 0.13 | 0.09 ± 0.05 | 8.4E-08 | 0.017 |
| cg01307130 | 0.20 | 0.38 ± 0.13 | 0.18 ± 0.07 | 1.7E-07 | 0.018 |
| cg24960947 | 0.22 | 0.47 ± 0.14 | 0.25 ± 0.11 | 3.4E-07 | 0.021 |
| cg26074603 | 0.20 | 0.46 ± 0.14 | 0.25 ± 0.08 | 3.5E-07 | 0.020 |
| cg05575614 | 0.23 | 0.45 ± 0.14 | 0.22 ± 0.13 | 3.6E-07 | 0.020 |
| cg08309529 | 0.21 | 0.52 ± 0.14 | 0.31 ± 0.10 | 4.5E-07 | 0.019 |
| cg24879782 | 0.21 | 0.53 ± 0.14 | 0.32 ± 0.12 | 5.3E-07 | 0.018 |
| cg17538572 | 0.25 | 0.41 ± 0.18 | 0.17 ± 0.09 | 9.2E-07 | 0.032 |
| cg14516100 | 0.23 | 0.64 ± 0.15 | 0.41 ± 0.14 | 9.5E-07 | 0.022 |
| cg25740565 | 0.24 | 0.42 ± 0.19 | 0.18 ± 0.08 | 2.0E-06 | 0.035 |
| cg21045464 | 0.20 | 0.40 ± 0.16 | 0.20 ± 0.08 | 2.8E-06 | 0.025 |
| cg23955842 | 0.22 | 0.37 ± 0.16 | 0.14 ± 0.13 | 3.6E-06 | 0.026 |
| cg22964918 | 0.21 | 0.26 ± 0.18 | 0.05 ± 0.02 | 1.3E-05 | 0.032 |
| cg00061551 | 0.27 | 0.55 ± 0.25 | 0.28 ± 0.23 | 3.1E-04 | 0.063 |
| cg04610028 | 0.28 | 0.54 ± 0.30 | 0.26 ± 0.23 | 8.2E-04 | 0.088 |
| cg20139683 | 0.27 | 0.69 ± 0.29 | 0.41 ± 0.29 | 1.8E-03 | 0.083 |
| cg09549987 | -0.21 | 0.35 ± 0.28 | 0.56 ± 0.18 | 2.8E-03 | 0.077 |
| cg02299007 | -0.26 | 0.37 ± 0.33 | 0.63 ± 0.25 | 3.2E-03 | 0.107 |
| cg17917970 | 0.20 | 0.36 ± 0.32 | 0.16 ± 0.26 | 2.1E-02 | 0.103 |

(2) Multivariate analysis of clinical samples using CpG biomarker candidates

Cluster analysis and principal component analysis for all 48 samples were performed based on methylation levels of the 34 CpG sets. As a result, in the cluster analysis (FIG. 8A), a majority of UC cancer patient samples accumulated in the same cluster (within a frame, in the drawing). In addition, in the principal component analysis (FIG. 8B, the vertical axis is a second principal component), the UC cancer patient samples (●) and the non-cancerous UC patient samples (▲) each formed independent clusters, in a first principal component (horizontal axis) direction. That is, in any of the CpG sets, it was possible to clearly distinguish between the 24 UC cancer patient samples and the 24 non-cancerous UC patient samples.

(3) Evaluation of the likelihood of colorectal cancer development in clinical samples using CpG biomarker candidates

Accuracy of determination of the presence or absence of colorectal cancer development in an ulcerative colitis patient was investigated in a case where methylation rates of the three CpG sites of the CpG site (cg10931190) in the base sequence represented by SEQ ID NO: 34, the CpG site (cg13677149) in the base sequence represented by SEQ ID NO: 37, and the CpG site (cg14516100) in the base sequence represented by SEQ ID NO: 56 are used as markers, among the 34 CpG sets.

Specifically, based on a logistic regression model using numerical values ($\beta$ values) of methylation levels of the three CpG sites of specimens collected from the rectums of 24 UC cancerous patients and 24 non-cancerous UC patients, a discrimination expression was created to discriminate between UC cancerous patients and non-cancerous UC patients. As a result, sensitivity (proportion of patients evaluated as positive among the UC cancerous patients) was 95.8%, specificity (proportion of patients evaluated as negative among the non-cancerous UC patients) was 91.7%, positive predictive value (proportion of UC cancerous patients among patients evaluated as positive) was 92%, and negative predictive value (proportion of non-cancerous UC patients among patients evaluated as negative) was 95.6%, indicating that all were as high as 90% or more. In addition, FIG. 9 shows a receiver operating characteristic (ROC) curve. An AUC (area under the ROC curve) was 0.98. From these results, it was confirmed that the likelihood of colorectal cancer development in an ulcerative colitis patient can be evaluated with high sensitivity and high specificity based on methylation rates of several CpG sites selected from the 34 CpG sets.

[Example 3]

CpG biomarker candidates were extracted from the DNA methylation levels ($\beta$ values) of the respective CpG sites of the specimens collected from the rectums of ulcerative colitis patients obtained in Example 1 and the DNA methylation levels ($\beta$ values) of the respective CpG sites of ulcerative colitis patients obtained in Example 2.

(1) Extraction of CpG biomarker candidates

Specifically, 172 CpG sites with an absolute value of $\Delta\beta$ higher than 0.2 were extracted from 485,577 CpG sites. Subsequently, from the 172 CpG sites, two types of logistic regression models were created in the same manner as in Example 2, and the top 10 CpG sites were selected for each of the above four criteria. As a result, 18 CpG sites (18 CpG sets) listed in Tables 11 and 12 were chosen. The results of the respective CpG sites are shown in Table 19.

[Table 19]

| CpG ID | Δ β value | Average β value (cancerous UC) n=24 | Average β value (non-cancerous UC) n=24 | p value (t assay, cancerous UC vs non-cancerous UC) | β value unbiased variance (cancerous UC) n=24 |
|---|---|---|---|---|---|
| cg10339295 | -0.21 | 0.46±0.11 | 0.67±0.10 | 3.4E-11 | 0.013 |
| cg24887265 | 0.26 | 0.60±0.12 | 0.34±0.14 | 5.4E-11 | 0.014 |
| cg22797031 | 0.24 | 0.73±0.12 | 0.49±0.12 | 5.8E-11 | 0.014 |
| cg01736784 | 0.20 | 0.42±0.12 | 0.22±0.09 | 1.2E-10 | 0.013 |
| cg22158650 | 0.24 | 0.52±0.13 | 0.29±0.14 | 1.0E-09 | 0.016 |
| cg00723994 | 0.27 | 0.62±0.16 | 0.36±0.14 | 1.2E-09 | 0.024 |
| cg26315862 | 0.20 | 0.40±0.13 | 0.20±0.10 | 1.3E-09 | 0.016 |
| cg19937061 | 0.21 | 0.31±0.14 | 0.10±0.08 | 3.6E-09 | 0.021 |
| cg04004787 | 0.20 | 0.68±0.12 | 0.48±0.11 | 3.7E-09 | 0.015 |
| cg03409187 | 0.21 | 0.38±0.15 | 0.16±0.09 | 6.9E-09 | 0.023 |
| cg00282249 | 0.21 | 0.35±0.15 | 0.14±0.09 | 1.8E-08 | 0.022 |
| cg20148575 | 0.21 | 0.37±0.15 | 0.16±0.10 | 3.1E-08 | 0.024 |
| cg21229570 | 0.25 | 0.54±0.18 | 0.29±0.14 | 4.7E-08 | 0.033 |
| cg14416371 | 0.21 | 0.31±0.17 | 0.11±0.08 | 1.1E-07 | 0.028 |
| cg26081900 | -0.24 | 0.40±0.23 | 0.64±0.08 | 2.5E-06 | 0.054 |
| cg10168149 | 0.21 | 0.39±0.21 | 0.18±0.08 | 3.7E-06 | 0.042 |
| cg25366315 | -0.21 | 0.63±0.28 | 0.84±0.08 | 3.3E-04 | 0.080 |
| cg19850149 | -0.22 | 0.73±0.38 | 0.95±0.02 | 3.2E-03 | 0.146 |

(2) Multivariate analysis of clinical samples using CpG biomarker candidates

Based on the methylation levels of the 18 CpG sets, cluster analysis and principal component analysis for all 64 samples were performed. As a result, in the cluster analysis (FIG. 10A), a majority of UC cancer patient samples accumulated in the same cluster (within a frame, in the drawing). In addition, in the principal component analysis (FIG. 10B, the vertical axis is a second principal component), the UC cancer patient samples (●) and the non-cancerous UC patient samples (▲) each formed independent clusters, in a first principal component (horizontal axis) direction. That is, in any of the CpG sets, it was possible to clearly distinguish between the 32 UC cancer patient samples and the 32 non-cancerous UC patient samples.

[Example 4]

DMR biomarker candidates were extracted from an average methylation rate (average β value; additive average value of methylation levels (β values) of CpG sites present in each DMR) of each DMR of specimens collected from the rectums of 24 UC cancerous patients and 24 non-cancerous UC patients obtained in Example 2.

(1) Extraction of DMR biomarker candidates

Specifically, firstly, methylation data (IDAT format) of 485,577 CpG sites is input to the ChAMP pipeline (Bioinformatics, 30, 428, 2014; http://bioconductor.org/packages/release/bioc/html/ChAMP.html), and 2,549 DMR's determined as significant between the two groups of UC cancerous patients and non-cancerous UC patients were extracted. Among these, in a case of setting an absolute value of Δβ value ([average β value (cancerous UC)] - [average β value (non-cancerous UC)]) to higher than 0.15, narrowing-down to 39 locations occurred. Furthermore, among 484 sites where an absolute value of the Δβ value is higher than 0.1, 80 locations where the Δβ value of the UC cancerous patients and the non-cancerous UC patients obtained in Example 1 was higher than 0.15 were added, so that a total of 112 locations (DMR numbers 1 to 112) were set as DMR biomarker candidates. The results of the 112 DMR's (112 DMR sets) are shown in Tables 20 to 22.

[Table 20]

| DMR no. | Average β value (cancerous UC) n=24 | Average β value (non-cancerous UC) n=24 | Δ β value | 58DMR | 11DMR |
|---|---|---|---|---|---|
| 1 | 0.37±0.10 | 0.48±0.08 | -0.11 | # | # |
| 2 | 0.63±0.10 | 0.41 ±0.10 | 0.22 | # | # |
| 3 | 0.41±0.09 | 0.51±0.08 | -0.11 | # | # |
| 4 | 0.53±0.11 | 0.67±0.07 | -0.15 | # | # |
| 5 | 0.58±0.10 | 0.70±0.07 | -0.12 | # | # |
| 6 | 0.43±0.08 | 0.53±0.07 | -0.10 | # | # |
| 7 | 0.59±0.13 | 0.70±0.09 | -0.11 | # | # |
| 8 | 0.63±0.13 | 0.76±0.07 | -0.13 | # | # |
| 9 | 0.52±0.11 | 0.63±0.07 | -0.11 | # | # |
| 10 | 0.40±0.10 | 0.53±0.08 | -0.12 | # | # |
| 11 | 0.27±0.09 | 0.37±0.09 | -0.11 | # | # |
| 12 | 0.58±0.10 | 0.69±0.08 | -0.11 | # | |
| 13 | 0.43±0.09 | 0.54±0.09 | -0.11 | # | |
| 14 | 0.49±0.11 | 0.63±0.08 | -0.14 | # | |
| 15 | 0.47±0.12 | 0.61±0.10 | -0.14 | # | |
| 16 | 0.62±0.11 | 0.74±0.06 | -0.12 | # | |
| 17 | 0.55±0.12 | 0.67±0.08 | -0.12 | # | |
| 18 | 0.56±0.11 | 0.67±0.10 | -0.11 | # | |
| 19 | 0.54±0.12 | 0.69±0.08 | -0.15 | # | |
| 20 | 0.39±0.10 | 0.52±0.07 | -0.13 | # | |
| 21 | 0.28±0.14 | 0.12±0.06 | 0.16 | # | |
| 22 | 0.59±0.11 | 0.42±0.13 | 0.17 | # | |
| 23 | 045±0.10 | 0.59±0.07 | -0.13 | # | |
| 24 | 0.47±0.11 | 0.58±0.08 | -0.11 | # | |
| 25 | 0.45±0.10 | 0.59±0.08 | -0.14 | # | |
| 26 | 0.41±0.11 | 0.53±0.07 | -0.13 | # | |
| 27 | 0.60±0.09 | 0.71 ±0.07 | -0.11 | # | |
| 28 | 0.49±0.10 | 0.59±0.07 | -0.11 | # | |
| 29 | 0.47+0.11 | 0.31±0.10 | 0.16 | # | |
| 30 | 0.31±0.11 | 0.15±0.06 | 0.16 | # | |
| 31 | 0.47±0.11 | 0.58±0.07 | -0.11 | # | |
| 32 | 0.60±0.13 | 0.72±0.07 | -0.12 | # | |

(continued)

| DMR no. | Average β value (cancerous UC) n=24 | Average β value (non-cancerous UC) n=24 | Δ β value | 58DMR | 11DMR |
|---|---|---|---|---|---|
| 33 | 0.51 ±0.12 | 0.65±0.09 | -0.14 | # | |
| 34 | 0.46±0.11 | 0.59±010 | -0.12 | # | |
| 35 | 0.50±0.09 | 0.61±0.09 | -0.11 | # | |
| 36 | 0.34±0.09 | 0.46±0.07 | -0.12 | # | |
| 37 | 0.61±0.12 | 0.72±0.09 | -0.11 | # | |
| 38 | 0.55±0.10 | 0.65±0.10 | -0.11 | # | |

[Table 21]

| DMR no. | Average β value (cancerous UC) n=24 | Average β value (non-cancerous UC) n=24 | Δ β value | 58DMR | 11DMR |
|---|---|---|---|---|---|
| 39 | 0.44±0.11 | 0.55±0.09 | -0.11 | # | |
| 40 | 0.46±0.12 | 0.61±0.08 | -0.15 | # | |
| 41 | 0.49±0.15 | 0.65±0.10 | -0.15 | # | |
| 42 | 0.48±0.11 | 0.63±0.08 | -0.15 | # | |
| 43 | 0.53±0.10 | 0.65±0.08 | -0.11 | # | |
| 44 | 0.56±0.11 | 0.69±0.06 | -0.13 | # | |
| 45 | 0.53±0.10 | 0.66±0,07 | -0.13 | # | |
| 46 | 0.58±0.11 | 0.70±0.07 | -0.12 | # | |
| 47 | 0.36±0.13 | 0.24±0.09 | 0.12 | # | |
| 48 | 0.48±0.15 | 0.28±0.10 | 0.21 | # | |
| 49 | 0.52±0.12 | 0.64±0.08 | -0.12 | # | |
| 50 | 0.54±0.10 | 0.64±0.07 | -0.11 | # | |
| 51 | 0.62±0.14 | 0.75±0.07 | -0.13 | # | |
| 52 | 0.51±0.10 | 0.64±0.06 | -0.13 | # | |
| 53 | 0.33±0.11 | 0.48±0.10 | -0.15 | # | |
| 54 | 0.56±0.12 | 0.68±0.07 | -0.11 | # | |
| 55 | 0.56±0.13 | 0.70±0.07 | -0.14 | # | |
| 56 | 0.66±016 | 0.84±0.11 | -0.19 | # | |
| 57 | 0.57±0.10 | 0.67±0.07 | -0.10 | # | |
| 58 | 0.63±0.11 | 0.73±0.07 | -0.10 | # | |
| 59 | 0.56±0.12 | 0.68±0.08 | -0.12 | | |
| 60 | 0.52±0.14 | 0.65±0.07 | -0.13 | | |
| 61 | 0.28±0.10 | 0.18±0.08 | 0.10 | | |
| 62 | 0.54±0.12 | 0.66±0.09 | -0.13 | | |
| 63 | 0.35±0.12 | 0.19±0.13 | 0.15 | | |
| 64 | 0.41±0.08 | 0.26±0.09 | 0.15 | | |
| 65 | 0.50±0.10 | 0.62±0.09 | -0.12 | | |

(continued)

| DMR no. | Average β value (cancerous UC) n=24 | Average β value (non-cancerous UC) n=24 | Δ β value | 58DMR | 11DMR |
|---|---|---|---|---|---|
| 66 | 0.61±0.10 | 0.72±0.08 | -0.11 | | |
| 67 | 0.41±0.10 | 0.53±0.08 | -0.12 | | |
| 68 | 0.44±0.10 | 0.55±0.08 | -0.12 | | |
| 69 | 0.46±0.11 | 0.60±0.10 | -0.13 | | |
| 70 | 0.36±0.12 | 0.20±0.08 | 0.16 | | |
| 71 | 0.46±0.11 | 0.57±0.08 | -0.12 | | |
| 72 | 0.32±0.13 | 0.16±0.07 | 0.15 | | |
| 73 | 0.63±0.13 | 0.77±0.09 | -0.14 | | |
| 74 | 0.43±0.10 | 0.54±0.07 | -0.11 | | |
| 75 | 0.37±0.14 | 0.21±0.09 | 0.16 | | |
| 76 | 0.32±0.10 | 0.16±0.08 | 0.15 | | |

[Table 22]

| DMR no. | Average β value (cancerous UC) n=24 | Average β value (non-cancerous UC) n=24 | Δ β value | 58DMR | 11DMR |
|---|---|---|---|---|---|
| 77 | 0.54±0.12 | 0.39±0.12 | 0.15 | | |
| 78 | 0.26±0.12 | 0.10±0.05 | 0.16 | | |
| 79 | 0.44±0.11 | 0.58±0.10 | -0.14 | | |
| 80 | 0.33±0.15 | 0.17±0.08 | 0.16 | | |
| 81 | 0.27±0.09 | 0.39±0.08 | -0.13 | | |
| 82 | 0.30±0.08 | 0.41±0.07 | -0.11 | | |
| 83 | 0.45±0.13 | 0.27±0.08 | 0.18 | | |
| 84 | 0.59±0.09 | 0.71±0.06 | -0.12 | | |
| 85 | 0.42±0.12 | 0.25±0.08 | 0.17 | | |
| 86 | 0.44±0.10 | 0.57±0.08 | -0.12 | | |
| 87 | 0.46±0.10 | 0.57±0.07 | -0.11 | | |
| 88 | 0.43±0.13 | 0.25±0.07 | 0.18 | | |
| 89 | 0.34±0.14 | 0.18±0.10 | 0.16 | | |
| 90 | 0.65±0.13 | 0.78±0.07 | -0.13 | | |
| 91 | 0.62±0.12 | 0.73±0.05 | -0.11 | | |
| 92 | 0.39±0.09 | 0.50±0.08 | -0.10 | | |
| 93 | 0.68±0.10 | 0.53±0.11 | 0.15 | | |
| 94 | 0.31±0.15 | 0.15±0.06 | 0.15 | | |
| 95 | 0.45±0.11 | 0.60±0.10 | -0.15 | | |
| 96 | 0.31±0.15 | 0.15±0.04 | 0.16 | | |
| 97 | 0.33±0.15 | 0.16±0.06 | 0.16 | | |
| 98 | 0.46±0.11 | 0.30±0.09 | 0.16 | | |

(continued)

| DMR no. | Average β value (cancerous UC) n=24 | Average β value (non-cancerous UC) n=24 | Δ β value | 58DMR | 11DMR |
|---|---|---|---|---|---|
| 99 | 0.48±0.08 | 0.32±0.08 | 0.16 | | |
| 100 | 0.38±0.12 | 0.23±0.10 | 0.15 | | |
| 101 | 0.42±0.10 | 0.53±0.07 | -0.11 | | |
| 102 | 0.42±0.13 | 0.24±0.12 | 0.18 | | |
| 103 | 0.37±0.09 | 0.47±0.07 | -0.10 | | |
| 104 | 0.33±0.12 | 0.17±0.13 | 0.16 | | |
| 105 | 0.48±0.13 | 0.29±0.13 | 0.19 | | |
| 106 | 0.52±0.09 | 0.36±0.10 | 0.16 | | |
| 107 | 0.50±0.12 | 0.33±0.09 | 0.16 | | |
| 108 | 0.54±0.11 | 0.38±0.11 | 0.16 | | |
| 109 | 0.32±0.09 | 0.43±0.08 | -0.11 | | |
| 110 | 0.53±0.11 | 0.66±0.08 | -0.13 | | |
| 111 | 0.30±0.15 | 0.13±0.09 | 0.17 | | |
| 112 | 0.38±0.10 | 0.50±0.09 | -0.13 | | |

Next, 227,920 logistic regression models based on combinations of all three DMR's selected from the 112 DMR sets were created. Regarding the obtained discrimination expression, 79 discrimination expressions with sensitivity of 95% were chosen, in which 58 DMR's appeared (58 DMR in the tables). Furthermore, a frequency of DMR's appearing in the 79 discrimination expressions was obtained, and 11 DMR's appeared 4 times or more (11 DMR's, in the tables).

(2) Multivariate analysis of clinical samples using DMR biomarker candidates

Cluster analysis and principal component analysis for all 48 samples of Example 2 were performed based on the methylation rates of the 112 DMR sets. As a result, in cluster analysis, a majority of UC cancer patient samples accumulated in the same cluster (within a frame, in FIG. 11). In addition, in the principal component analysis (FIG. 12), the UC cancer patient samples (●) and the non-cancerous UC patient samples (▲) each formed independent clusters, in a first principal component (horizontal axis) direction.

(3) Evaluation of the likelihood of colorectal cancer development in clinical samples using DMR biomarker candidates

Accuracy of determination of the presence or absence of colorectal cancer development in an ulcerative colitis patient was investigated in a case where methylation rates in regions of DMR numbers 2 (SIX 10), 10 (CEP 112), and 55 (HNF 4 A) among the 112 DMR sets are used as markers.

Specifically, based on a logistic regression model using numerical values (β values) of methylation levels of the three DMR's of specimens collected from the rectums of 24 UC cancerous patients and 24 non-cancerous UC patients, a discrimination expression was created to discriminate between UC cancerous patients and non-cancerous UC patients. As a result, sensitivity (proportion of patients evaluated as positive among the UC cancerous patients) was 95.8%, specificity (proportion of patients evaluated as negative among the non-cancerous UC patients) was 95.8%, positive predictive value (proportion of UC cancerous patients among patients evaluated as positive) was 95.8%, and negative predictive value (proportion of non-cancerous UC patients among patients evaluated as negative) was 95.8%, indicating that all were as high as 95% or more. FIG. 13 shows a ROC curve. As a result, an AUC (area under the ROC curve) was 0.974. From these results, it was confirmed that the likelihood of colorectal cancer development in an ulcerative colitis patient can be evaluated with high sensitivity and high specificity based on methylation rates of several DMR's selected from the 112 DMR sets.

Reference Signs List

1: kit for collecting large intestinal mucosa
2, 2A, 2B, 2C: collection tool
3a: first clamping piece
3b: second clamping piece
4: connection portion
5: clamping surface
5a: first clamping surface
5b: second clamping surface
6a, 6a': side edge portion of first clamping surface 5a
6b, 6b': side edge portion of second clamping surface 5b
7a: first bending portion
7b: second bending portion
8a, 8b: protrusion portion
9a, 9b: cylindrical portion
10a: buffer portion
10b: elastic part
11, 11A, 11B: collection auxiliary tool
12: collection tool introduction portion
13: slit
14: gripping portion
15: tip end side edge portion
16: proximal side edge portion

Alternative expressions of the inventive concept are provided in the following clauses:

1. A method for determining the likelihood of colorectal cancer development in a human ulcerative colitis patient, the method comprising:

a measurement step of measuring methylation rates of one or more CpG sites present in respective differentially methylated regions represented by differentially methylated region numbers 1 to 112 listed in Tables 1 to 4, in DNA recovered from a biological sample collected from the human ulcerative colitis patient; and
a determination step of determining the likelihood of colorectal cancer development in the human ulcerative colitis patient, based on average methylation rates of the differentially methylated regions which are calculated based on the methylation rates measured in the measurement step and a preset reference value or a preset multivariate discrimination expression,
wherein the average methylation rate of the differentially methylated region is an average value of methylation rates of all CpG sites, for which the methylation rate is measured in the measurement step, among the CpG sites in the differentially methylated region,
the reference value is a value for identifying a cancerous ulcerative colitis patient and a non-cancerous ulcerative colitis patient, which is set for the average methylation rate of each differentially methylated region, and
the multivariate discrimination expression includes, as variables, average methylation rates of one or more differentially methylated regions among the differentially methylated regions represented by the differentially methylated region numbers 1 to 112.

[Table 1]

| DMR no. | Gene Symbol | Ensembl ID | Chromosome no. | DMR start | DMR end | Width | ± |
|---|---|---|---|---|---|---|---|
| 1 | MTMR11 | ENSG00000014914 | 1 | 149907598 | 149909051 | 1454 | − |
| 2 | SIX2 | ENSG00000170577 | 2 | 45233485 | 45233784 | 300 | + |
| 3 | COL3A1 | ENSG00000168542 | 2 | 189838986 | 189839961 | 976 | − |
| 4 | ARL14 | ENSG00000179674 | 3 | 160393670 | 160397766 | 4097 | − |
| 5 | S100P | ENSG00000163993 | 4 | 6695204 | 6695433 | 230 | − |
| 6 | VTRNA1-2 | ENSG00000202111 | 5 | 140098089 | 140099064 | 976 | − |
| 7 | PDGFA | ENSG00000197461 | 7 | 544037 | 545463 | 1427 | − |
| 8 | C9orf152 | ENSG00000188959 | 9 | 112970134 | 112970675 | 542 | − |
| 9 | TMPRSS4 | ENSG00000137648 | 11 | 117947606 | 117948147 | 542 | − |
| 10 | CEP112 | ENSG00000154240 | 17 | 63623628 | 63625636 | 2009 | − |
| 11 | ZMYND8 | ENSG00000101040 | 20 | 45946538 | 45947713 | 1176 | − |
| 12 | CASZ1 | ENSG00000130940 | 1 | 10839179 | 10839844 | 666 | − |
| 13 | KAZN | ENSG00000189337 | 1 | 15271343 | 15272595 | 1253 | − |
| 14 | RNF186; RP11-91K11.2 | ENSG00000178828; ENSG00000235434 | 1 | 20138780 | 20142876 | 4097 | − |
| 15 | SELENBP1 | ENSG00000143416 | 1 | 151344319 | 151345394 | 1076 | − |
| 16 | C1orf106 | ENSG00000163362 | 1 | 200862559 | 200865970 | 3412 | − |
| 17 | C4BPB | ENSG00000123843 | 1 | 207262158 | 207262699 | 542 | − |
| 18 | | ENSG00000224037 | 1 | 234851858 | 234853830 | 1973 | − |
| 19 | MALL | ENSG00000144063 | 2 | 110872470 | 110872878 | 409 | − |
| 20 | NOSTRIN | ENSG00000163072 | 2 | 169658610 | 169659453 | 844 | − |
| 21 | SATB2; SATB2-AS1 | ENSG00000119042; ENSG00000225953 | 2 | 200334655 | 200335051 | 397 | + |
| 22 | HDAC4 | ENSG00000068024 | 2 | 240174125 | 240175146 | 1022 | + |
| 23 | HRH1 | ENSG00000196639 | 3 | 11266750 | 11267368 | 619 | − |
| 24 | ATP13A4-AS1; ATP13A4 | ENSG00000225473; ENSG00000127249 | 3 | 193272384 | 193272925 | 542 | − |
| 25 | ARHGAP24 | ENSG00000138639 | 4 | 86748456 | 86749527 | 1072 | − |
| 26 | RP11-335O4.3; TRIM2 | ENSG00000235872; ENSG00000109654 | 4 | 154125233 | 154126208 | 976 | − |
| 27 | PDLIM3 | ENSG00000154553 | 4 | 186425209 | 186426241 | 1033 | − |
| 28 | FAM134B | ENSG00000154153 | 5 | 16508433 | 16509611 | 1179 | − |
| 29 | | ENSG00000222366 | 6 | 28944243 | 28946445 | 2203 | + |
| 30 | OR2I1P | ENSG00000237988 | 6 | 29520800 | 29521885 | 1086 | + |

[Table 2]

| DMR no. | Gene Symbol | Ensembl ID | Chromosome no. | DMR start | DMR end | Width | ± |
|---|---|---|---|---|---|---|---|
| 31 | FRK | ENSG00000111816 | 6 | 116381823 | 116382002 | 180 | − |
| 32 | IYD | ENSG00000009765 | 6 | 150689855 | 150690414 | 560 | − |
| 33 | SNX9 | ENSG00000130340 | 6 | 158374746 | 158376752 | 2007 | − |
| 34 | HOXA3 | ENSG00000243394; ENSG00000105997; ENSG00000240154 | 7 | 27154541 | 27155088 | 548 | − |
| 35 | DIP2C; PRR26 | ENSG00000151240; ENSG00000180525 | 10 | 695357 | 696843 | 1487 | − |
| 36 | TNKS1BP1 | ENSG00000149115 | 11 | 57087702 | 57091030 | 3329 | − |
| 37 | LRP5 | ENSG00000162337 | 11 | 68173589 | 68174773 | 1185 | − |
| 38 | LINC00940 | ENSG00000235049 | 12 | 2044784 | 2046983 | 2200 | − |
| 39 | DOCK9 | ENSG00000088387 | 13 | 99629723 | 99631071 | 1349 | − |
| 40 | IFI27 | ENSG00000165949 | 14 | 94576831 | 94577488 | 658 | − |
| 41 | TNFAIP2 | ENSG00000185215 | 14 | 103593425 | 103593599 | 175 | − |
| 42 | C14orf2 | ENSG00000156411 | 14 | 104354891 | 104357110 | 2220 | − |
| 43 | PRSS8 | ENSG00000052344 | 16 | 31146195 | 31147170 | 976 | − |
| 44 | | ENSG00000213472 | 16 | 57653646 | 57654187 | 542 | − |
| 45 | C16orf47 | ENSG00000197445 | 16 | 73205055 | 73208273 | 3219 | − |
| 46 | NOS2 | ENSG00000007171 | 17 | 26127399 | 26127624 | 226 | − |
| 47 | TTLL6 | ENSG00000170703 | 17 | 46827430 | 46827674 | 245 | + |
| 48 | SOX9-AS1 | ENSG00000234899 | 17 | 70214796 | 70217271 | 2476 | + |
| 49 | MISP | ENSG00000099812 | 19 | 750971 | 751512 | 542 | − |
| 50 | FXYD3 | ENSG00000089356 | 19 | 35606461 | 35607002 | 542 | − |
| 51 | LGALS4 | ENSG00000171747 | 19 | 39303428 | 39303969 | 542 | − |
| 52 | SULT2B1 | ENSG00000088002 | 19 | 49054848 | 49055525 | 678 | − |
| 53 | RIN2 | ENSG00000132669 | 20 | 19865804 | 19868083 | 2280 | − |
| 54 | SGK2 | ENSG00000101049 | 20 | 42187567 | 42188108 | 542 | − |
| 55 | HNF4A | ENSG00000101076 | 20 | 42984091 | 42985366 | 1276 | − |
| 56 | HNF4A | ENSG00000101076 | 20 | 43029911 | 43030079 | 169 | − |
| 57 | TFF1 | ENSG00000160182 | 21 | 43786546 | 43786709 | 164 | − |
| 58 | BAIAP2L2; PLA2G6 | ENSG00000128298; ENSG00000184381 | 22 | 38505808 | 38510180 | 4373 | − |
| 59 | RP3-395M20.3; PLCH2 | ENSG00000229393; ENSG00000149527 | 1 | 2425373 | 2426522 | 1150 | − |
| 60 | | ENSG00000184157 | 1 | 43751338 | 43751678 | 341 | − |

[Table 3]

| DMR no. | Gene Symbol | Ensembl ID | Chromosome no. | DMR start | DMR end | Width | ± |
|---|---|---|---|---|---|---|---|
| 61 | RP11-543D5.1 | ENSG00000227947 | 1 | 48190866 | 48191292 | 427 | + |
| 62 | B3GALT2; CDC73 | ENSG00000162630; ENSG00000134371 | 1 | 193154938 | 193155661 | 724 | − |
| 63 | AC016747.3; KIAA1841; C2orf74 | ENSG00000212978; ENSG00000162929; ENSG00000237651 | 2 | 61371986 | 61372587 | 602 | + |
| 64 | AC007392.3 | ENSG00000232046 | 2 | 66809757 | 66810771 | 1015 | + |
| 65 | KCNE4 | ENSG00000152049 | 2 | 223916558 | 223916687 | 130 | − |
| 66 | AGAP1 | ENSG00000157985 | 2 | 236444053 | 236444434 | 382 | − |
| 67 | PPP2R3A | ENSG00000073711 | 3 | 135684043 | 135684227 | 185 | − |
| 68 | APOD | ENSG00000189058 | 3 | 195310802 | 195311018 | 217 | − |
| 69 | MUC4 | ENSG00000145113 | 3 | 195536032 | 195537321 | 1290 | − |
| 70 | MCIDAS | ENSG00000234602 | 5 | 54518579 | 54519189 | 611 | + |
| 71 | OCLN | ENSG00000197822 | 5 | 68787631 | 68787825 | 195 | − |
| 72 | PCDHGA2; NA | ENSG00000081853; ENSG00000241325 | 5 | 140797155 | 140797364 | 210 | + |
| 73 | C6orf195 | ENSG00000164385 | 6 | 2514359 | 2516276 | 1918 | − |
| 74 | | ENSG00000196333 | 6 | 19179779 | 19182021 | 2243 | − |
| 75 | HCG16 | ENSG00000244349 | 6 | 28956144 | 28956970 | 827 | + |
| 76 | HCG9 | ENSG00000204625 | 6 | 29943251 | 29943629 | 379 | + |
| 77 | RNF39 | ENSG00000204618 | 6 | 30039051 | 30039749 | 699 | + |
| 78 | SLC22A16 | ENSG00000004809 | 6 | 110797397 | 110797584 | 188 | + |
| 79 | PARK2 | ENSG00000185345 | 6 | 161796297 | 161797341 | 1045 | − |
| 80 | WBSCR17 | ENSG00000185274 | 7 | 70597038 | 70597093 | 56 | + |
| 81 | RN7SL76P | ENSG00000241959 | 7 | 151156201 | 151158179 | 1979 | − |
| 82 | SPIDR | ENSG00000164808 | 8 | 48571960 | 48573044 | 1085 | − |
| 83 | CA3 | ENSG00000164879 | 8 | 86350503 | 86350656 | 154 | + |
| 84 | PPP1R16A; GPT | ENSG00000160972; ENSG00000167701 | 8 | 145728374 | 145729865 | 1492 | − |
| 85 | NPY4R | ENSG00000204174 | 10 | 47083219 | 47083381 | 163 | + |
| 86 | C10orf107 | ENSG00000183346 | 10 | 63422447 | 63422576 | 130 | − |
| 87 | LINC00857 | ENSG00000237523 | 10 | 81967370 | 81967832 | 463 | − |
| 88 | VAX1 | ENSG00000148704 | 10 | 118891415 | 118891890 | 476 | + |
| 89 | TACC2 | ENSG00000138162 | 10 | 123922971 | 123923178 | 208 | + |
| 90 | MUC2 | ENSG00000198788 | 11 | 1058891 | 1062477 | 3587 | − |

[Table 4]

| DMR no. | Gene Symbol | Ensembl ID | Chromosome no. | DMR start | DMR end | Width | ± |
|---|---|---|---|---|---|---|---|
| 91 | MUC2 | ENSG00000198788 | 11 | 1074614 | 1075155 | 542 | − |
| 92 | TEAD1 | ENSG00000187079 | 11 | 12697507 | 12701324 | 3818 | − |
| 93 | RP11-121M22.1 | ENSG00000175773 | 11 | 130270828 | 130272842 | 2015 | + |
| 94 | KCNC2 | ENSG00000166006 | 12 | 75601683 | 75601943 | 261 | + |
| 95 | NCOR2 | ENSG00000196498 | 12 | 124906454 | 124908279 | 1826 | − |
| 96 | PDX1 | ENSG00000139515 | 13 | 28498306 | 28498463 | 158 | + |
| 97 | PDX1 | ENSG00000139515 | 13 | 28500855 | 28501186 | 332 | + |
| 98 | | ENSG00000198348 | 14 | 101922989 | 101923532 | 544 | + |
| 99 | MEIS2 | ENSG00000134138 | 15 | 37387445 | 37387655 | 211 | + |
| 100 | CCDC64B | ENSG00000162069 | 16 | 3079798 | 3080032 | 235 | + |
| 101 | ADCY9 | ENSG00000162104 | 16 | 3999535 | 4001924 | 2390 | − |
| 102 | | ENSG00000227093 | 16 | 54407005 | 54408952 | 1948 | + |
| 103 | GRB7 | ENSG00000141738 | 17 | 37895616 | 37896445 | 830 | − |
| 104 | RAPGEFL1 | ENSG00000108352 | 17 | 38347581 | 38347738 | 158 | + |
| 105 | WNK4 | ENSG00000126562 | 17 | 40936617 | 40936916 | 300 | + |
| 106 | HOXB6; HOXB-AS3 | ENSG00000239558; ENSG00000108511; ENSG00000233101 | 17 | 46674245 | 46674664 | 420 | + |
| 107 | CHAD; ACSF2 | ENSG00000136457; ENSG00000167107 | 17 | 48546115 | 48546272 | 158 | + |
| 108 | | ENSG00000230792 | 17 | 55212625 | 55214595 | 1971 | + |
| 109 | | ENSG00000171282 | 17 | 79393453 | 79393610 | 158 | − |
| 110 | TPM4 | ENSG00000167460 | 19 | 16178026 | 16178163 | 138 | − |
| 111 | | ENSG00000248094 | 19 | 21646440 | 21646771 | 332 | + |
| 112 | RP6-109B7.4; MIRLET7BHG | ENSG00000235159; ENSG00000197182; ENSG00000245020 | 22 | 46461776 | 46465514 | 3739 | − |

2. The method for determining the likelihood of colorectal cancer development according to clause 1, wherein in the determination step, in a case where one or more among the differentially methylated regions represented by differentially methylated region numbers 1, 3 to 20, 23 to 28, 31 to 46, 49 to 60, 62, 65 to 69, 71, 73, 74, 79, 81, 82, 84, 86, 87, 90 to 92, 95, 101, 103, 109, 110, and 112 have an average methylation rate of equal to or lower than the preset reference value, or one or more among the differentially methylated regions represented by differentially methylated region numbers 2, 21, 22, 29, 30, 47, 48, 61, 63, 64, 70, 72, 75 to 78, 80, 83, 85, 88, 89, 93, 94, 96 to 100, 102, 104 to 108, and 111 have an average methylation rate of equal to or higher than the preset reference value, it is determined that there is a high likelihood of colorectal cancer development in the human ulcerative colitis patient.

3. The method for determining the likelihood of colorectal cancer development according to clause 1, wherein in the measurement step, the methylation rates of the one or more CpG sites present in the differentially methylated region, of which an average methylation rate is included as a variable in the multivariate discrimination expression, are measured, and

in the determination step, in a case where based on the average methylation rate of the differentially methylated region calculated based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.

4. The method for determining the likelihood of colorectal cancer development according to clause 3,
wherein the multivariate discrimination expression includes, as variables, average methylation rates of two or more differentially methylated regions selected from the differentially methylated regions represented by the differentially methylated region numbers 1 to 112.

5. The method for determining the likelihood of colorectal cancer development according to clause 3,
wherein the multivariate discrimination expression includes, as variables, average methylation rates of three or more differentially methylated regions selected from the differentially methylated regions represented by the differentially methylated region numbers 1 to 112.

6. The method for determining the likelihood of colorectal cancer development according to clause 3,
wherein the multivariate discrimination expression includes, as variables, average methylation rate of one or more differentially methylated regions selected from the group consisting of the differentially methylated regions represented by the differentially methylated region numbers 1 to 58.

7. The method for determining the likelihood of colorectal cancer development according to clause 3,
wherein the multivariate discrimination expression includes, as variables, average methylation rates of one or more differentially methylated regions selected from the group consisting of the differentially methylated regions represented by the differentially methylated region numbers 1 to 11.

8. A method for determining the likelihood of colorectal cancer development in a human ulcerative colitis patient, the method comprising:

a measurement step of measuring methylation rates of one or more CpG sites selected from the group consisting of CpG sites in base sequences represented by SEQ ID NOs: 1 to 80, in DNA recovered from a biological sample collected from the human ulcerative colitis patient; and
a determination step of determining the likelihood of colorectal cancer development in the human ulcerative colitis patient, based on the methylation rates measured in the measurement step and a preset reference value or a preset multivariate discrimination expression,
wherein the reference value is a value for identifying a cancerous ulcerative colitis patient and a non-cancerous ulcerative colitis patient, which is set for the methylation rate of each CpG site, and
the multivariate discrimination expression includes, as a variable, a methylation rate of at least one CpG site among the CpG sites in the base sequences represented by SEQ ID NOs: 1 to 80.

9. The method for determining the likelihood of colorectal cancer development according to clause 8,
wherein in the measurement step, methylation rates of 2 to 10 CpG sites are measured.

10. The method for determining the likelihood of colorectal cancer development according to clause 8 or 9,
wherein in the determination step, in a case where one or more among CpG sites in the base sequences represented by SEQ ID NOs: 1, 2, 11, 12, 14 to 18, 21 to 24, 26, 27, 29, 31, 45, 64, 65, 67, 77, 79, and 80 have a methylation rate of equal to or lower than the preset reference value, or one or more among CpG sites in the base sequences represented by SEQ ID NOs: 3 to 10, 13, 19, 20, 25, 28, 30, 32 to 44, 46 to 63, 66, 68 to 76, and 78 have a methylation rate of equal to or higher than the preset reference value, it is determined that there is a high likelihood of colorectal cancer development in the human ulcerative colitis patient.

11. The method for determining the likelihood of colorectal cancer development according to any one of clauses 8 to 10,
wherein in the measurement step, methylation rates of CpG sites in the base sequences represented by SEQ ID NOs: 1 to 32 are measured, and
in the determination step, in a case where one or more among CpG sites in the base sequences represented by SEQ ID NOs: 1, 2, 11, 12, 14 to 18, 21 to 24, 26, 27, 29, and 31 have a methylation rate of equal to or lower than the preset reference value, or one or more among CpG sites in the base sequences represented by SEQ ID NOs:

3 to 10, 13, 19, 20, 25, 28, 30, and 32 have a methylation rate of equal to or higher than the preset reference value, it is determined that there is a high likelihood of colorectal cancer development in the human ulcerative colitis patient.

12. The method for determining the likelihood of colorectal cancer development according to any one of clauses 8 to 11,
wherein in the determination step, in a case where a sum of the number of CpG sites having a methylation rate equal to or lower than the preset reference value among CpG sites in the base sequences represented by SEQ ID NOs: 1, 2, 11, 12, 14 to 18, 21 to 24, 26, 27, 29 and 31, and the number of CpG sites having a methylation rate equal to or higher than the preset reference value among CpG sites in the base sequences represented by SEQ ID NOs: 3 to 10, 13, 19, 20, 25, 28, 30, and 32 is three or more, it is determined that there is a high likelihood of colorectal cancer development in the human ulcerative colitis patient.

13. The method for determining the likelihood of colorectal cancer development according to any one of clauses 8 to 10,
wherein in the measurement step, methylation rates of CpG sites in the base sequences represented by SEQ ID NOs: 1 to 16 are measured, and
in the determination step, in a case where one or more among CpG sites in the base sequences represented by SEQ ID NOs: 1, 2, 11, 12, and 14 to 16 have a methylation rate of equal to or lower than the preset reference value, or one or more among CpG sites in the base sequences represented by SEQ ID NOs: 3 to 10 and 13 have a methylation rate of equal to or higher than the preset reference value, it is determined that there is a high likelihood of colorectal cancer development in the human ulcerative colitis patient.

14. The method for determining the likelihood of colorectal cancer development according to any one of clauses 8 to 10 and 13,
wherein in the determination step, in a case where a sum of the number of CpG sites having a methylation rate equal to or lower than the preset reference value among CpG sites in the base sequences represented by SEQ ID NOs: 1, 2, 11, 12, and 14 to 16, and the number of CpG sites having a methylation rate equal to or higher than the preset reference value among CpG sites in the base sequences represented by SEQ ID NOs: 3 to 10 and 13 is three or more, it is determined that there is a high likelihood of colorectal cancer development in the human ulcerative colitis patient.

15. The method for determining the likelihood of colorectal cancer development according to any one of clauses 8 to 10,
wherein in the measurement step, methylation rates of CpG sites in the base sequences represented by SEQ ID NOs: 1 to 9 are measured, and
in the determination step, in a case where one or more among CpG sites in the base sequences represented by SEQ ID NOs: 1 and 2 have a methylation rate of equal to or lower than the preset reference value, or one or more among CpG sites in the base sequences represented by SEQ ID NOs: 3 to 9 have a methylation rate of equal to or higher than the preset reference value, it is determined that there is a high likelihood of colorectal cancer development in the human ulcerative colitis patient.

16. The method for determining the likelihood of colorectal cancer development according to any one of clauses 8 to 10 and 15,
wherein in the determination step, in a case where a sum of the number of CpG sites having a methylation rate equal to or lower than the preset reference value among CpG sites in the base sequences represented by SEQ ID NOs: 1 and 2, and the number of CpG sites having a methylation rate equal to or higher than the preset reference value among CpG sites in the base sequences represented by SEQ ID NOs: 3 to 9 is three or more, it is determined that there is a high likelihood of colorectal cancer development in the human ulcerative colitis patient.

17. The method for determining the likelihood of colorectal cancer development according to any one of clauses 8 to 10,
wherein in the measurement step, methylation rates of one or more CpG sites selected from the group consisting of CpG sites in the base sequences represented by SEQ ID NOs: 33 to 66 are measured, and
in the determination step, in a case where one or more among CpG sites in the base sequences represented by SEQ ID NOs: 45, 64, and 65 have a methylation rate of equal to or lower than the preset reference value, or one or more among CpG sites in the base sequences represented by SEQ ID NOs: 32 to 44, 46 to 63, and 66 have a methylation rate of equal to or higher than the preset reference value, it is determined that there is a high likelihood of colorectal cancer development in the human ulcerative colitis patient.

18. The method for determining the likelihood of colorectal cancer development according to any one of clauses 8 to 10 and 17,
wherein in the determination step, in a case where a sum of the number of CpG sites having a methylation rate equal to or lower than the preset reference value among CpG sites in the base sequences represented by SEQ ID NOs: 45, 64, and 65, and the number of CpG sites having a methylation rate equal to or higher than the preset reference value among CpG sites in the base sequences represented by SEQ ID NOs: 32 to 44, 46 to 63, and 66 is two or more, it is determined that there is a high likelihood of colorectal cancer development in the human ulcerative colitis patient.

19. The method for determining the likelihood of colorectal cancer development according to any one of clauses 8 to 10,
wherein in the measurement step, methylation rates of one or more CpG sites selected from the group consisting of CpG sites in the base sequences represented by SEQ ID NOs: 33, 35, 36, 43, and 67 to 80 are measured, and in the determination step, in a case where one or more among CpG sites in the base sequences represented by SEQ ID NOs: 67, 77, 79, and 80 have a methylation rate of equal to or lower than the preset reference value, or one or more among CpG sites in the base sequences represented by SEQ ID NOs: 33, 35, 36, 43, 68 to 76, and 78 have a methylation rate of equal to or higher than the preset reference value, it is determined that there is a high likelihood of colorectal cancer development in the human ulcerative colitis patient.

20. The method for determining the likelihood of colorectal cancer development according to any one of clauses 8 to 10 and 19,
wherein in the determination step, in a case where a sum of the number of CpG sites having a methylation rate equal to or lower than the preset reference value among CpG sites in the base sequences represented by SEQ ID NOs: 67, 77, 79, and 80, and the number of CpG sites having a methylation rate equal to or higher than the preset reference value among CpG sites in the base sequences represented by SEQ ID NOs: 33, 35, 36, 43, 68 to 76, and 78 is two or more, it is determined that there is a high likelihood of colorectal cancer development in the human ulcerative colitis patient.

21. The method for determining the likelihood of colorectal cancer development according to clause 12, 14, 16, 18, or 20,
wherein in a case where the sum is five or more, it is determined that there is a high likelihood of colorectal cancer development in the human ulcerative colitis patient.

22. The method for determining the likelihood of colorectal cancer development according to clause 8 or 9,
wherein the multivariate discrimination expression includes, as variables, methylation rates of one or more CpG sites selected from the group consisting of CpG sites in the base sequences represented by SEQ ID NOs: 33 to 66, in the measurement step, a methylation rate of the CpG site which is included as a variable in the multivariate discrimination expression is measured, and
in the determination step, in a case where based on the methylation rate measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.

23. The method for determining the likelihood of colorectal cancer development according to clause 8 or 9,
wherein the multivariate discrimination expression includes, as variables, methylation rates of one or more CpG sites selected from the group consisting of CpG sites in the base sequences represented by SEQ ID NOs: 33, 35, 36, 43, and 67 to 80,
in the measurement step, a methylation rate of the CpG site which is included as a variable in the multivariate discrimination expression is measured, and
in the determination step, in a case where based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.

24. The method for determining the likelihood of colorectal cancer development according to any one of clauses 1 to 23,

wherein the multivariate discrimination expression is a logistic regression expression, a linear discrimination expression, an expression created by Naive Bayes classifier, or an expression created by Support Vector Machine.

25. The method for determining the likelihood of colorectal cancer development according to any one of clauses 1 to 24,
wherein the biological sample is intestinal tract tissue.

26. The method for determining the likelihood of colorectal cancer development according to any one of clauses 1 to 25,
wherein the biological sample is rectal mucosal tissue.

27. The method for determining the likelihood of colorectal cancer development according to clause 26,
wherein the rectal mucosal tissue is collected by a kit for collecting large intestinal mucosa which includes a collection tool and a collection auxiliary tool,
the collection tool has

a first plate-like clamping piece with a first clamping surface for clamping large intestinal mucosa formed at one end thereof,
a second plate-like clamping piece with a second clamping surface for clamping large intestinal mucosa formed at one end thereof, and
a connection portion that connects the first clamping piece and the second clamping piece in a mutually opposed state at an end portion where the first clamping surface and the second clamping surface are not formed,

at least one of the first clamping surface and the second clamping surface is cup-shaped,
the collection auxiliary tool has

a truncated cone-shaped collection tool introduction portion having a slit on a side wall, and
a rod-like gripping portion,

one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width of one end portion of the first clamping piece and one end portion of the second clamping piece, and
the collection tool introduction portion has a larger outer diameter of 30 to 70 mm and a length in a rotation axis direction of 50 to 150 mm.

28. The method for determining the likelihood of colorectal cancer development according to clause 27,
wherein the collection tool has
a first bending portion on a side of an end portion where the first clamping surface is formed, rather than a center portion of the first clamping piece, and
a second bending portion on a side of an end portion where the second clamping surface is formed, rather than a center portion of the second clamping piece.

29. A kit for collecting large intestinal mucosa, comprising:

a collection tool; and
a collection auxiliary tool,
wherein the collection tool has

a first plate-like clamping piece with a first clamping surface for clamping large intestinal mucosa formed at one end thereof,
a second plate-like clamping piece with a second clamping surface for clamping large intestinal mucosa formed at one end thereof, and
a connection portion that connects the first clamping piece and the second clamping piece in a mutually opposed state at an end portion where the first clamping surface and the second clamping surface are not formed,

at least one of the first clamping surface and the second clamping surface is cup-shaped,
the collection auxiliary tool has

a truncated cone-shaped collection tool introduction portion having a slit on a side wall, and
a rod-like gripping portion,

one end of the gripping portion is connected in the vicinity of a side edge portion having a larger outer diameter of the collection tool introduction portion,
the slit is provided from a side edge portion having a smaller outer diameter of the collection tool introduction portion toward the side edge portion having a larger outer diameter,
a width of the slit is wider than a width of one end portion of the first clamping piece and one end portion of the second clamping piece, and
the collection tool introduction portion has a larger outer diameter of 30 to 70 mm and a length in a rotation axis direction of 50 to 150 mm.

30. The kit for collecting large intestinal mucosa according to clause 29,
wherein the collection tool has
a first bending portion on a side of an end portion where the first clamping surface is formed, rather than a center portion of the first clamping piece, and
a second bending portion on a side of an end portion where the second clamping surface is formed, rather than a center portion of the second clamping piece.

31. The kit for collecting large intestinal mucosa according to clause 29 or 30,
wherein both the first clamping surface and the second clamping surface are cup-shaped.

32. The kit for collecting large intestinal mucosa according to any one of clauses 29 to 31,
wherein the collection auxiliary tool has a through-hole in a rotation axis direction, and the collection tool introduction portion has a larger outer diameter of 30 to 70 mm and a length in a rotation axis direction of 50 to 150 mm, and the cup-shaped side edge portion has an inner diameter of 2 to 3 mm.

33. The kit for collecting large intestinal mucosa according to any one of clauses 29 to 32,
wherein the first clamping surface and the second clamping surface have serrated side edge portions.

34. A marker for analyzing a DNA methylation rate, comprising:

a DNA fragment having a partial base sequence containing one or more CpG sites selected from the group consisting of CpG sites in the base sequences represented by SEQ ID NOs: 1 to 80,
wherein the marker is used to determine the likelihood of colorectal cancer development in an ulcerative colitis patient.

## Claims

1. A method for determining the likelihood of colorectal cancer development in a human ulcerative colitis patient, the method comprising:

   a measurement step of measuring methylation rates of three or more CpG sites present in respective differentially methylated regions selected from the differentially methylated regions represented by differentially methylated region numbers 1 to 112 listed in Tables 1 to 4, in DNA recovered from a biological sample collected from the human ulcerative colitis patient; and

   a determination step of determining the likelihood of colorectal cancer development in the human ulcerative colitis patient, based on average methylation rates of the differentially methylated regions which are calculated based on the methylation rates measured in the measurement step and a preset reference value or a preset multivariate discrimination expression,

   wherein the average methylation rate of the differentially methylated region is an average value of methylation rates of all CpG sites, for which the methylation rate is measured in the measurement step, among the CpG sites in the differentially methylated region,

   the reference value is a value for identifying a cancerous ulcerative colitis patient and a non-cancerous ulcerative colitis patient, which is set for the average methylation rate of each differentially methylated region, and the multivariate discrimination expression includes, as variables, average methylation rates of the selected differentially methylated regions, wherein the selected differentially methylated regions comprise differentially methylated regions represented by the differentially methylated region numbers 21, 77 and 107; 14, 21 and 77; 14, 21 and 107; or 14, 77 and 107.

[Table 1]

| DMR no. | Gene Symbol | Ensembl ID | Chromosome no. | DMR start | DMR end | Width | ± |
|---|---|---|---|---|---|---|---|
| 1 | MTMR11 | ENSG00000014914 | 1 | 149907598 | 149909051 | 1454 | − |
| 2 | SIX2 | ENSG00000170577 | 2 | 45233485 | 45233784 | 300 | + |
| 3 | COL3A1 | ENSG00000168542 | 2 | 189838986 | 189839961 | 976 | − |
| 4 | ARL14 | ENSG00000179674 | 3 | 160393670 | 160397766 | 4097 | − |
| 5 | S100P | ENSG00000163993 | 4 | 6695204 | 6695433 | 230 | − |
| 6 | VTRNA1-2 | ENSG00000202111 | 5 | 140098089 | 140099064 | 976 | − |
| 7 | PDGFA | ENSG00000197461 | 7 | 544037 | 545463 | 1427 | − |
| 8 | C9orf152 | ENSG00000188959 | 9 | 112970134 | 112970675 | 542 | − |
| 9 | TMPRSS4 | ENSG00000137648 | 11 | 117947606 | 117948147 | 542 | − |
| 10 | CEP112 | ENSG00000154240 | 17 | 63623628 | 63625636 | 2009 | − |
| 11 | ZMYND8 | ENSG00000101040 | 20 | 45946538 | 45947713 | 1176 | − |
| 12 | CASZ1 | ENSG00000130940 | 1 | 10839179 | 10839844 | 666 | − |
| 13 | KAZN | ENSG00000189337 | 1 | 15271343 | 15272595 | 1253 | − |
| 14 | RNF186; RP11-91K11.2 | ENSG00000178828; ENSG00000235434 | 1 | 20138780 | 20142876 | 4097 | − |
| 15 | SELENBP1 | ENSG00000143416 | 1 | 151344319 | 151345394 | 1076 | − |
| 16 | C1orf106 | ENSG00000163362 | 1 | 200862559 | 200865970 | 3412 | − |
| 17 | C4BPB | ENSG00000123843 | 1 | 207262158 | 207262699 | 542 | − |
| 18 |  | ENSG00000224037 | 1 | 234851858 | 234853830 | 1973 | − |
| 19 | MALL | ENSG00000144063 | 2 | 110872470 | 110872878 | 409 | − |
| 20 | NOSTRIN | ENSG00000163072 | 2 | 169658610 | 169659453 | 844 | − |
| 21 | SATB2; SATB2-AS1 | ENSG00000119042; ENSG00000225953 | 2 | 200334655 | 200335051 | 397 | + |
| 22 | HDAC4 | ENSG00000068024 | 2 | 240174125 | 240175146 | 1022 | + |
| 23 | HRH1 | ENSG00000196639 | 3 | 11266750 | 11267368 | 619 | − |
| 24 | ATP13A4-AS1; ATP13A4 | ENSG00000225473; ENSG00000127249 | 3 | 193272384 | 193272925 | 542 | − |
| 25 | ARHGAP24 | ENSG00000138639 | 4 | 86748456 | 86749527 | 1072 | − |
| 26 | RP11-335O4.3; TRIM2 | ENSG00000235872; ENSG00000109654 | 4 | 154125233 | 154126208 | 976 | − |
| 27 | PDLIM3 | ENSG00000154553 | 4 | 186425209 | 186426241 | 1033 | − |
| 28 | FAM134B | ENSG00000154153 | 5 | 16508433 | 16509611 | 1179 | − |
| 29 |  | ENSG00000222366 | 6 | 28944243 | 28946445 | 2203 | + |
| 30 | OR2I1P | ENSG00000237988 | 6 | 29520800 | 29521885 | 1086 | + |

[Table 2]

| DMR no. | Gene Symbol | Ensembl ID | Chromosome no. | DMR start | DMR end | Width | ± |
|---|---|---|---|---|---|---|---|
| 31 | FRK | ENSG00000111816 | 6 | 116381823 | 116382002 | 180 | − |
| 32 | IYD | ENSG00000009765 | 6 | 150689855 | 150690414 | 560 | − |
| 33 | SNX9 | ENSG00000130340 | 6 | 158374746 | 158376752 | 2007 | − |
| 34 | HOXA3 | ENSG00000243394; ENSG00000105997; ENSG00000240154 | 7 | 27154541 | 27155088 | 548 | − |
| 35 | DIP2C; PRR26 | ENSG00000151240; ENSG00000180525 | 10 | 695357 | 696843 | 1487 | − |
| 36 | TNKS1BP1 | ENSG00000149115 | 11 | 57087702 | 57091030 | 3329 | − |
| 37 | LRP5 | ENSG00000162337 | 11 | 68173589 | 68174773 | 1185 | − |
| 38 | LINC00940 | ENSG00000235049 | 12 | 2044784 | 2046983 | 2200 | − |
| 39 | DOCK9 | ENSG00000088387 | 13 | 99629723 | 99631071 | 1349 | − |
| 40 | IFI27 | ENSG00000165949 | 14 | 94576831 | 94577488 | 658 | − |
| 41 | TNFAIP2 | ENSG00000185215 | 14 | 103593425 | 103593599 | 175 | − |
| 42 | C14orf2 | ENSG00000156411 | 14 | 104354891 | 104357110 | 2220 | − |
| 43 | PRSS8 | ENSG00000052344 | 16 | 31146195 | 31147170 | 976 | − |
| 44 | | ENSG00000213472 | 16 | 57653646 | 57654187 | 542 | − |
| 45 | C16orf47 | ENSG00000197445 | 16 | 73205055 | 73208273 | 3219 | − |
| 46 | NOS2 | ENSG00000007171 | 17 | 26127399 | 26127624 | 226 | − |
| 47 | TTLL6 | ENSG00000170703 | 17 | 46827430 | 46827674 | 245 | + |
| 48 | SOX9-AS1 | ENSG00000234899 | 17 | 70214796 | 70217271 | 2476 | + |
| 49 | MISP | ENSG00000099812 | 19 | 750971 | 751512 | 542 | − |
| 50 | FXYD3 | ENSG00000089356 | 19 | 35606461 | 35607002 | 542 | − |
| 51 | LGALS4 | ENSG00000171747 | 19 | 39303428 | 39303969 | 542 | − |
| 52 | SULT2B1 | ENSG00000088002 | 19 | 49054848 | 49055525 | 678 | − |
| 53 | RIN2 | ENSG00000132669 | 20 | 19865804 | 19868083 | 2280 | − |
| 54 | SGK2 | ENSG00000101049 | 20 | 42187567 | 42188108 | 542 | − |
| 55 | HNF4A | ENSG00000101076 | 20 | 42984091 | 42985366 | 1276 | − |
| 56 | HNF4A | ENSG00000101076 | 20 | 43029911 | 43030079 | 169 | − |
| 57 | TFF1 | ENSG00000160182 | 21 | 43786546 | 43786709 | 164 | − |
| 58 | BAIAP2L2; PLA2G6 | ENSG00000128298; ENSG00000184381 | 22 | 38505808 | 38510180 | 4373 | − |
| 59 | RP3-395M20.3; PLCH2 | ENSG00000229393; ENSG00000149527 | 1 | 2425373 | 2426522 | 1150 | − |
| 60 | | ENSG00000184157 | 1 | 43751338 | 43751678 | 341 | − |

[Table 3]

| DMR no. | Gene Symbol | Ensembl ID | Chromosome no. | DMR start | DMR end | Width | ± |
|---|---|---|---|---|---|---|---|
| 61 | RP11-543D5.1 | ENSG00000227947 | 1 | 48190866 | 48191292 | 427 | + |
| 62 | B3GALT2;<br>CDC73 | ENSG00000162630;<br>ENSG00000134371 | 1 | 193154938 | 193155661 | 724 | − |
| 63 | AC016747.3;<br>KIAA1841;<br>C2orf74 | ENSG00000212978;<br>ENSG00000162929;<br>ENSG00000237651 | 2 | 61371986 | 61372587 | 602 | + |
| 64 | AC007392.3 | ENSG00000232046 | 2 | 66809757 | 66810771 | 1015 | + |
| 65 | KCNE4 | ENSG00000152049 | 2 | 223916558 | 223916687 | 130 | − |
| 66 | AGAP1 | ENSG00000157985 | 2 | 236444053 | 236444434 | 382 | − |
| 67 | PPP2R3A | ENSG00000073711 | 3 | 135684043 | 135684227 | 185 | − |
| 68 | APOD | ENSG00000189058 | 3 | 195310802 | 195311018 | 217 | − |
| 69 | MUC4 | ENSG00000145113 | 3 | 195536032 | 195537321 | 1290 | − |
| 70 | MCIDAS | ENSG00000234602 | 5 | 54518579 | 54519189 | 611 | + |
| 71 | OCLN | ENSG00000197822 | 5 | 68787631 | 68787825 | 195 | − |
| 72 | PCDHGA2;<br>NA | ENSG00000081853;<br>ENSG00000241325 | 5 | 140797155 | 140797364 | 210 | + |
| 73 | C6orf195 | ENSG00000164385 | 6 | 2514359 | 2516276 | 1918 | − |
| 74 | | ENSG00000196333 | 6 | 19179779 | 19182021 | 2243 | − |
| 75 | HCG16 | ENSG00000244349 | 6 | 28956144 | 28956970 | 827 | + |
| 76 | HCG9 | ENSG00000204625 | 6 | 29943251 | 29943629 | 379 | + |
| 77 | RNF39 | ENSG00000204618 | 6 | 30039051 | 30039749 | 699 | + |
| 78 | SLC22A16 | ENSG00000004809 | 6 | 110797397 | 110797584 | 188 | + |
| 79 | PARK2 | ENSG00000185345 | 6 | 161796297 | 161797341 | 1045 | − |
| 80 | WBSCR17 | ENSG00000185274 | 7 | 70597038 | 70597093 | 56 | + |
| 81 | RN7SL76P | ENSG00000241959 | 7 | 151156201 | 151158179 | 1979 | − |
| 82 | SPIDR | ENSG00000164808 | 8 | 48571960 | 48573044 | 1085 | − |
| 83 | CA3 | ENSG00000164879 | 8 | 86350503 | 86350656 | 154 | + |
| 84 | PPP1R16A;<br>GPT | ENSG00000160972;<br>ENSG00000167701 | 8 | 145728374 | 145729865 | 1492 | − |
| 85 | NPY4R | ENSG00000204174 | 10 | 47083219 | 47083381 | 163 | + |
| 86 | C10orf107 | ENSG00000183346 | 10 | 63422447 | 63422576 | 130 | − |
| 87 | LINC00857 | ENSG00000237523 | 10 | 81967370 | 81967832 | 463 | − |
| 88 | VAX1 | ENSG00000148704 | 10 | 118891415 | 118891890 | 476 | + |
| 89 | TACC2 | ENSG00000138162 | 10 | 123922971 | 123923178 | 208 | + |
| 90 | MUC2 | ENSG00000198788 | 11 | 1058891 | 1062477 | 3587 | − |

[Table 4]

| DMR no. | Gene Symbol | Ensembl ID | Chromosome no. | DMR start | DMR end | Width | ± |
|---|---|---|---|---|---|---|---|
| 91 | MUC2 | ENSG00000198788 | 11 | 1074614 | 1075155 | 542 | − |
| 92 | TEAD1 | ENSG00000187079 | 11 | 12697507 | 12701324 | 3818 | − |
| 93 | RP11-121M22.1 | ENSG00000175773 | 11 | 130270828 | 130272842 | 2015 | + |
| 94 | KCNC2 | ENSG00000166006 | 12 | 75601683 | 75601943 | 261 | + |
| 95 | NCOR2 | ENSG00000196498 | 12 | 124906454 | 124908279 | 1826 | − |
| 96 | PDX1 | ENSG00000139515 | 13 | 28498306 | 28498463 | 158 | + |
| 97 | PDX1 | ENSG00000139515 | 13 | 28500855 | 28501186 | 332 | + |
| 98 | | ENSG00000198348 | 14 | 101922989 | 101923532 | 544 | + |
| 99 | MEIS2 | ENSG00000134138 | 15 | 37387445 | 37387655 | 211 | + |
| 100 | CCDC64B | ENSG00000162069 | 16 | 3079798 | 3080032 | 235 | + |
| 101 | ADCY9 | ENSG00000162104 | 16 | 3999535 | 4001924 | 2390 | − |
| 102 | | ENSG00000227093 | 16 | 54407005 | 54408952 | 1948 | + |
| 103 | GRB7 | ENSG00000141738 | 17 | 37895616 | 37896445 | 830 | − |
| 104 | RAPGEFL1 | ENSG00000108352 | 17 | 38347581 | 38347738 | 158 | + |
| 105 | WNK4 | ENSG00000126562 | 17 | 40936617 | 40936916 | 300 | + |
| 106 | HOXB6; HOXB-AS3 | ENSG00000239558; ENSG00000108511; ENSG00000233101 | 17 | 46674245 | 46674664 | 420 | + |
| 107 | CHAD; ACSF2 | ENSG00000136457; ENSG00000167107 | 17 | 48546115 | 48546272 | 158 | + |
| 108 | | ENSG00000230792 | 17 | 55212625 | 55214595 | 1971 | + |
| 109 | | ENSG00000171282 | 17 | 79393453 | 79393610 | 158 | − |
| 110 | TPM4 | ENSG00000167460 | 19 | 16178026 | 16178163 | 138 | − |
| 111 | | ENSG00000248094 | 19 | 21646440 | 21646771 | 332 | + |
| 112 | RP6-109B7.4; MIRLET7BHG | ENSG00000235159; ENSG00000197182; ENSG00000245020 | 22 | 46461776 | 46465514 | 3739 | − |

**2.** The method for determining the likelihood of colorectal cancer development according to claim 1, wherein the multivariate discrimination expression includes, as variables, average methylation rate of one or more differentially methylated regions selected from the group consisting of the differentially methylated regions represented by the differentially methylated region numbers 1 to 58.

**3.** The method for determining the likelihood of colorectal cancer development according to claim 1, wherein the multivariate discrimination expression includes, as variables, average methylation rate of two or more differentially methylated regions selected from the group consisting of the differentially methylated regions represented by the differentially methylated region numbers 1 to 58.

**4.** The method for determining the likelihood of colorectal cancer development according to any one of claims 1 to 3, wherein in the determination step, in a case where one or more among the differentially methylated regions represented by differentially methylated region numbers 1, 3 to 20, 23 to 28, 31 to 46, 49 to 60, 62, 65 to 69, 71, 73, 74, 79, 81, 82, 84, 86, 87, 90 to 92, 95, 101, 103, 109, 110, and 112 have an average methylation rate of equal to or lower than the preset reference value, or one or more among the differentially methylated regions represented by differentially methylated region numbers 2, 21, 22, 29, 30, 47, 48, 61, 63, 64, 70, 72, 75 to 78, 80, 83, 85, 88, 89, 93, 94, 96 to 100, 102, 104 to 108, and 111 have an average methylation rate of equal to or higher than the preset reference value, it is determined that there is a high likelihood of colorectal cancer development in the human ulcerative colitis patient.

**5.** The method for determining the likelihood of colorectal cancer development according to claim 1, wherein the selected differentially methylated regions are the differentially methylated regions represented by the differentially methylated region numbers 21, 77 and 107; 14, 21 and 77; 14, 21 and 107; or 14, 77 and 107; and
wherein in the determination step, in a case where the differentially methylated region represented by differentially methylated region number 14 has an average methylation rate of equal to or lower than the preset reference value, and the differentially methylated regions represented by differentially methylated region numbers 21, 77 and 107 have an average methylation rate of equal to or higher than the preset reference value, it is determined that there is a high likelihood of colorectal cancer development in the human ulcerative colitis patient.

**6.** The method for determining the likelihood of colorectal cancer development according to claim 1,
wherein in the determination step, in a case where based on the average methylation rate of the differentially methylated region calculated based on the methylation rates measured in the measurement step, and the multivariate discrimination expression, a discrimination value which is a value of the multivariate discrimination expression is calculated, and the discrimination value is equal to or higher than a preset reference discrimination value, it is determined that there is a high likelihood of colorectal cancer development in the human ulcerative colitis patient.

**7.** The method for determining the likelihood of colorectal cancer development according to any one of claims 1 to 6, wherein the multivariate discrimination expression is a logistic regression expression, a linear discrimination expression, an expression created by Naive Bayes classifier, or an expression created by Support Vector Machine.

**8.** The method for determining the likelihood of colorectal cancer development according to any one of claims 1 to 7, wherein the biological sample is intestinal tract tissue.

**9.** The method for determining the likelihood of colorectal cancer development according to any one of claims 1 to 8, wherein the biological sample is rectal mucosal tissue.

FIG. 1

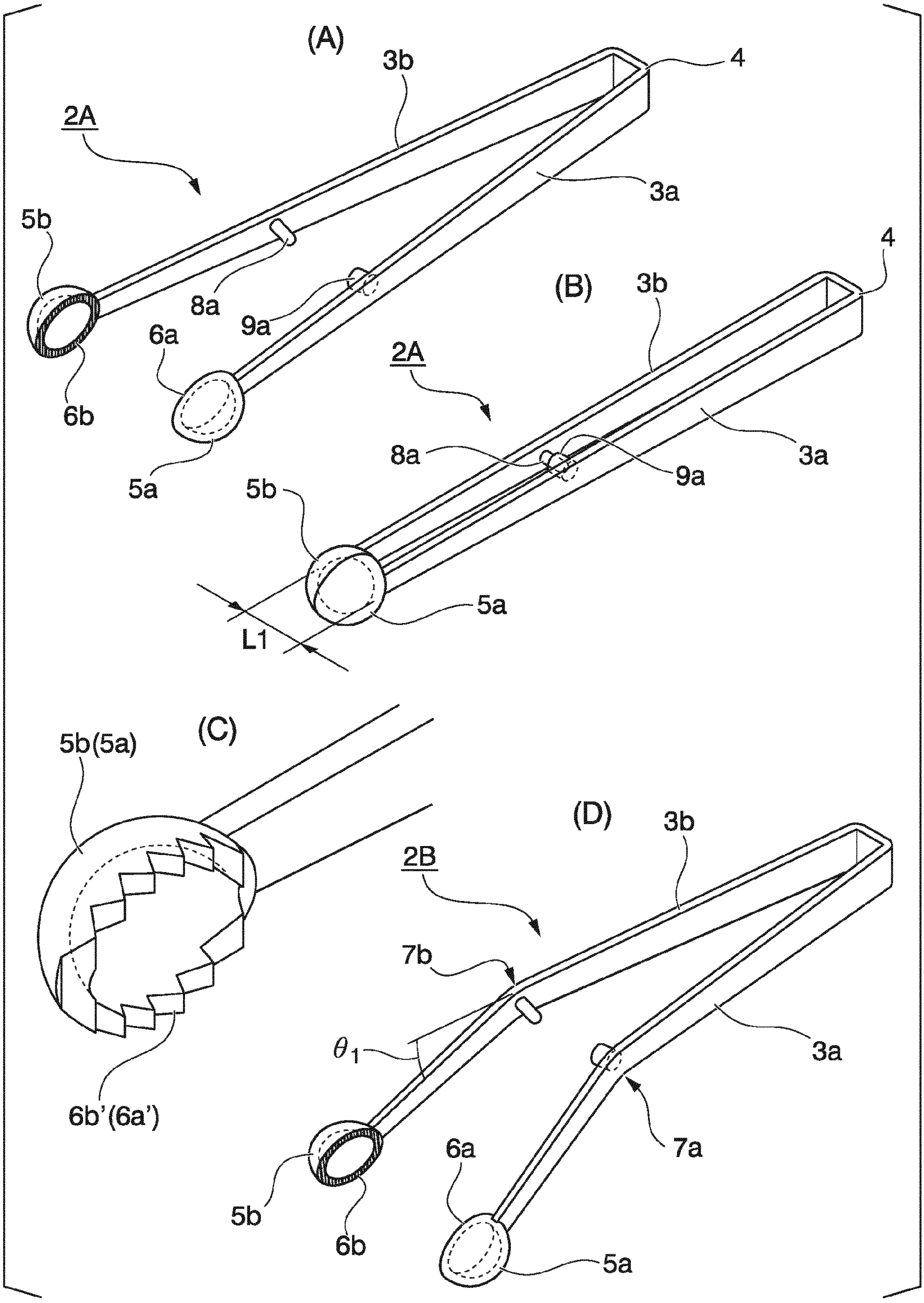
(A)
3b
4
2A
3a
5b
8a
9a
6a
6b
5a
(B)
3b
4
2A
8a
9a
3a
5b
5a
L1
(C)
5b(5a)
6b'(6a')
(D)
3b
2B
7b
$\theta_1$
3a
7a
6a
5b
6b
5a

*FIG. 2*

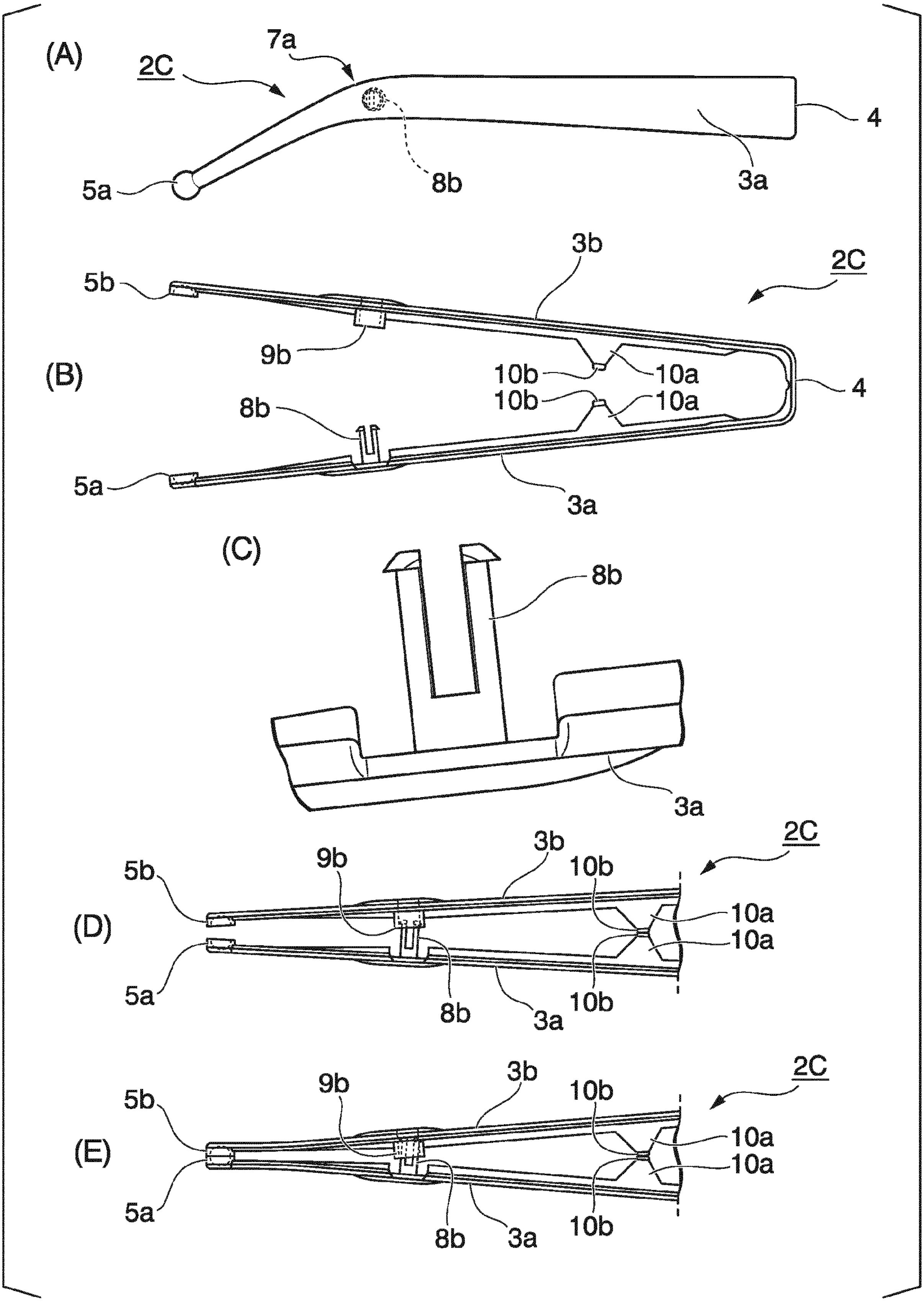
(A)
2C
7a
4
5a
8b
3a
(B)
5b
3b
2C
9b
10b
10a
4
8b
5a
3a
(C)
8b
3a
(D)
5b
9b
3b
10b
2C
10a
10a
5a
8b
3a
10b
(E)
5b
9b
3b
10b
2C
10a
10a
5a
8b
3a
10b

*FIG. 3*

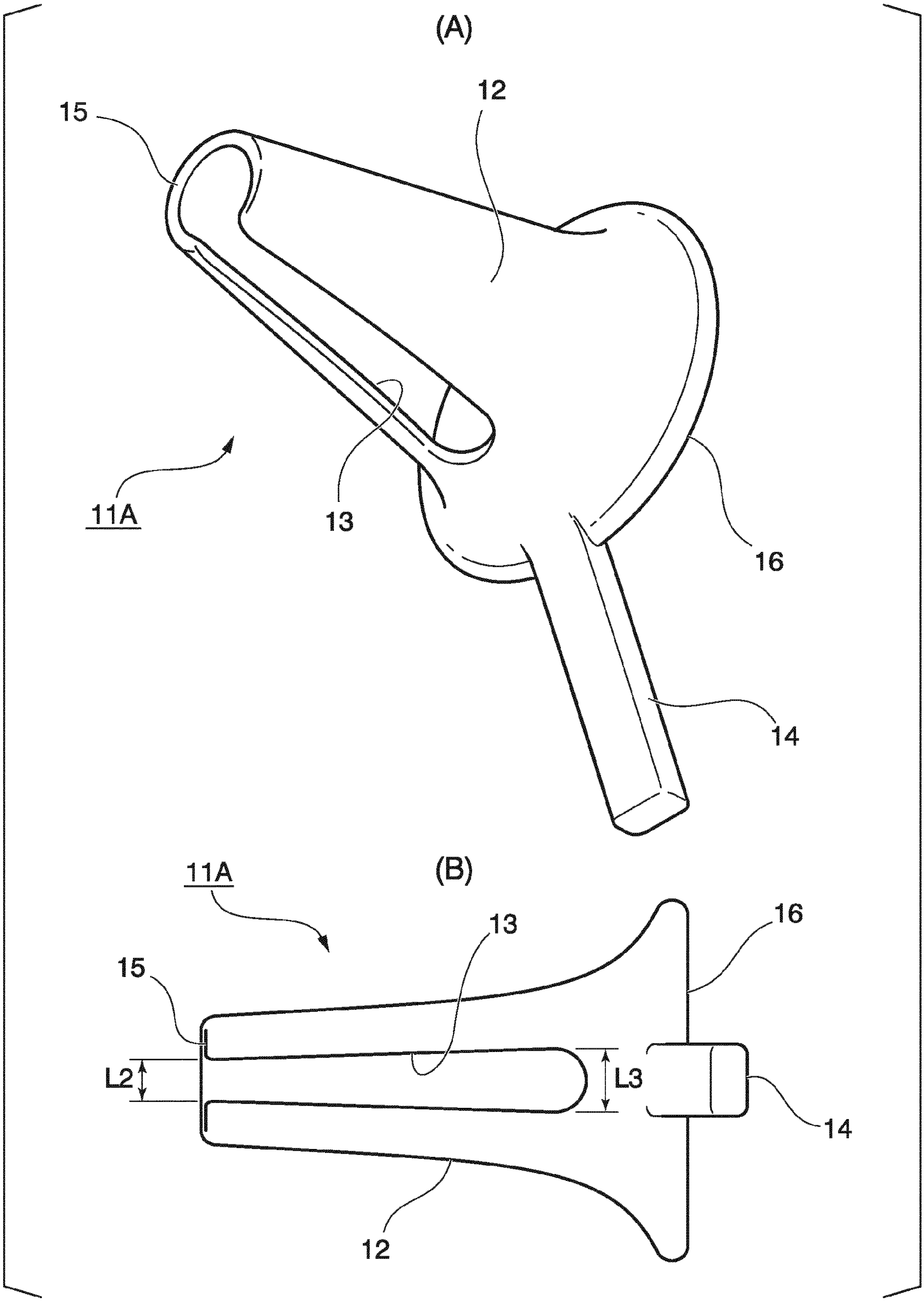

*FIG. 4*

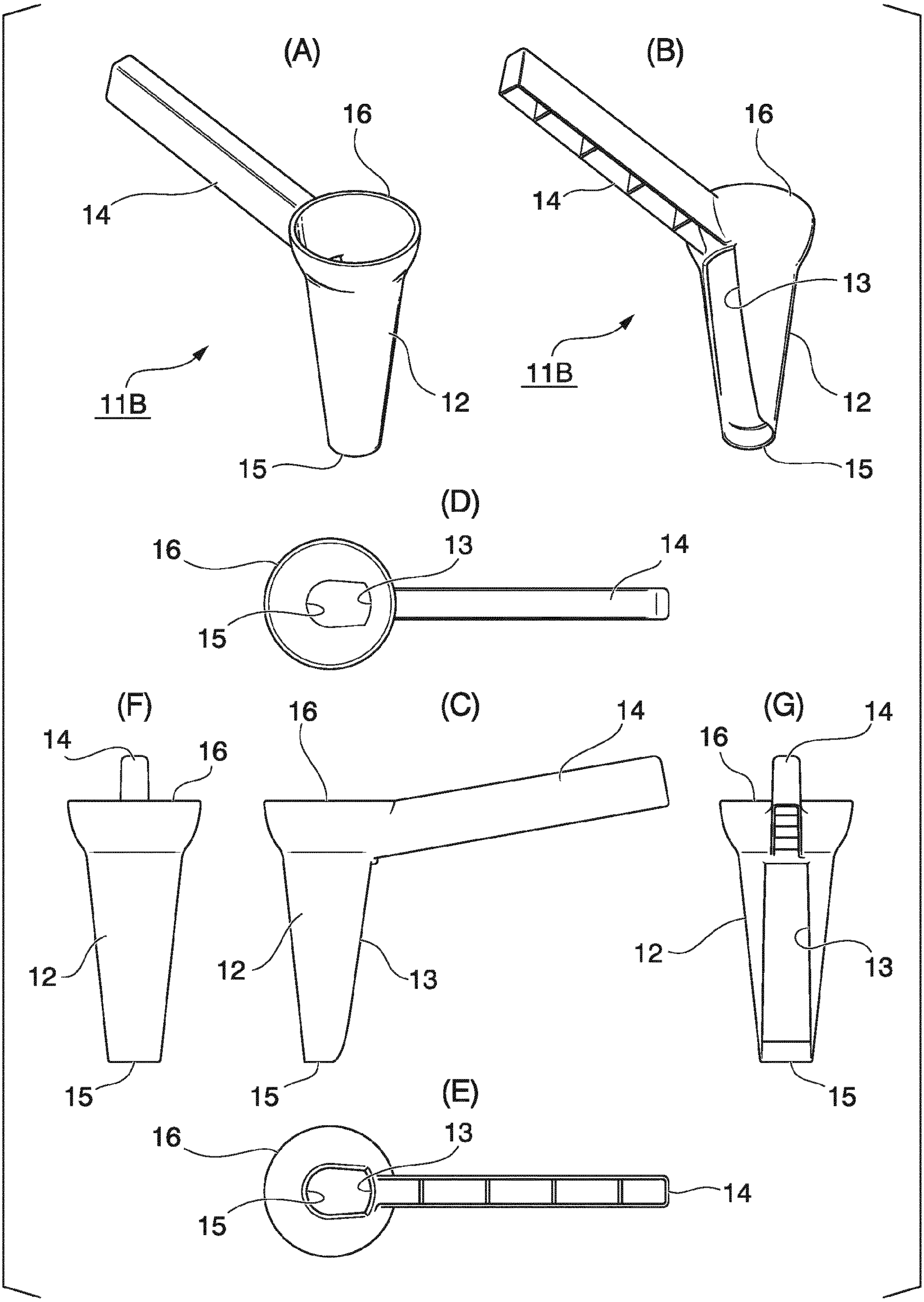
(A)
16
14
11B
12
15
(B)
16
14
13
11B
12
15
(D)
16
13
14
15
(F)
14
16
12
15
(C)
16
14
12
13
15
(G)
16
14
12
13
15
(E)
16
13
15
14

*FIG. 5*

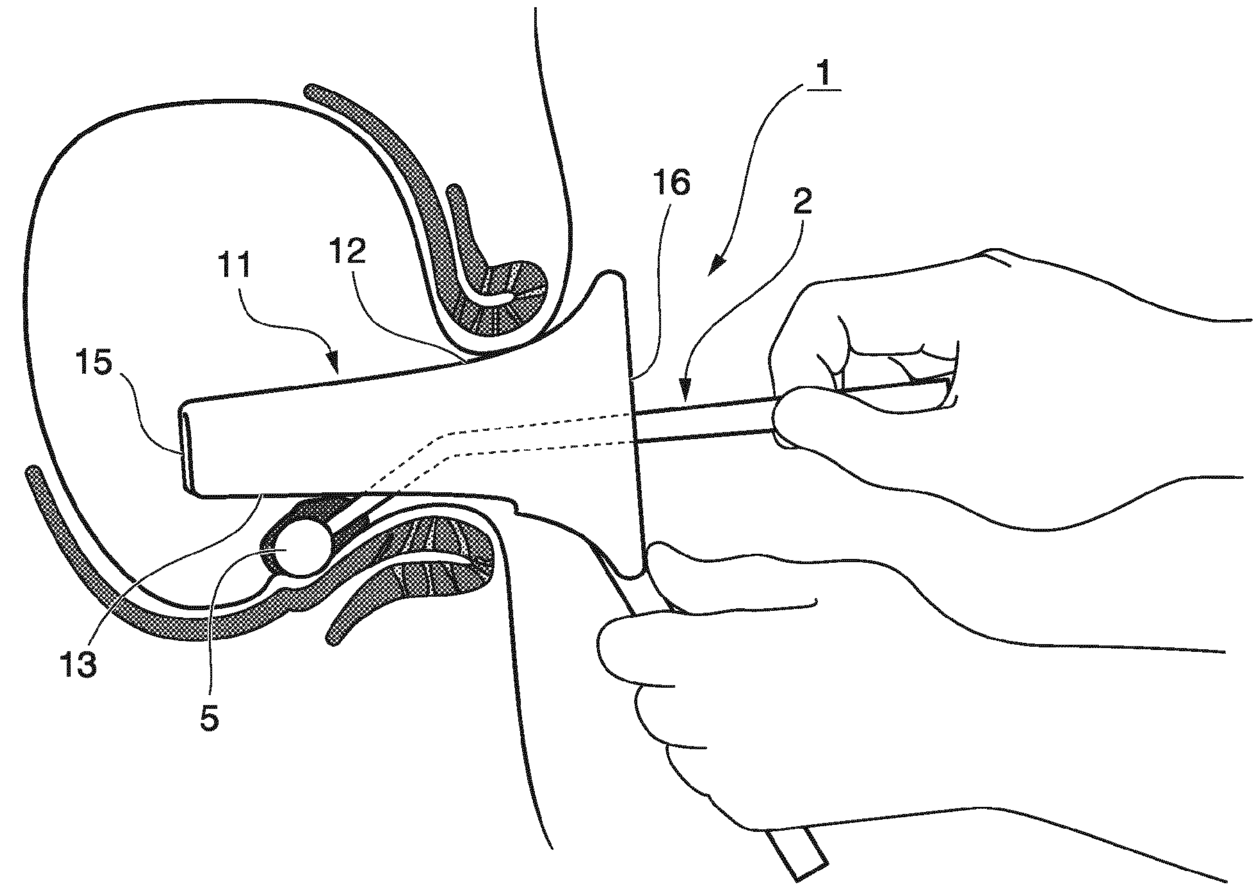

FIG. 6A

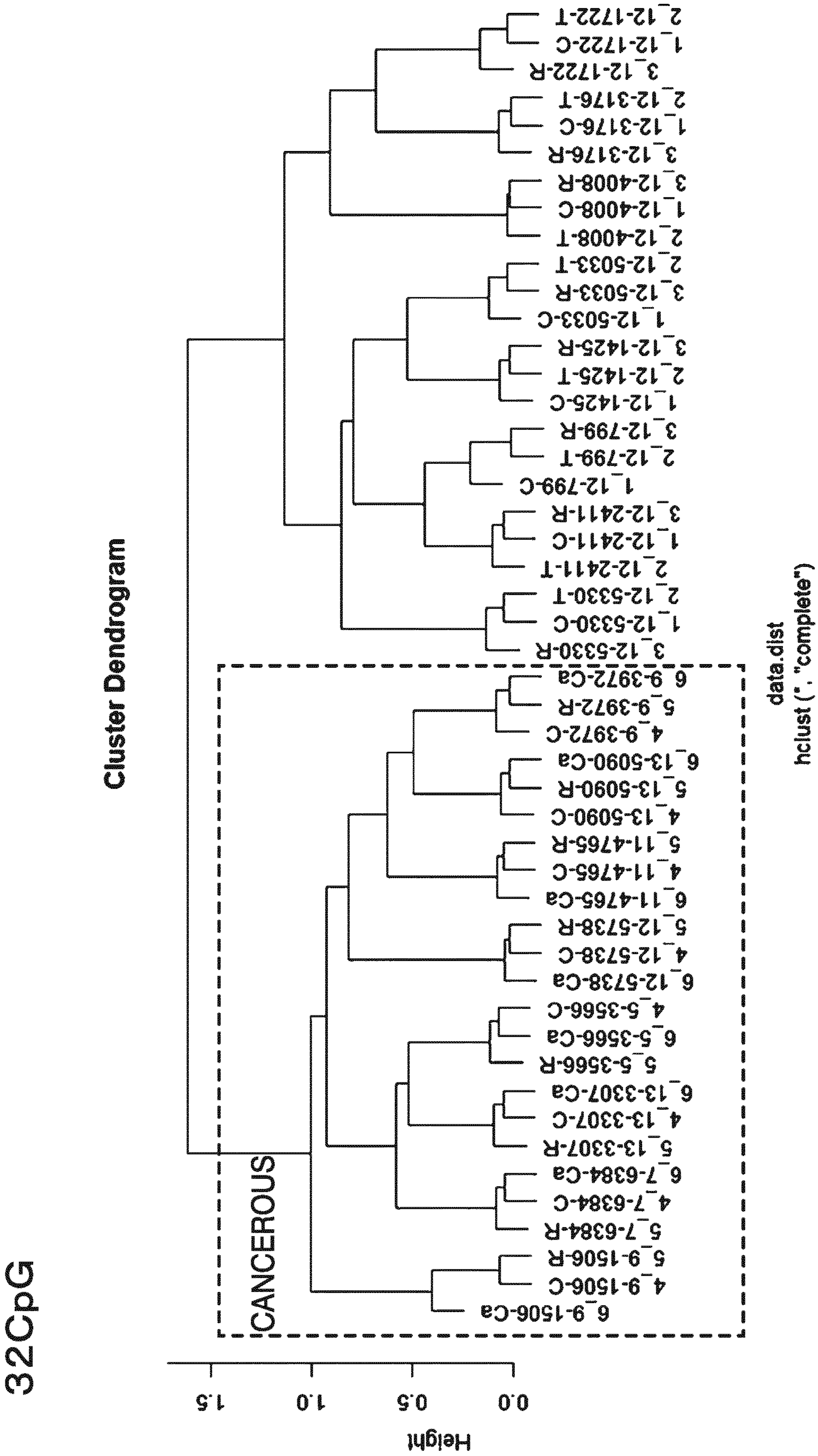
32CpG
Cluster Dendrogram
CANCEROUS
Height
0.0
0.5
1.0
1.5
data.dist
hclust (*, "complete")
2_12-1722-T
1_12-1722-C
3_12-1722-R
2_12-3176-T
1_12-3176-C
3_12-3176-R
3_12-4008-R
1_12-4008-C
2_12-4008-T
2_12-5033-T
3_12-5033-R
1_12-5033-C
3_12-1425-R
2_12-1425-T
1_12-1425-C
3_12-799-R
2_12-799-T
1_12-799-C
3_12-2411-R
1_12-2411-C
2_12-2411-T
2_12-5330-T
1_12-5330-C
3_12-5330-R
6_9-3972-Ca
5_9-3972-R
4_9-3972-C
6_13-5090-Ca
5_13-5090-R
4_13-5090-C
5_11-4765-R
4_11-4765-C
6_11-4765-Ca
5_12-5738-R
4_12-5738-C
6_12-5738-Ca
4_5-3566-C
6_5-3566-Ca
5_5-3566-R
6_13-3307-Ca
4_13-3307-C
5_13-3307-R
6_7-6384-Ca
4_7-6384-C
5_7-6384-R
5_9-1506-R
4_9-1506-C
6_9-1506-Ca

FIG. 6B

32CpG

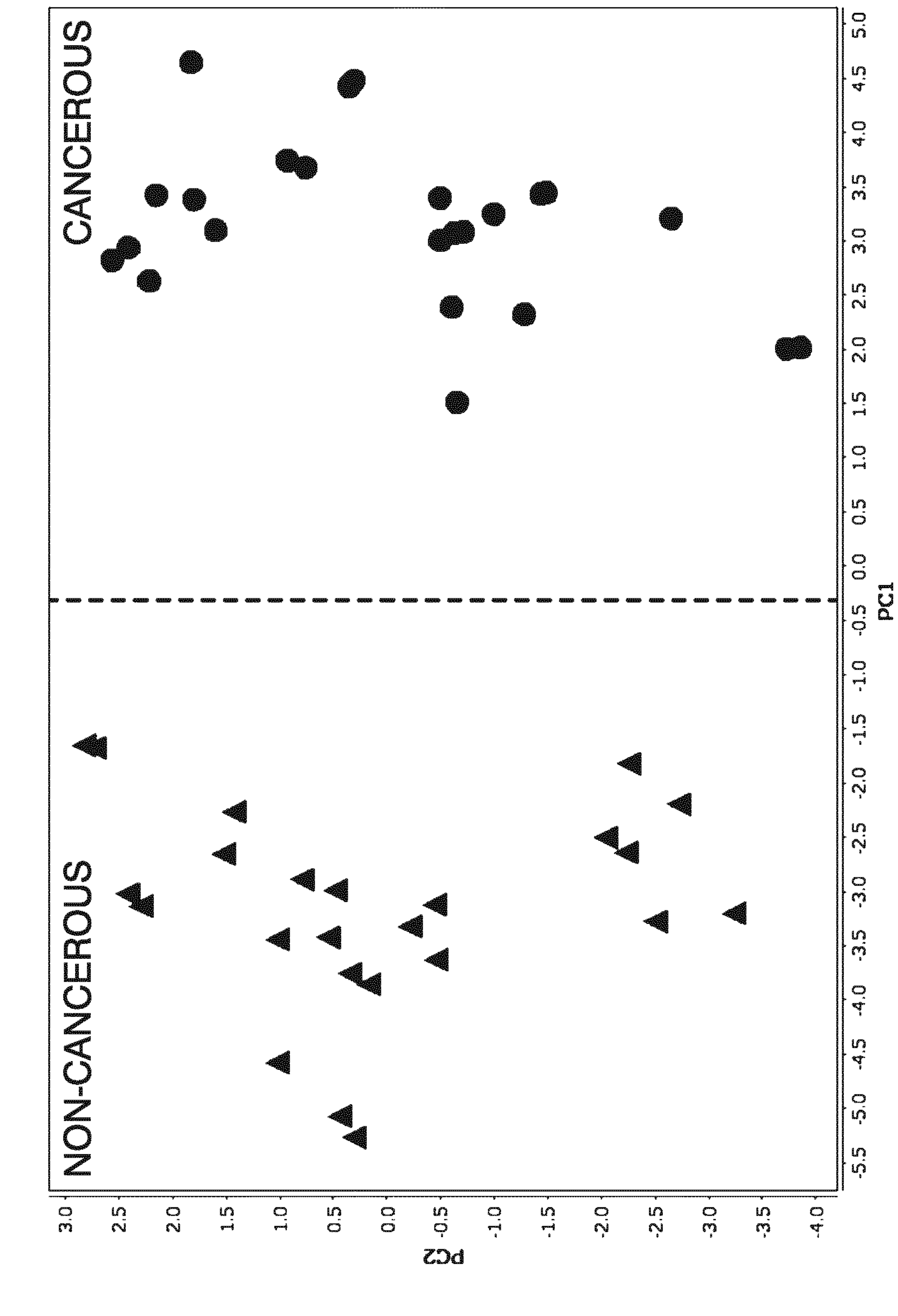
NON-CANCEROUS
CANCEROUS
PC1
PC2
-5.5 -5.0 -4.5 -4.0 -3.5 -3.0 -2.5 -2.0 -1.5 -1.0 -0.5 0.0 0.5 1.0 1.5 2.0 2.5 3.0 3.5 4.0 4.5 5.0
3.0 2.5 2.0 1.5 1.0 0.5 0.0 -0.5 -1.0 -1.5 -2.0 -2.5 -3.0 -3.5 -4.0

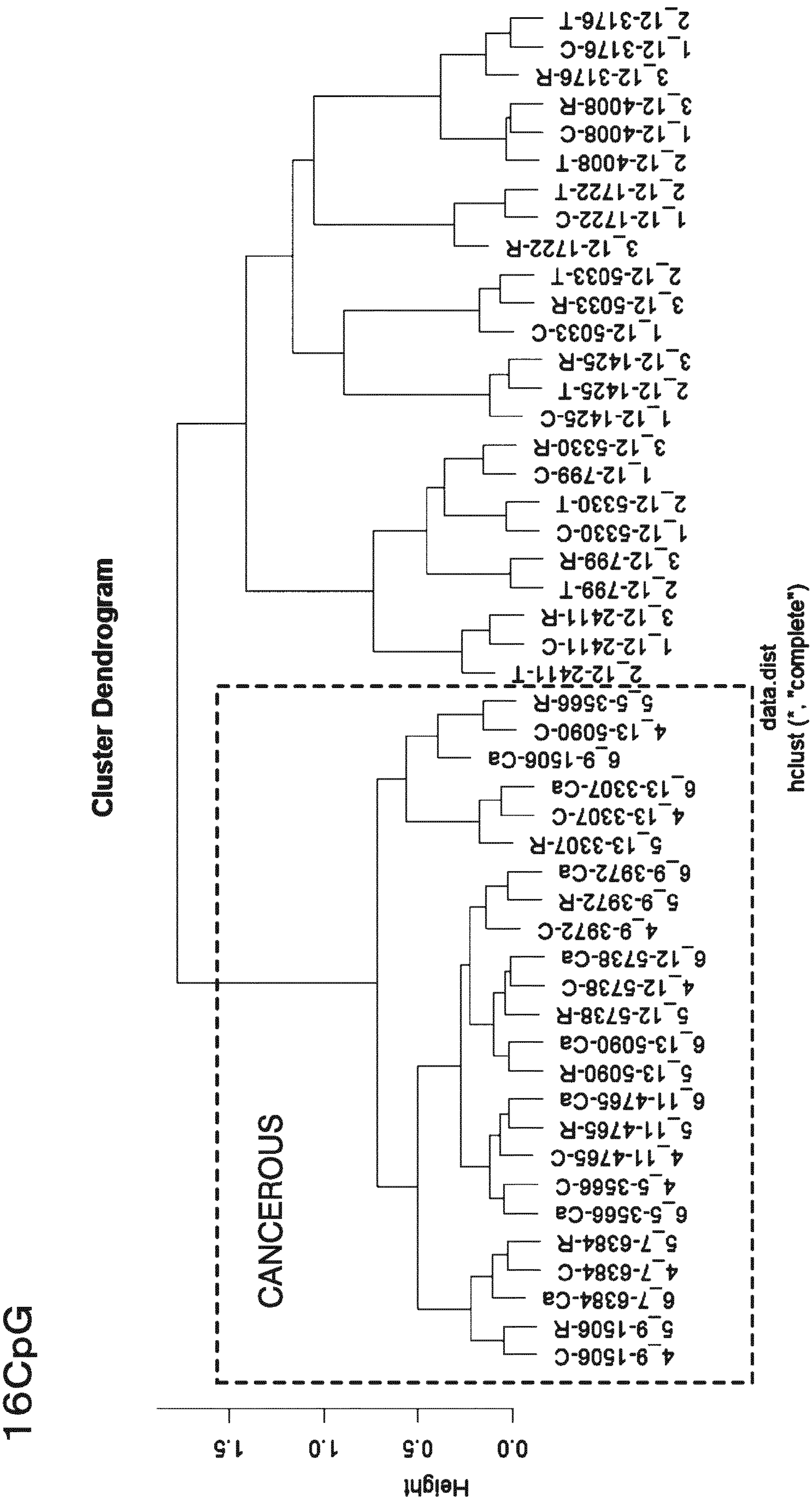
16CpG
Cluster Dendrogram
CANCEROUS
2_12-3176-T
1_12-3176-C
3_12-3176-R
3_12-4008-R
1_12-4008-C
2_12-4008-T
2_12-1722-T
1_12-1722-C
3_12-1722-R
2_12-5033-T
3_12-5033-R
1_12-5033-C
3_12-1425-R
2_12-1425-T
1_12-1425-C
3_12-5330-R
1_12-799-C
2_12-5330-T
1_12-5330-C
3_12-799-R
2_12-799-T
3_12-2411-R
1_12-2411-C
2_12-2411-T
5_5-3566-R
4_13-5090-C
6_9-1506-Ca
6_13-3307-Ca
4_13-3307-C
5_13-3307-R
6_9-3972-Ca
5_9-3972-R
4_9-3972-C
6_12-5738-Ca
4_12-5738-C
5_12-5738-R
6_13-5090-Ca
5_13-5090-R
6_11-4765-Ca
5_11-4765-R
4_11-4765-C
4_5-3566-C
6_5-3566-Ca
5_7-6384-R
4_7-6384-C
6_7-6384-Ca
5_9-1506-R
4_9-1506-C
Height
0.0
0.5
1.0
1.5
data.dist
hclust (*, "complete")

FIG. 6C

FIG. 6D

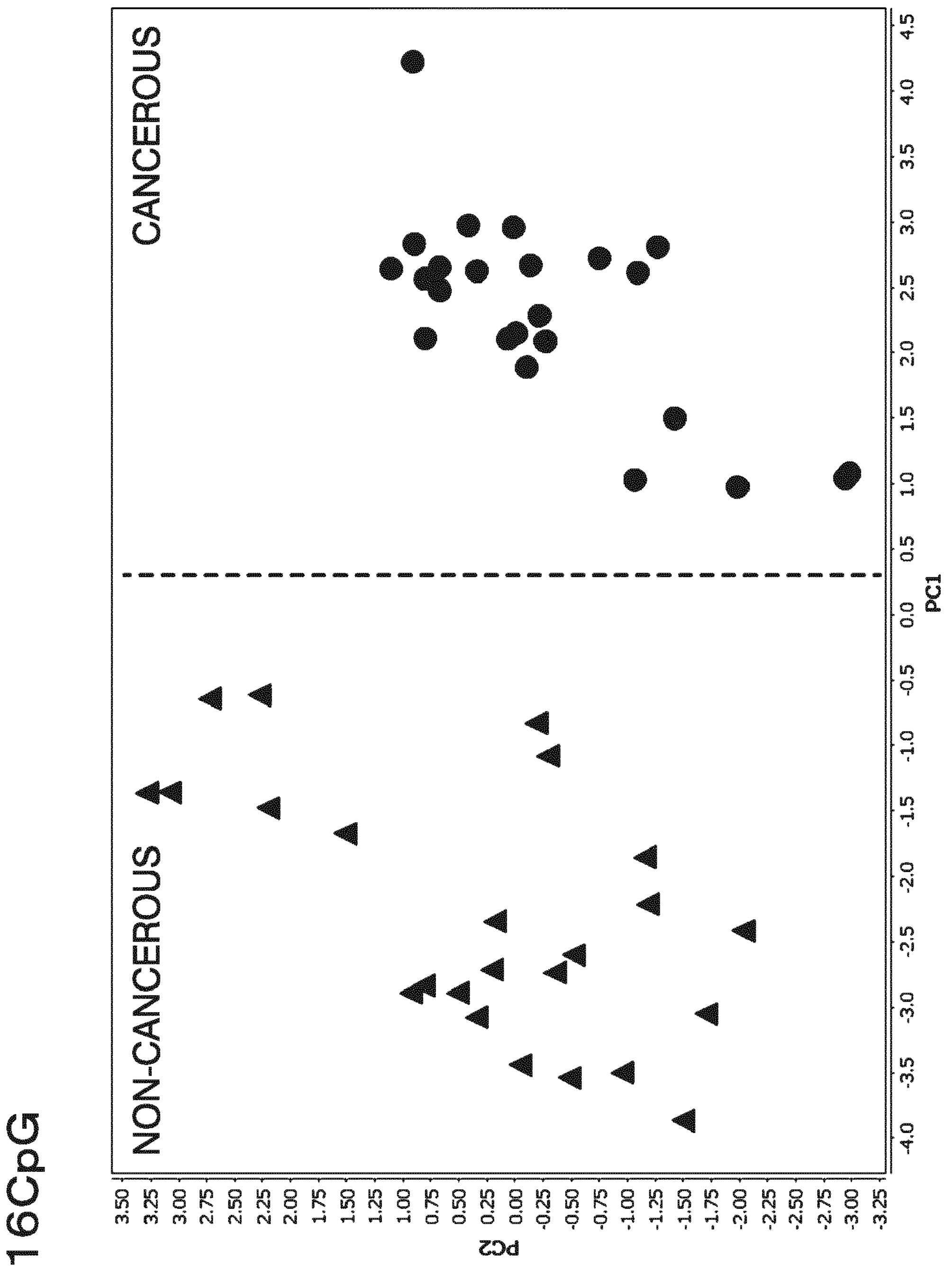

FIG. 6E

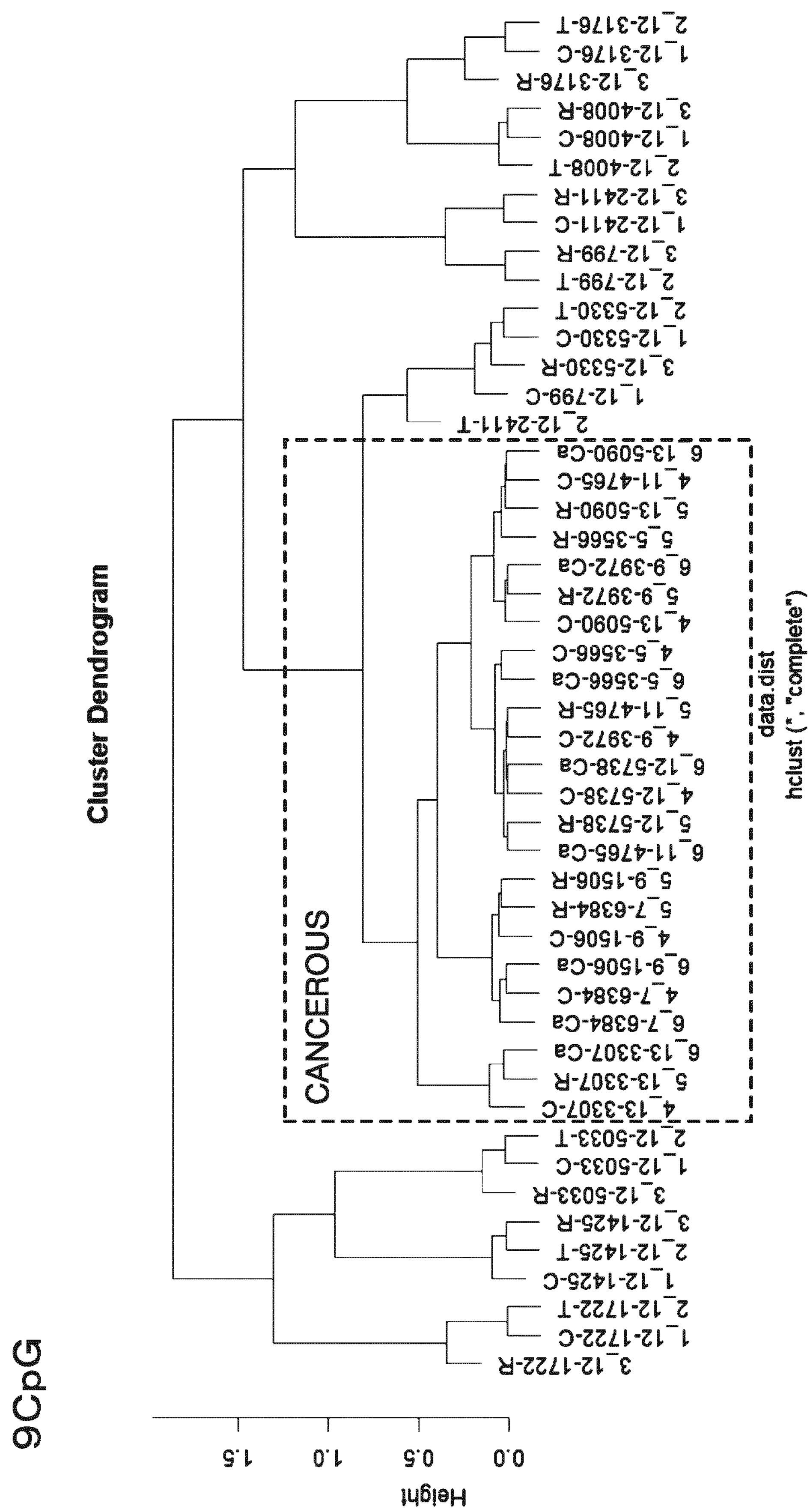
Cluster Dendrogram
9CpG
Height
0.0
0.5
1.0
1.5
CANCEROUS
data.dist
hclust (*, "complete")
2_12-3176-T
1_12-3176-C
3_12-3176-R
3_12-4008-R
1_12-4008-C
2_12-4008-T
3_12-2411-R
1_12-2411-C
3_12-799-R
2_12-799-T
2_12-5330-T
1_12-5330-C
3_12-5330-R
1_12-799-C
2_12-2411-T
6_13-5090-Ca
4_11-4765-C
5_13-5090-R
5_5-3566-R
6_9-3972-Ca
5_9-3972-R
4_13-5090-C
4_5-3566-C
6_5-3566-Ca
5_11-4765-R
4_9-3972-C
6_12-5738-Ca
4_12-5738-C
5_12-5738-R
6_11-4765-Ca
5_9-1506-R
5_7-6384-R
4_9-1506-C
6_9-1506-Ca
4_7-6384-C
6_7-6384-Ca
6_13-3307-Ca
5_13-3307-R
4_13-3307-C
2_12-5033-T
1_12-5033-C
3_12-5033-R
3_12-1425-R
2_12-1425-T
1_12-1425-C
2_12-1722-T
1_12-1722-C
3_12-1722-R

*FIG. 6F*

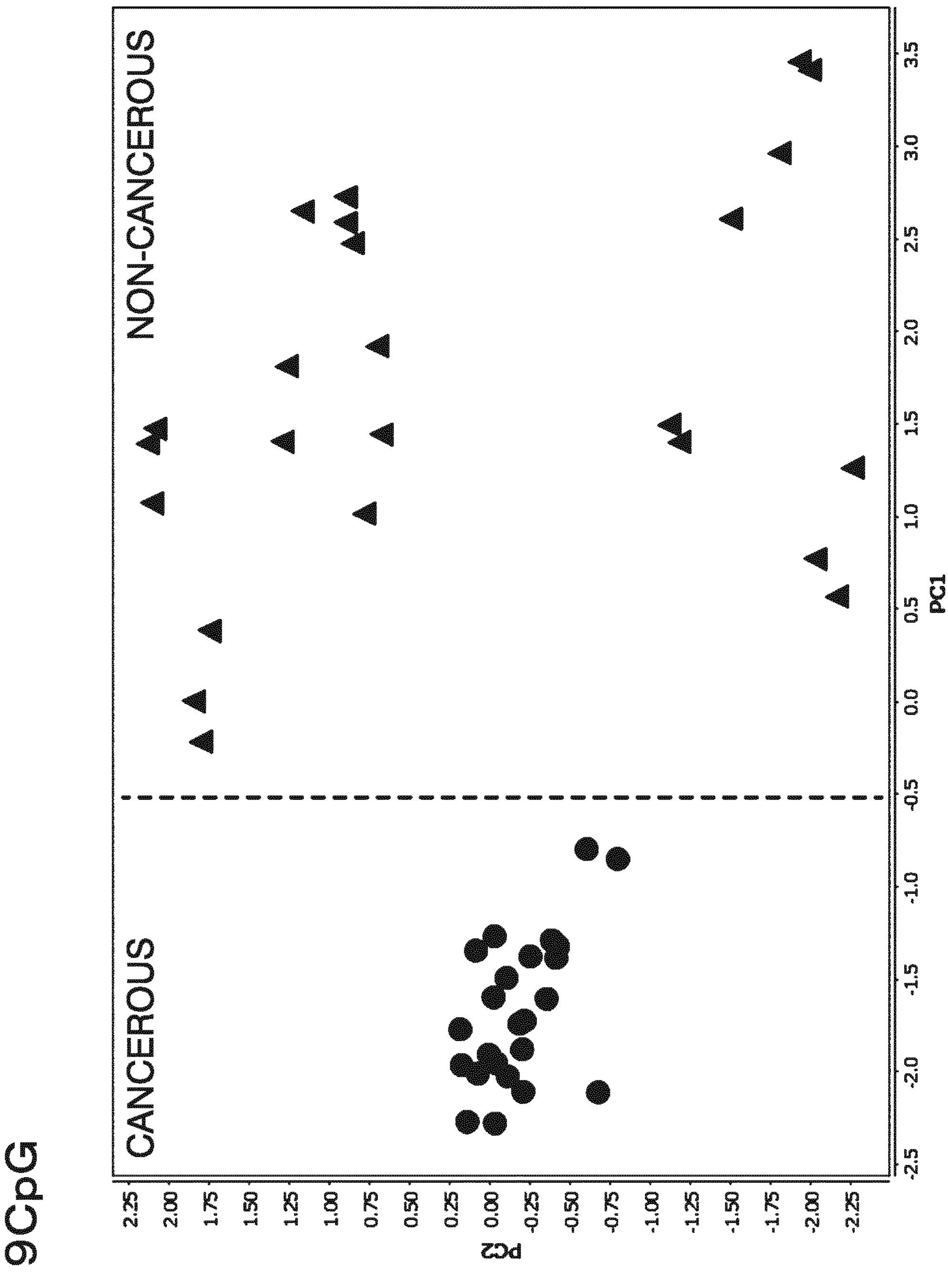
9CpG
CANCEROUS
NON-CANCEROUS
PC1
PC2
-2.5
-2.0
-1.5
-1.0
-0.5
0.0
0.5
1.0
1.5
2.0
2.5
3.0
3.5
2.25
2.00
1.75
1.50
1.25
1.00
0.75
0.50
0.25
0.00
-0.25
-0.50
-0.75
-1.00
-1.25
-1.50
-1.75
-2.00
-2.25

FIG. 7A

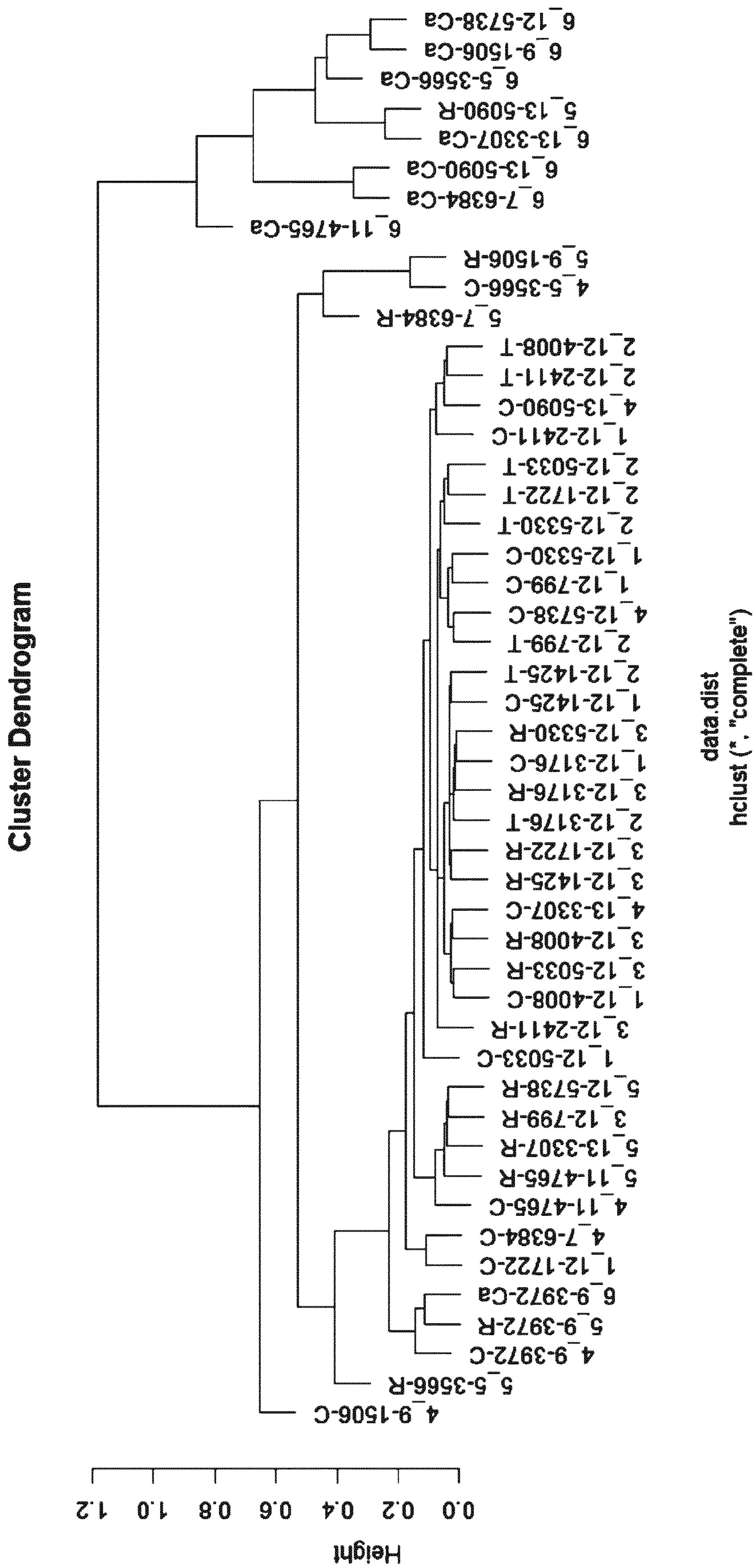
Cluster Dendrogram
6_12-5738-Ca
6_9-1506-Ca
6_5-3566-Ca
5_13-5090-R
6_13-3307-Ca
6_13-5090-Ca
6_7-6384-Ca
6_11-4765-Ca
5_9-1506-R
4_5-3566-C
5_7-6384-R
2_12-4008-T
2_12-2411-T
4_13-5090-C
1_12-2411-C
2_12-5033-T
2_12-1722-T
2_12-5330-T
1_12-5330-C
1_12-799-C
4_12-5738-C
2_12-799-T
2_12-1425-T
1_12-1425-C
3_12-5330-R
1_12-3176-C
3_12-3176-R
2_12-3176-T
3_12-1722-R
3_12-1425-R
4_13-3307-C
3_12-4008-R
3_12-5033-R
1_12-4008-C
3_12-2411-R
1_12-5033-C
5_12-5738-R
3_12-799-R
5_13-3307-R
5_11-4765-R
4_11-4765-C
4_7-6384-C
1_12-1722-C
6_9-3972-Ca
5_9-3972-R
4_9-3972-C
5_5-3566-R
4_9-1506-C
0.0
0.2
0.4
0.6
0.8
1.0
1.2
Height
data.dist
hclust (*, "complete")

*FIG. 7B*

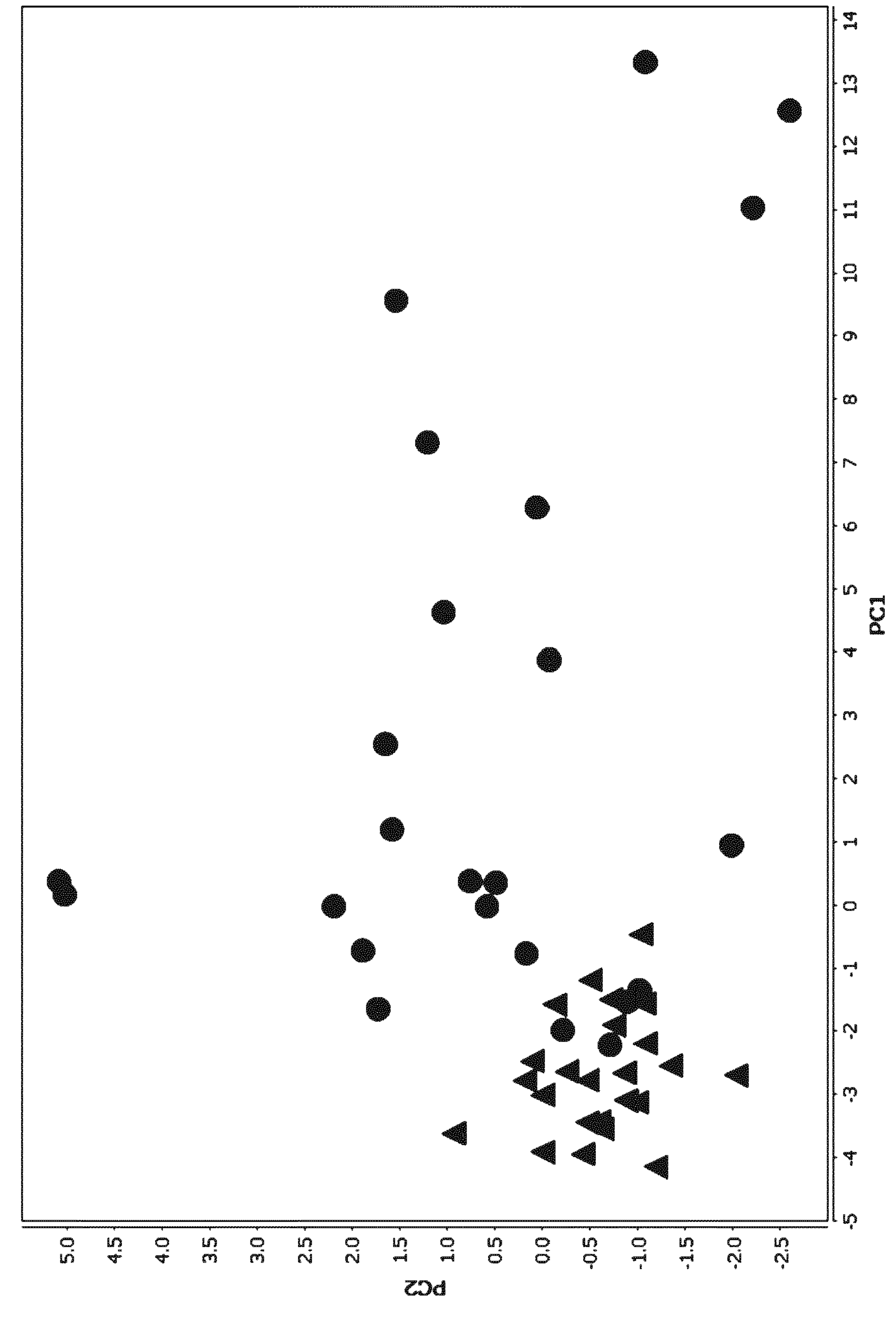
PC1
-5
-4
-3
-2
-1
0
1
2
3
4
5
6
7
8
9
10
11
12
13
14
PC2
5.0
4.5
4.0
3.5
3.0
2.5
2.0
1.5
1.0
0.5
0.0
-0.5
-1.0
-1.5
-2.0
-2.5

FIG. 8A

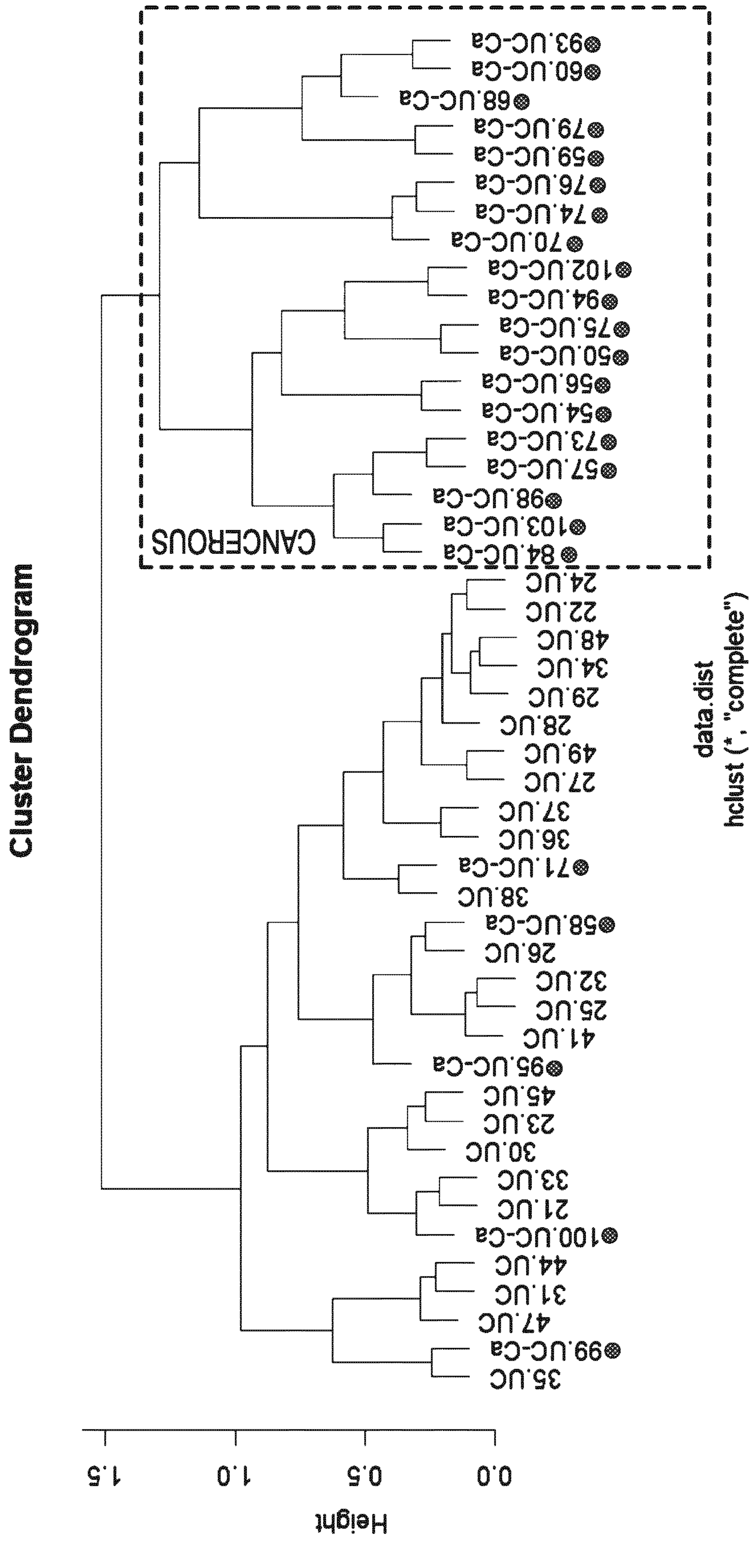
Cluster Dendrogram
CANCEROUS
93.UC-Ca
60.UC-Ca
68.UC-Ca
79.UC-Ca
59.UC-Ca
76.UC-Ca
74.UC-Ca
70.UC-Ca
102.UC-Ca
94.UC-Ca
75.UC-Ca
50.UC-Ca
56.UC-Ca
54.UC-Ca
73.UC-Ca
57.UC-Ca
98.UC-Ca
103.UC-Ca
84.UC-Ca
24.UC
22.UC
48.UC
34.UC
29.UC
28.UC
49.UC
27.UC
37.UC
36.UC
71.UC-Ca
38.UC
58.UC-Ca
26.UC
32.UC
25.UC
41.UC
95.UC-Ca
45.UC
23.UC
30.UC
33.UC
21.UC
100.UC-Ca
44.UC
31.UC
47.UC
99.UC-Ca
35.UC
0.0
0.5
1.0
1.5
Height
data.dist
hclust (*, "complete")

FIG. 8B

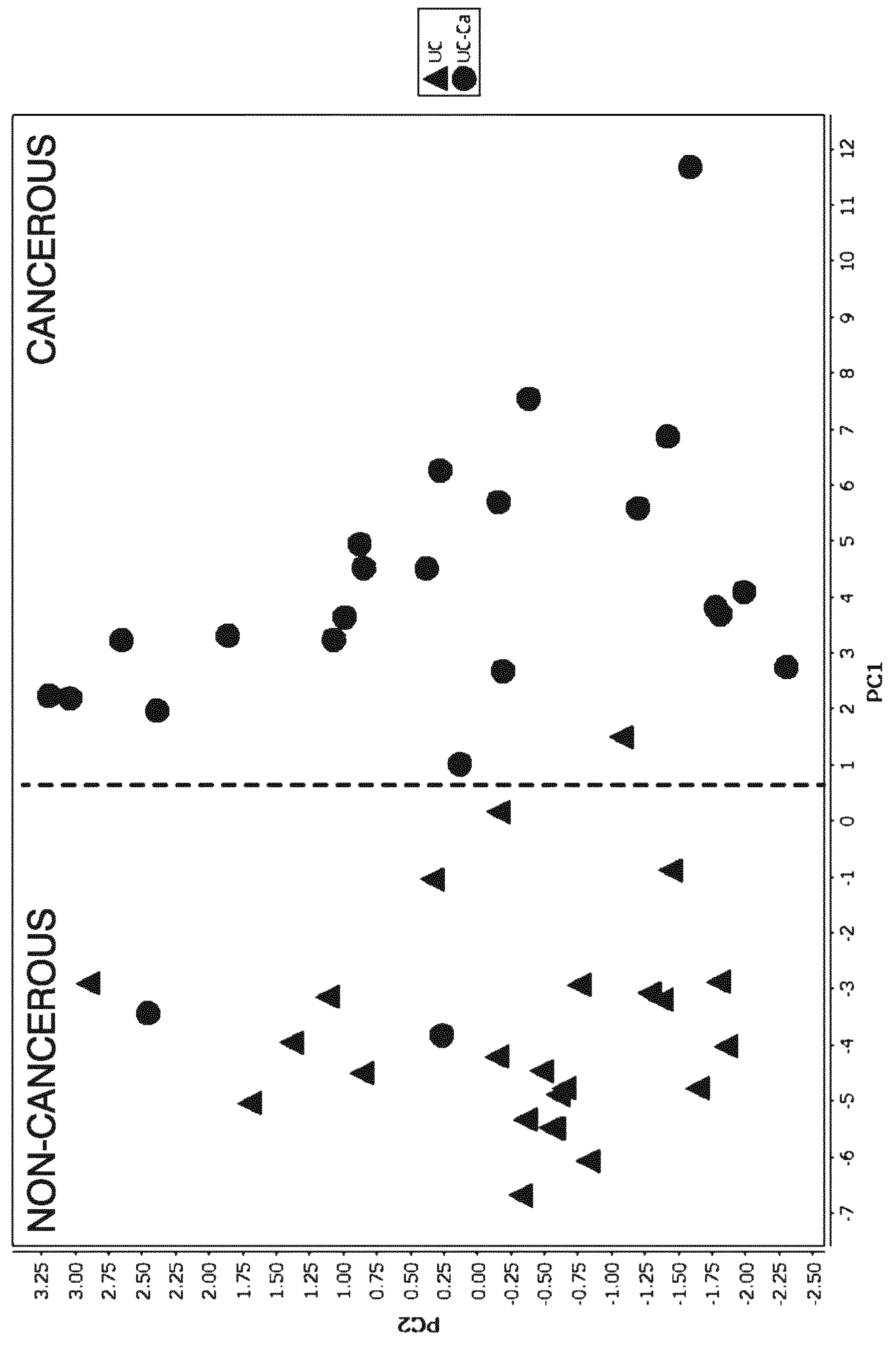
NON-CANCEROUS
CANCEROUS
UC
UC-Ca
PC1
PC2
-7 -6 -5 -4 -3 -2 -1 0 1 2 3 4 5 6 7 8 9 10 11 12
3.25 3.00 2.75 2.50 2.25 2.00 1.75 1.50 1.25 1.00 0.75 0.50 0.25 0.00 -0.25 -0.50 -0.75 -1.00 -1.25 -1.50 -1.75 -2.00 -2.25 -2.50

*FIG. 9*

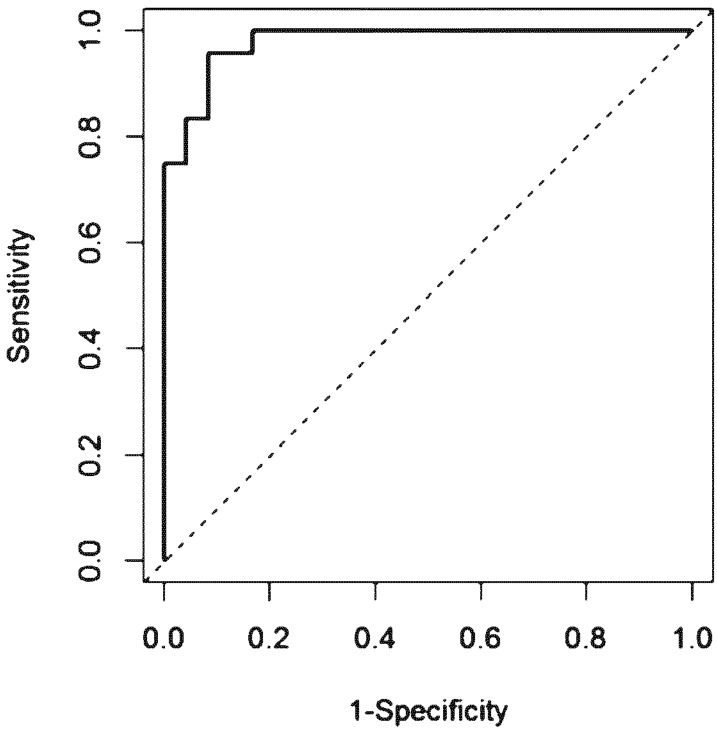

FIG. 10A

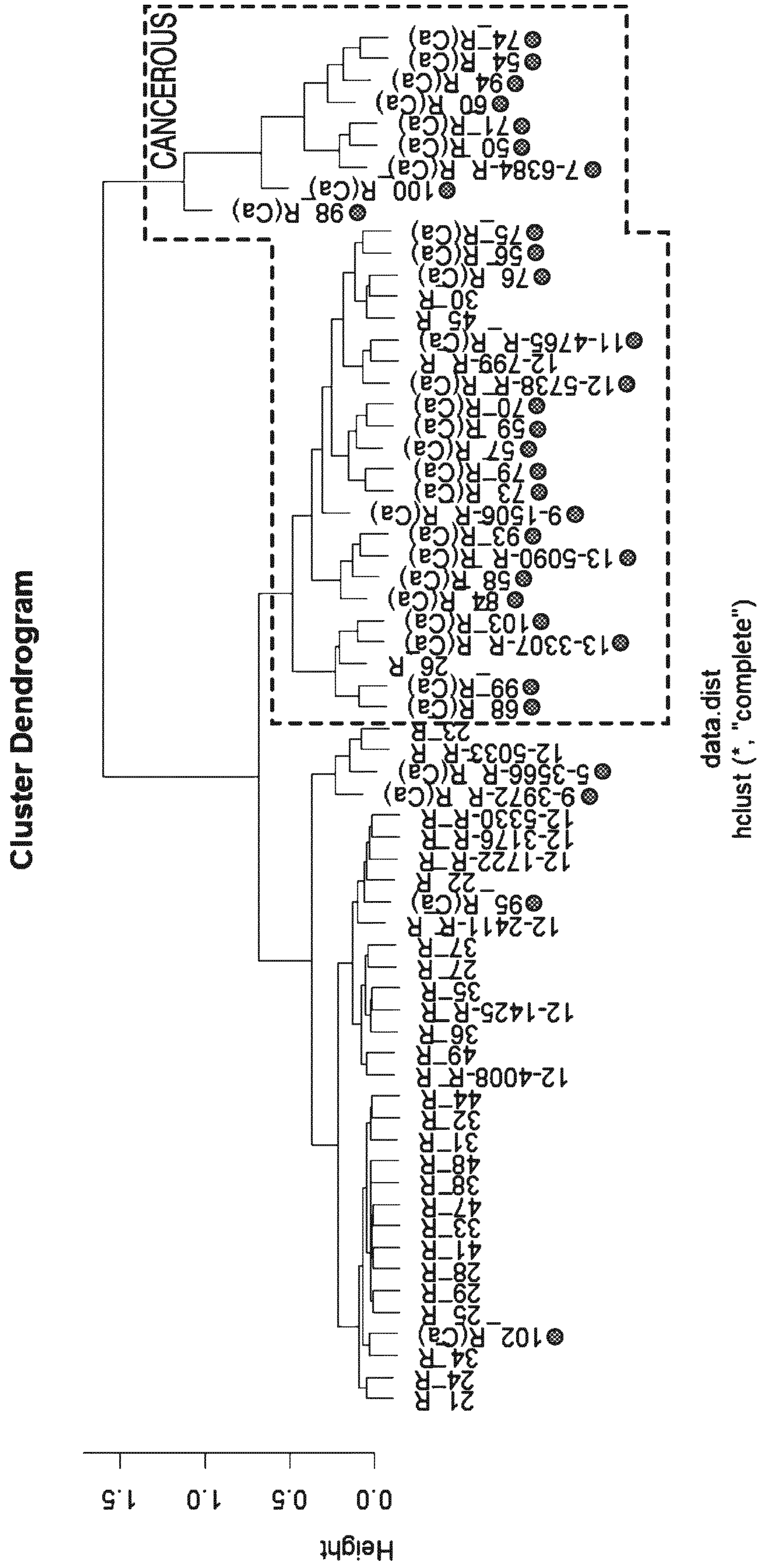
Cluster Dendrogram
CANCEROUS
Height
0.0
0.5
1.0
1.5
data.dist
hclust (*, "complete")

*FIG. 10B*

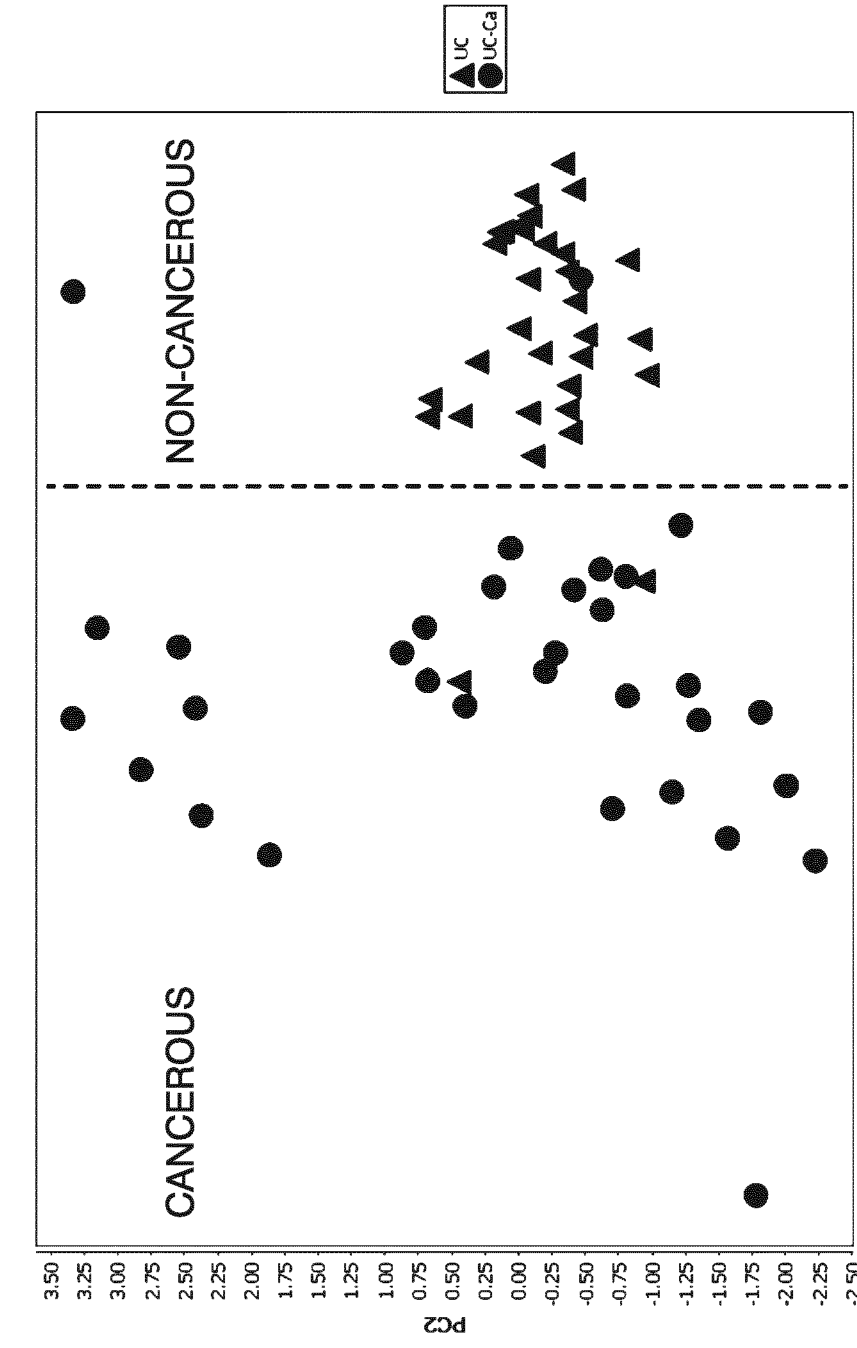

*FIG. 11*

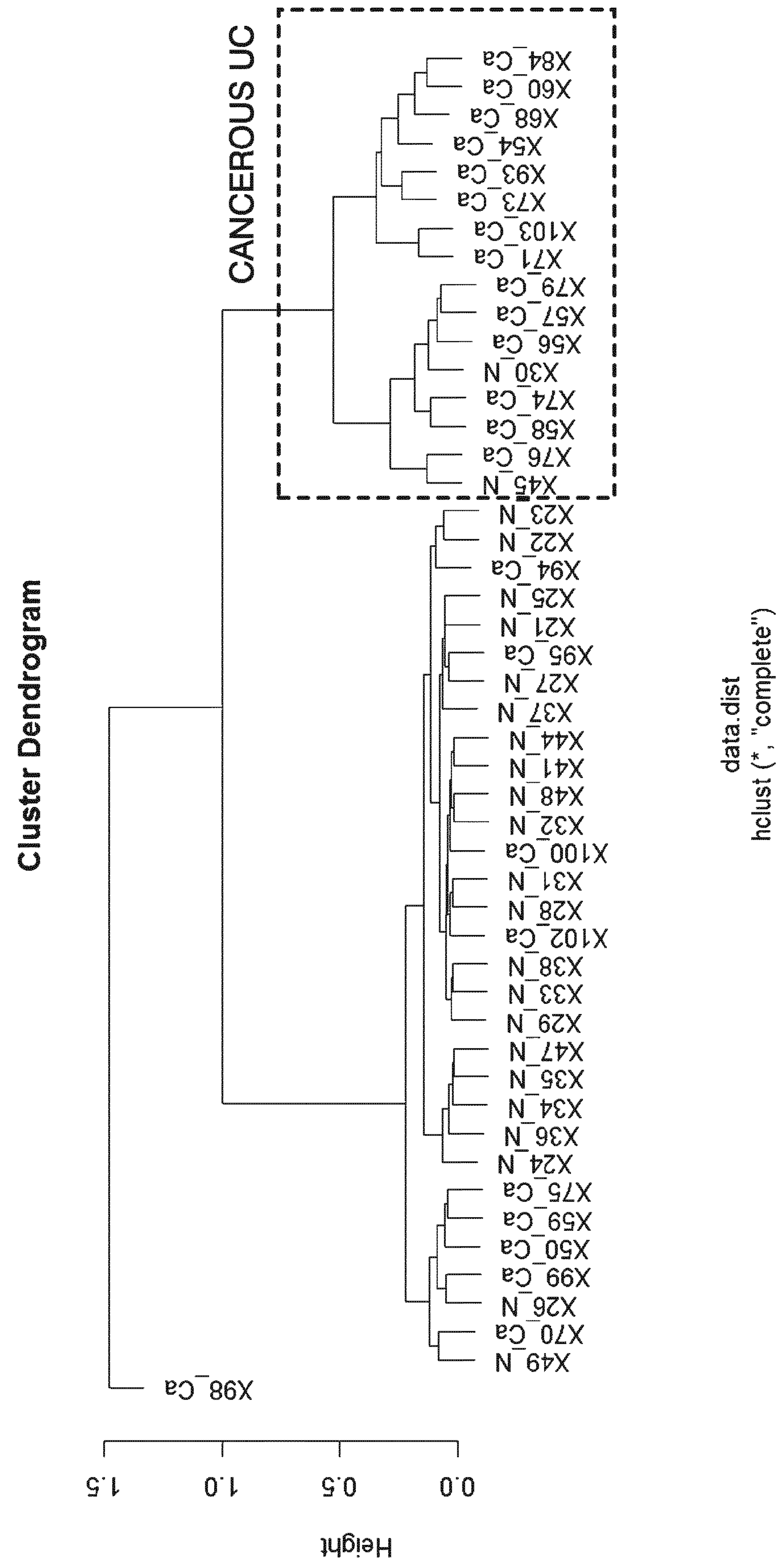
Cluster Dendrogram
CANCEROUS UC
X84_Ca
X60_Ca
X68_Ca
X54_Ca
X93_Ca
X73_Ca
X103_Ca
X71_Ca
X79_Ca
X57_Ca
X56_Ca
X30_N
X74_Ca
X58_Ca
X76_Ca
X45_N
X23_N
X22_N
X94_Ca
X25_N
X21_N
X95_Ca
X27_N
X37_N
X44_N
X41_N
X48_N
X32_N
X100_Ca
X31_N
X28_N
X102_Ca
X38_N
X33_N
X29_N
X47_N
X35_N
X34_N
X36_N
X24_N
X75_Ca
X59_Ca
X50_Ca
X99_Ca
X26_N
X70_Ca
X49_N
X98_Ca
Height
0.0
0.5
1.0
1.5
data.dist
hclust (*, "complete")

FIG. 12

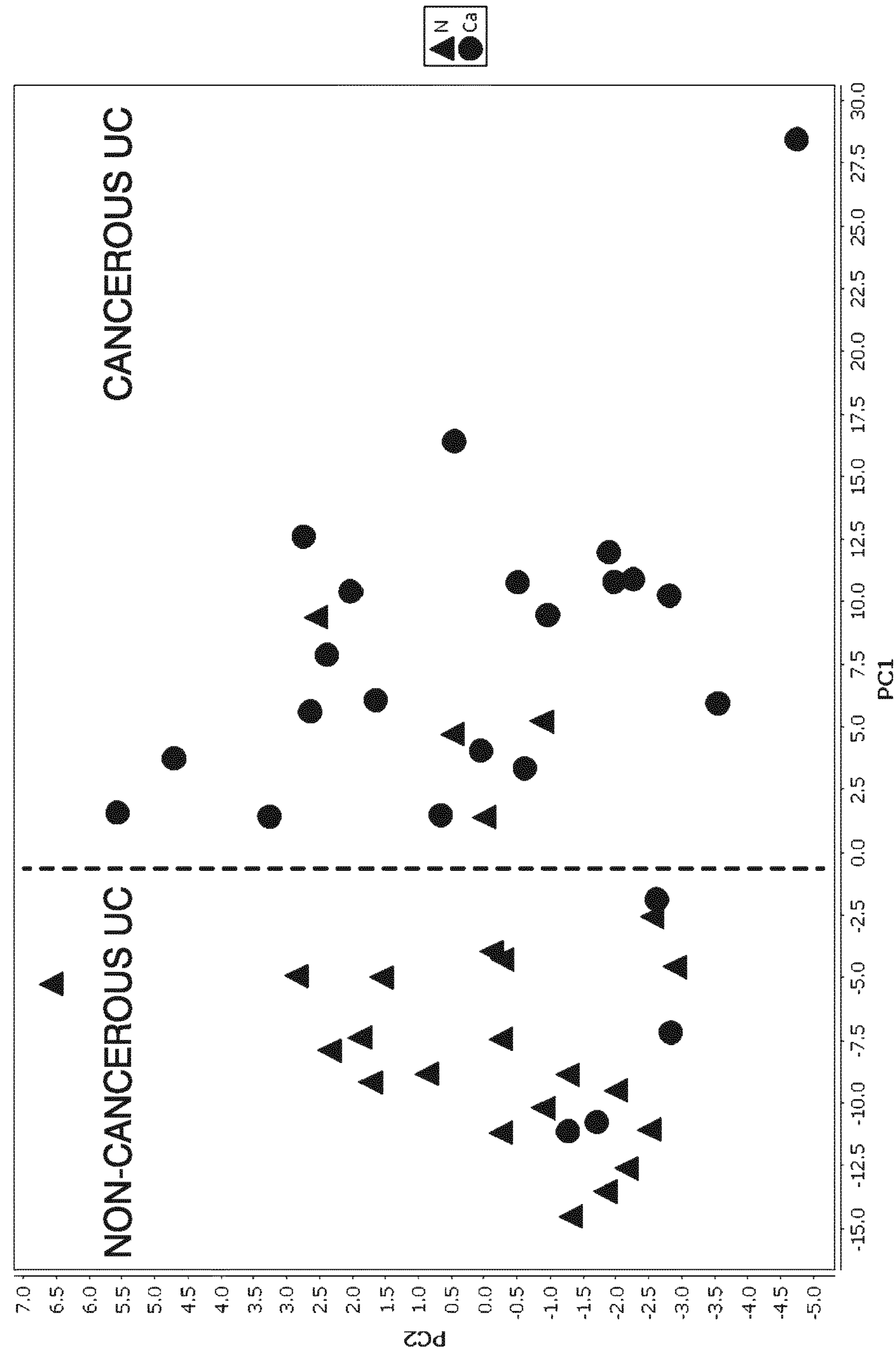

*FIG. 13*

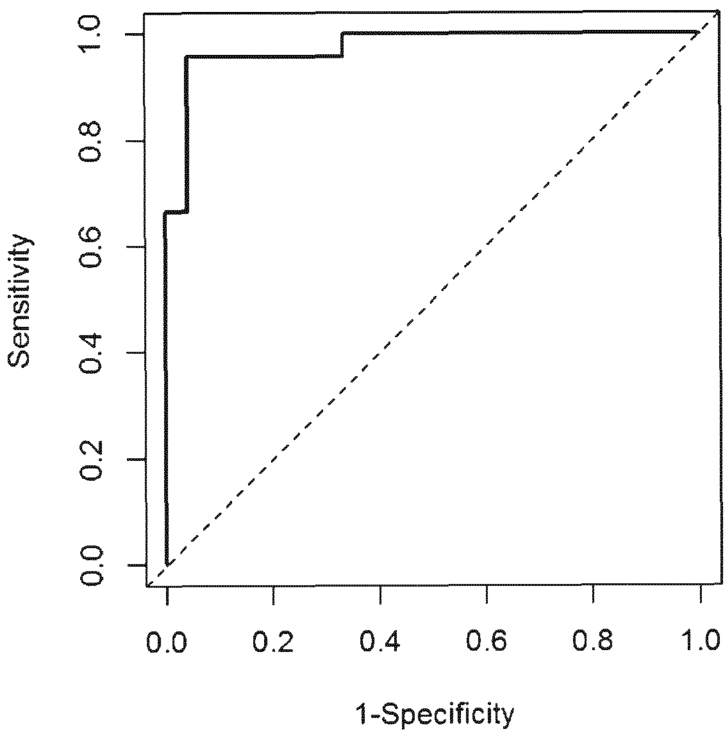

Europäisches Patentamt
European Patent Office
Office européen des brevets

# EUROPEAN SEARCH REPORT

Application Number
EP 21 15 2410

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | Anonymous: "CpG Loci Identification", , 1 January 2010 (2010-01-01), XP055655431, Retrieved from the Internet: URL:https://www.illumina.com/Documents/products/technotes/technote_cpg_loci_identification.pdf [retrieved on 2020-01-08] * the whole document * ----- | 1-9 | INV. C12Q1/6886 |
| X | WO 2014/151551 A1 (BAYLOR RES INST [US]) 25 September 2014 (2014-09-25) * claims 1-46; examples 1-4 * ----- | 1-9 | |
| X | US 2012/094289 A1 (GARRITY-PARK MEGAN [US] ET AL) 19 April 2012 (2012-04-19) * claims 1-18; example 1 * ----- | 1-9 | |
| X | K. TOMINAGA: "Prediction of Colorectal Neoplasia by Quantitative Methylation Analysis of Estrogen Receptor Gene in Nonneoplastic Epithelium from Patients with Ulcerative Colitis", CLINICAL CANCER RESEARCH, vol. 11, no. 24, 15 December 2005 (2005-12-15), pages 8880-8885, XP055655557, US ISSN: 1078-0432, DOI: 10.1158/1078-0432.CCR-05-1309 * abstract; figures 2-3 * ----- | 1-9 | TECHNICAL FIELDS SEARCHED (IPC) C12Q |
| | -/-- | | |

1 The present search report has been drawn up for all claims

| Place of search | Date of completion of the search | Examiner |
|---|---|---|
| Munich | 26 May 2021 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 2410

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| X | MARCO SCARPA ET AL: "Aberrant gene methylation in non-neoplastic mucosa as a predictive marker of ulcerative colitis-associated CRC", ONCOTARGET, vol. 7, no. 9, 4 February 2016 (2016-02-04), XP055655566, United States ISSN: 1949-2553, DOI: 10.18632/oncotarget.7188 * abstract; figures 2-4; table 1 * ----- | 1-9 | |
| X | KEI KOIZUMI ET AL: "Array-based identification of common DNA methylation alterations in ulcerative colitis", INTERNATIONAL JOURNAL OF ONCOLOGY, vol. 40, no. 4, 6 December 2011 (2011-12-06), pages 983-994, XP055655561, GR ISSN: 1019-6439, DOI: 10.3892/ijo.2011.1283 * the whole document * ----- | 1-9 | TECHNICAL FIELDS SEARCHED (IPC) |
| X | ISSA J P ET AL: "Accelerated age-related CpG island methylation in ulcerative colitis", CANCER RESEARCH, AMERICAN ASSOCIATION FOR CANCER RESEARCH, US, vol. 61, no. 9, 1 May 2001 (2001-05-01), pages 3573-3577, XP002437847, ISSN: 0008-5472 * abstract; figures 1,3 * ----- | 1-9 | |
| | -/-- | | |

The present search report has been drawn up for all claims

1

| Place of search | Date of completion of the search | Examiner |
|---|---|---|
| Munich | 26 May 2021 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 15 2410

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | J. BROCATO ET AL: "SATB1 and 2 in colorectal cancer", CARCINOGENESIS, vol. 36, no. 2, 27 December 2014 (2014-12-27), pages 186-191, XP055682587, GB ISSN: 0143-3334, DOI: 10.1093/carcin/bgu322 * the whole document * ----- | 1-9 | |
| A | MAGNUSSON KRISTINA ET AL: "SATB2 in Combination With Cytokeratin 20 Identifies Over 95% of all Colorectal Carcinomas", AMERICAN JOURNAL OF SURGICAL PATHOLOGY., vol. 35, no. 7, 1 July 2011 (2011-07-01), pages 937-948, XP055807098, US ISSN: 0147-5185, DOI: 10.1097/PAS.0b013e31821c3dae * the whole document * ----- | 1-9 | TECHNICAL FIELDS SEARCHED (IPC) |
| A | MOH MICHELLE ET AL: "SATB2 Expression Distinguishes Ovarian Metastases of Colorectal and Appendiceal Origin From Primary Ovarian Tumors of Mucinous or Endometrioid Type", AMERICAN JOURNAL OF SURGICAL PATHOLOGY., vol. 40, no. 3, 1 March 2016 (2016-03-01), pages 419-432, XP055807116, US ISSN: 0147-5185, DOI: 10.1097/PAS.0000000000000553 * the whole document * ----- | 1-9 | |

The present search report has been drawn up for all claims

1

| Place of search | Date of completion of the search | Examiner |
|---|---|---|
| Munich | 26 May 2021 | Bradbrook, Derek |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 21 15 2410

This annex lists the patent family members relating to the patent documents cited in the above-mentioned European search report. The members are as contained in the European Patent Office EDP file on The European Patent Office is in no way liable for these particulars which are merely given for the purpose of information.

26-05-2021

| Patent document cited in search report | Publication date | Patent family member(s) | Publication date |
|---|---|---|---|
| WO 2014151551 A1 | 25-09-2014 | CA 2905949 A1 | 25-09-2014 |
| | | EP 2971149 A1 | 20-01-2016 |
| | | EP 3366785 A2 | 29-08-2018 |
| | | US 2016115548 A1 | 28-04-2016 |
| | | US 2018030545 A1 | 01-02-2018 |
| | | WO 2014151551 A1 | 25-09-2014 |
| US 2012094289 A1 | 19-04-2012 | US 2012094289 A1 | 19-04-2012 |
| | | US 2018364239 A1 | 20-12-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016070330 W
- JP 2017007725 A
- WO 2014151551 A


### Non-patent literature cited in the description


- *BMC Med genomics,* 2011, vol. 4, 50
- *Sex Dev vol.,* 2011, vol. 5, 70
- *Bioinformatics,* 2014, vol. 30, 428, http://bioconductor.org/packages/release/bioc/html/ChAMP.html

SEQUENCE LISTING

<110> EA Pharma Co., Ltd.
<110> Hanumat Co., Ltd.

<120> METHOD FOR JUDGING ONSET POSSIBILITY OF LARGE BOWEL CANCER

<130> 007763626

<140> EP
<141> 2017-07-07

<150> EP 17824343.2
<151> 2017-07-07

<150> PCT/JP2017/024956
<151> 2017-07-07

<150> PCT/JP2016/070330
<151> 2016-07-08

<150> JP2017-007725
<151> 2017-01-19

<160> 80

<210> 1
<211> 122
<212> DNA
<213> Homo sapiens
<220>
<222> (61)..(62)
<223> CpG ID_cg05795005
<400> 1
ccatcagggt aagggtacct ggacttgcgg ctttttaggt cggcctggct ccgctccttc 60
cgcggtgacg aggtcccccg gcctcctagg gttgggaaga gctgctttcc tgactctcgt 120
tc 122

<210> 2
<211> 122
<212> DNA
<213> Homo sapiens
<220>
<222> (61)..(62)
<223> CpG ID_cg05208607
<400> 2
ggcttgactt ctcccacgcc ccatagaccc ggcaccgtgt aataactggg cccgtgtcct 60
cgcctgaaaa ctgggggtca cacggcctgt cctgaagaac tctgatgtga taaacaccat 120
ag 122

<210> 3
<211> 122
<212> DNA
<213> Homo sapiens
<220>
<222> (61)..(62)
<223> CpG ID_cg20795417
<400> 3
gaggccaaga cgggaggatc acttgaactc aggagttcga gaccagcctg ggtaacacag 60
cgagacactg tgtgaaaaaa atgtaaaaat taactgggtg tggtggtgtg cgcctgtagt 120
cc 122

<210> 4

<211> 122
<212> DNA
<213> Homo sapiens
<220>
<222> (61)..(62)
<223> CpG ID_cg10528424
<400> 4
gggcagcccc tgcagcactg ggcagacatg ctggcccacg cccggcggcc cattgcccag 60
cggcaccccc tgcggccagc cagggaggtg gaccgcatgc tggccctgca gccccgcctt 120
cg 122

<210> 5
<211> 122
<212> DNA
<213> Homo sapiens
<220>
<222> (61)..(62)
<223> CpG ID_cg05876883
<400> 5
caagctggaa aagggtggaa ctcatggctg ggcagacagg acagttctcc agggatctgg 60
cggtagatct gtgtctggaa cccaggttcc ctgatgtctg tgtcagggtg ccaccccaga 120
cc 122

<210> 6
<211> 122
<212> DNA
<213> Homo sapiens
<220>
<222> (61)..(62)
<223> CpG ID_cg03978067
<400> 6
gcgccggcag gagggccctg agcagacccg gcccgggggc ccggccaagg ccgcctgccc 60
cgagacccca ctcccagcac ccacagcaga gccactgggc cagggtgcct ctgccttcct 120
gg 122

<210> 7
<211> 122
<212> DNA
<213> Homo sapiens
<220>
<222> (61)..(62)
<223> CpG ID_cg10772532
<400> 7
cacatatgtc tgcctcctat catttcttca tgaggttcag ggcaaagggc ctagtcaagc 60
cgatgatctt tggttgcccc tacactttcc ccaaaccacc tacaaataaa caaaacaagg 120
gg 122

<210> 8
<211> 122
<212> DNA
<213> Homo sapiens
<220>
<222> (61)..(62)
<223> CpG ID_cg25287257
<400> 8
ctgggccgcg gggctcctac tggggcgcgg gctggtggct gggccgcggg ggcggcgagt 60
cgtcctccga ggagcagtcg gaggaggcgg cgtggacgct ggcgccgttg ctgtagggga 120
aa 122

<210> 9
<211> 122
<212> DNA
<213> Homo sapiens

<220>
<222> (61)..(62)
<223> CpG ID_cg19848924
<400> 9
ttgcgggcca gcgcgagttc cgggtgggtg ggggatgggc ggaccccgca ctcggagctg 60
cgagcaggcc ccaccggccc caggcagtga agggcttagc acctgggcca gcagctgctg 120
tg 122

<210> 10
<211> 122
<212> DNA
<213> Homo sapiens
<220>
<222> (61)..(62)
<223> CpG ID_cg05161773
<400> 10
ggctcaggag aaggggtaga acgggagggc ttcctggagg aaggcttcct aaccagagac 60
cggggtagga gtttgccagg caggtgatgc tggccagctt ctcttgccat tttccttttc 120
tt 122

<210> 11
<211> 122
<212> DNA
<213> Homo sapiens
<220>
<222> (61)..(62)
<223> CpG ID_cg07216619
<400> 11
acctttgcag cgagcgttac agctcttaaa ggtagcgtat cccgagtttt tcgttcctcc 60
cggtgggttc gtgggctggt tactctagcc gacttcagaa gtaaacccac agacctctgc 120
ag 122

<210> 12
<211> 122
<212> DNA
<213> Homo sapiens
<220>
<222> (61)..(62)
<223> CpG ID_cg11476907
<400> 12
ctggacacag ccagcttgac tctggaagaa ccgcctggca caaagcaatc aggcagtggg 60
cgttcccttt gacaggctgg ctgtctttac atagaaccta ctggaaacat cacatctgcc 120
tg 122

<210> 13
<211> 122
<212> DNA
<213> Homo sapiens
<220>
<222> (61)..(62)
<223> CpG ID_cg09084244
<400> 13
gcattttaat tcagactagc cacgtttcag cgctcagtag ccaccatagc taggggtcac 60
cgtattgaac agtgcagggc tgcagctact agcggagggc tcctgcgacg gacacaccgg 120
gt 122

<210> 14
<211> 122
<212> DNA
<213> Homo sapiens
<220>
<222> (61)..(62)
<223> CpG ID_cg00921266

<400> 14
accgacttgg gtatgtttct tatgaatatt acacgcggag cagcgtctgg tccgggggtg 60
cggtgggggg tgttggggcg ggcgggaggg gagaccaagg cggctgggga agcgcgggct 120
gg 122

<210> 15
<211> 122
<212> DNA
<213> Homo sapiens
<220>
<222> (61)..(62)
<223> CpG ID_cg01493009
<400> 15
agaaaacaga agagacttgt gtgtgtgtta cacatatgta cgtatacaca cacgtgcgtt 60
cgcaagcatg cctaaggaga tttctttcaa aaagaaggct ggcccaacaa tttcagtggc 120
ca 122

<210> 16
<211> 122
<212> DNA
<213> Homo sapiens
<220>
<222> (61)..(62)
<223> CpG ID_cg08101036
<400> 16
ctagtggcac cgacttgggt atgtttctta tgaatattac acgcggagca gcgtctggtc 60
cgggggtgcg gtgggggtg ttggggcggg cgggagggga gaccaaggcg gctggggaag 120
cg 122

<210> 17
<211> 122
<212> DNA
<213> Homo sapiens
<220>
<222> (61)..(62)
<223> CpG ID_cg20106077
<400> 17
gaatcccatg agtgatggcc aattcaggag gcgaagcacc cagcaagttc cccaccacag 60
cggacatgga acacgcacga gaggcagaga catgaaggac agaaggatgg aaggaagtac 120
gg 122

<210> 18
<211> 122
<212> DNA
<213> Homo sapiens
<220>
<222> (61)..(62)
<223> CpG ID_cg12908908
<400> 18
gggttgagaa ccactgattt agacattgct gtcccaatta atatttaaat agtcacagcc 60
cgttagctcc actaatccag ttgcattacc accggcatac aaaagattat tttttaaata 120
cc 122

<210> 19
<211> 122
<212> DNA
<213> Homo sapiens
<220>
<222> (61)..(62)
<223> CpG ID_cg04515524
<400> 19
cacttagatg ctcagtaaat gctccaggaa actgcagcac aaggaataat gaacttggag 60
cggggaagag ctggctttgt cccgggagag ctcgggcaag aggcctcgca tgtctgtgcc 120

tc 122

<210> 20
<211> 122
<212> DNA
<213> Homo sapiens
<220>
<222> (61)..(62)
<223> CpG ID_cg05380919
<400> 20
aatgcagtga ttaaaggaca caaggcctca gtgtgcatca ttctcattgt ggctttcagg 60
cggctgtgga agacagggtg gggatggtgg cttcgggagg tgaggtgctc tgggacttgg 120
gc 122

<210> 21
<211> 122
<212> DNA
<213> Homo sapiens
<220>
<222> (61)..(62)
<223> CpG ID_cg15360451
<400> 21
agggaccttc cttggacact cggctccctg ggcctgacgg tggactcatc ctttacaagg 60
cggctggaga cgacctgatt cttccatccc tttcccctgt gtgcaggttt tactgggctg 120
cg 122

<210> 22
<211> 122
<212> DNA
<213> Homo sapiens
<220>
<222> (61)..(62)
<223> CpG ID_cg19775763
<400> 22
gtcagtgggc tggggtgtga tctgtgggcg ggctggggtg cctgtgcagt gatctgtcgg 60
cgggccgggg tgtgatctgt gggcgggctg gggtgcctgt gcagtgatct gtccgcaggc 120
tg 122

<210> 23
<211> 122
<212> DNA
<213> Homo sapiens
<220>
<222> (61)..(62)
<223> CpG ID_cg01871025
<400> 23
ccaggatgcg ttgtcaccat aagttacagt acaagttggt tccctctctt ctctctcccc 60
cgcacctcga ccttctgccc tgtctcagac acacacacac acacacacac acacacacac 120
ac 122

<210> 24
<211> 122
<212> DNA
<213> Homo sapiens
<220>
<222> (61)..(62)
<223> CpG ID_cg05008296
<400> 24
attgggtttt ataactttat aaaagccttt catttgtttt gttccttatt cagtcattca 60
cgcatttgac aaacatttat ggcattccta tagtgtacta ggcactgtgc tgatgtccag 120
ca 122

<210> 25

<211> 122
<212> DNA
<213> Homo sapiens
<220>
<222> (61)..(62)
<223> CpG ID_cg08708231
<400> 25
gcttagattt ctcacattcc agcacatgca catggtctga cagtggttct tcatgaggag 60
cggaggtggg gagcatggag agtgtgtgag agccacctgg gcaccttttt gtcaaaatat 120
ac 122

<210> 26
<211> 122
<212> DNA
<213> Homo sapiens
<220>
<222> (61)..(62)
<223> CpG ID_cg27024127
<400> 26
tcactcattc attcatccag agacaggcac agacaggctg tgacacagga gctggcaatg 60
cggtctccac gtggccggaa ctgagcggct atctggaata aagggaggga ttgcagcggc 120
tg 122

<210> 27
<211> 122
<212> DNA
<213> Homo sapiens
<220>
<222> (61)..(62)
<223> CpG ID_cg22274196
<400> 27
gcaatataca aattaaagga tgggggtttt ttcccattca ttcaataaat cgttagtgaa 60
cgccttctgg atacatgaca gctaggccag ggaatgagcc tgcaaagacg aggaagatgt 120
ct 122

<210> 28
<211> 122
<212> DNA
<213> Homo sapiens
<220>
<222> (61)..(62)
<223> CpG ID_cg11844537
<400> 28
gagacgagct agtaatggag ggtgggccgt ggggtgagga aggtgcccaa atttgccgag 60
cggtaacctt accaaggact gggaagcagg gttttcacct actgaccccc gtccctcctc 120
gg 122

<210> 29
<211> 122
<212> DNA
<213> Homo sapiens
<220>
<222> (61)..(62)
<223> CpG ID_cg09908042
<400> 29
gggagagttc ttccaggata tgtctggctg tggactagca agtccagcct caccgtgtat 60
cgccaaattg ctctccaaac gataccaatc tccaccagca gcatctgaaa gttcccattg 120
ct 122

<210> 30
<211> 122
<212> DNA
<213> Homo sapiens

<220>
<222> (61)..(62)
<223> CpG ID_cg15828613
<400> 30
accaaagaaa atagttgcag cttaatgcct cacttgggag tttgcaaagt ctctgctctc 60
cgaaggcctt ggtgggtgaa aagcctaaat cgtccttatt tcccaccttg cttctctcct 120
tc 122

<210> 31
<211> 122
<212> DNA
<213> Homo sapiens
<220>
<222> (61)..(62)
<223> CpG ID_cg06461588
<400> 31
tggtggttga tagtgttgtt cagaacatcg atgtttttcc tgatttttgg tctgttctgt 60
cgatttctga gaaagtatta aaattaaagt tgggtcttgc atttttatcc attctgtcag 120
tc 122

<210> 32
<211> 122
<212> DNA
<213> Homo sapiens
<220>
<222> (61)..(62)
<223> CpG ID_cg08299859
<400> 32
agggactacc tttctgcgta ttcctttctg ttctttaaaa atgttaaacc atggggtgct 60
cgcttcggca gcacatatac taaaattgga acgatacaga gaagattagc atggcccctg 120
cg 122

<210> 33
<211> 122
<212> DNA
<213> Homo sapiens
<220>
<222> (61)..(62)
<223> CpG ID_cg24887265
<400> 33
cccaggggc ggcgggctga ggagcagtgc ggggctggat gatgagtggt ctggcgtccc 60
cgatggagtc ttctcatcct ccgagctgcc taacaccgac ttgccgctgc cattcagcgg 120
gt 122

<210> 34
<211> 122
<212> DNA
<213> Homo sapiens
<220>
<222> (61)..(62)
<223> CpG ID_cg10931190
<400> 34
gggctgaggc cccaggtgac agacgttttc cagtctacgc tgcgcggggc taagcctctg 60
cgaggcagag cgcactaaag cgtgcgccgc ctccgggaga gctgagctca ggacagcatc 120
gt 122

<210> 35
<211> 122
<212> DNA
<213> Homo sapiens
<220>
<222> (61)..(62)
<223> CpG ID_cg22797031

<400> 35
taacacgaat gacaagtggg tgattttcaa gaagcgcccg gtccctctag agaatgcgtc 60
cgaatatcag cggagccgac tgcgtatgcc tccggatgcc catctataaa ctctcttgct 120
tg 122

<210> 36
<211> 122
<212> DNA
<213> Homo sapiens
<220>
<222> (61)..(62)
<223> CpG ID_cg22158650
<400> 36
cgaggaacag cgagcccccg gacgctgact gcaggacgtc ccagtttgtg cccgggtctc 60
cgtccctccc cgtacggggc tcgtaccccc gggcctgggt ctgacccaca gggcgctgag 120
gc 122

<210> 37
<211> 122
<212> DNA
<213> Homo sapiens
<220>
<222> (61)..(62)
<223> CpG ID_cg13677149
<400> 37
ggcggcagtg gtgggggcgg ctcgcaaggc accctggcgt gcagcgccag tgaccagatg 60
cgtcgttacc gcaccgcctt cacccgagag cagattgcgc ggctggagaa ggaattctac 120
cg 122

<210> 38
<211> 122
<212> DNA
<213> Homo sapiens
<220>
<222> (61)..(62)
<223> CpG ID_cg22795586
<400> 38
gccttagcgc tctggtgacc tccgcgggat tctgagaaaa gcactgcgga acggcgggag 60
cgggccctgc tgcttgcttc gcgcccccca cccgcccggg gaccgcgact aagtccccga 120
cg 122

<210> 39
<211> 122
<212> DNA
<213> Homo sapiens
<220>
<222> (61)..(62)
<223> CpG ID_cg04389897
<400> 39
agcagtagca gcagcaggaa gggttgctga tcccggagct gtcacccgcc ggagggtggg 60
cgcgcggggg gctggtgagg cgtgggaggg gcggggcggg aggagagcct cactttctgt 120
gc 122

<210> 40
<211> 122
<212> DNA
<213> Homo sapiens
<220>
<222> (61)..(62)
<223> CpG ID_cg27651243
<400> 40
ctcagaccgc ccgtgggtca caagtgcaaa ggtaacagtg tcccctggga ggccgggatg 60
cgtcgggggc ggggagggcg cgcacctggg tctcggtgag catgagcgag gtggccacct 120

cg 122

<210> 41
<211> 122
<212> DNA
<213> Homo sapiens
<220>
<222> (61)..(62)
<223> CpG ID_cg09765089
<400> 41
ctcgcgcagg cagcgggcgc gtgtggcccg ggctgggcaa gccgaggaac agcgagcccc 60
cggacgctga ctgcaggacg tcccagtttg tgcccgggtc tccgtccctc cccgtacggg 120
gc 122

<210> 42
<211> 122
<212> DNA
<213> Homo sapiens
<220>
<222> (61)..(62)
<223> CpG ID_cg17542408
<400> 42
gcccgcggag ccacgtcagg cccccagctc ccccggatcc caccacgcac caggcccctc 60
cgcccggcaa gtggcccaag caggcatccg caacggaagg acaattttaa aaacaaaccc 120
tc 122

<210> 43
<211> 122
<212> DNA
<213> Homo sapiens
<220>
<222> (61)..(62)
<223> CpG ID_cg21229570
<400> 43
cctctcccac accaacctcc agcgcgcgaa gcagagaacg agaggaaagt ttgcggggtt 60
cgaatcgaaa atgtcgacat cttgctaatg gtctgcaaac ttccgccaat tatgactgac 120
ct 122

<210> 44
<211> 122
<212> DNA
<213> Homo sapiens
<220>
<222> (61)..(62)
<223> CpG ID_cg14394550
<400> 44
gagcggtgtc ttgctaggcc ggttggggta cttgcggggc cggatgggct tgagggtgag 60
cggcggctgg ggcaggctgc caaagcccgg gtggatctgc ttgtctttga atgccttgat 120
gg 122

<210> 45
<211> 122
<212> DNA
<213> Homo sapiens
<220>
<222> (61)..(62)
<223> CpG ID_cg20326647
<400> 45
agggagttta tagggactcc acggcgcggt ggctcgcctg ggctgagagg ctgactaacg 60
cgctgacacg gcggcacggg gctttacagg ccacgggccc tgccggcgag actgggaggg 120
ag 122

<210> 46

<211> 122
<212> DNA
<213> Homo sapiens
<220>
<222> (61)..(62)
<223> CpG ID_cg20373036
<400> 46
caccgcacac taaccacccc aacgcctggg gggccagccc ggcaccgaac ccgtctatca 60
cgtcaagcgg ccaacccctc aacgtgtact cgcagcctgg cttcaccgtg agcggcatgc 120
tg 122

<210> 47
<211> 122
<212> DNA
<213> Homo sapiens
<220>
<222> (61)..(62)
<223> CpG ID_cg19968840
<400> 47
ctcacagcgg gtccccccac tccccggcag ggtggcgttc tgcttctggc tcctctccaa 60
cgtgctgctc tccacgccgg ccccgctcta cggaggcctg gcactgctga ccaccggagc 120
ct 122

<210> 48
<211> 122
<212> DNA
<213> Homo sapiens
<220>
<222> (61)..(62)
<223> CpG ID_cg12162138
<400> 48
ccgcggggca agagcggggc tgcctgagcc cgcggagcca cgtcaggccc ccagctcccc 60
cggatcccac cacgcaccag gcccctccgc ccggcaagtg gcccaagcag gcatccgcaa 120
cg 122

<210> 49
<211> 122
<212> DNA
<213> Homo sapiens
<220>
<222> (61)..(62)
<223> CpG ID_cg01307130
<400> 49
gagggtcttt cctgcccggg tttcggacac tgttggagtt tcagggagct tgggcgcagg 60
cggcgatctc aaagcgcagc aggctccgca gaagaggcgg gctccgggca gagaccgcta 120
gc 122

<210> 50
<211> 122
<212> DNA
<213> Homo sapiens
<220>
<222> (61)..(62)
<223> CpG ID_cg24960947
<400> 50
cccgcccgcc tctcggcccc catcccggtc tggtccactc ccacccctcc aaccccatgc 60
cggccactgc agtactcaca ccgcacgcct gggctctgcc tctggcccgg gttgggggcg 120
gc 122

<210> 51
<211> 122
<212> DNA
<213> Homo sapiens

<220>
<222> (61)..(62)
<223> CpG ID_cg26074603
<400> 51
gtggttctgc tttggtttcc gagtggacga ggttctctgg gcagcgggac tgagtcttgg 60
cgcccaggtg agccgccctt ctccgacgag aaactacttg ttggcgtttt ccggattcag 120
gt 122

<210> 52
<211> 122
<212> DNA
<213> Homo sapiens
<220>
<222> (61)..(62)
<223> CpG ID_cg05575614
<400> 52
gacgaattcc ctttttccct ctacagcaat ccctcagatt tctgggggaa aatggggccc 60
cgttttccag tacacaggcc accccaggaa gaccgcgtcg ggcgctgtgt gatctggaga 120
gt 122

<210> 53
<211> 122
<212> DNA
<213> Homo sapiens
<220>
<222> (61)..(62)
<223> CpG ID_cg08309529
<400> 53
cagaatggcg gctccagagg cggtttcaag tttcataagt caggtaacac tgtgggtttc 60
cgccttctcg gacgcgggga aaggggagac aggaggcttc cccttgcgcg gggtgggtcg 120
gt 122

<210> 54
<211> 122
<212> DNA
<213> Homo sapiens
<220>
<222> (61)..(62)
<223> CpG ID_cg24879782
<400> 54
cagcctagaa gaagggtccc ctcagtagag accaggcctc cagctctccg tccggcgctc 60
cgctccacaa cccgccagtc gatgtgaggt ccgtcaaggg agcgatccct ccgtctgccc 120
gg 122

<210> 55
<211> 122
<212> DNA
<213> Homo sapiens
<220>
<222> (61)..(62)
<223> CpG ID_cg17538572
<400> 55
gggctgcgaa ccccaactgg cgggcgacgg ggactccgag cagcagcttg tggaggcctt 60
cgaggaaatg acccgcaatc tcttctcgct gcccatcgac gtgcccttca gcgggctgta 120
cc 122

<210> 56
<211> 122
<212> DNA
<213> Homo sapiens
<220>
<222> (61)..(62)
<223> CpG ID_cg14516100

<400> 56
gagctcaccc gggtgggaga cagagccggg gcgcgcgagc ttggtgtggg ggcgccactc 60
cggggcggag gggaggggct accagtgact tctccgagtc gggagctaga aagaggcttc 120
cg 122

<210> 57
<211> 122
<212> DNA
<213> Homo sapiens
<220>
<222> (61)..(62)
<223> CpG ID_cg25740565
<400> 57
tctttacccc cgactccctg gagcttggtc tcgggatgcc aacttggggc acggaggcga 60
cgggctgctc cgaagctgga gggtttctgc ttgggtcaga gggatcacga cctcagcaga 120
gc 122

<210> 58
<211> 122
<212> DNA
<213> Homo sapiens
<220>
<222> (61)..(62)
<223> CpG ID_cg21045464
<400> 58
gctgatcgat gaaggagaca agctggccca cggggaggtc aatacaatcg atgcggacct 60
cgacgaaacg gaagaatctc gcaggttcct gcgtgctggg ttccactcaa aatgtttcag 120
ga 122

<210> 59
<211> 122
<212> DNA
<213> Homo sapiens
<220>
<222> (61)..(62)
<223> CpG ID_cg23955842
<400> 59
gtggcagcga cggcggcggc agcggagatc ccaaggtccg taagcgggga actgggggt 60
cgcagggcgg gccggccaag aggcttggga gctgggcgtt gctgggggtg gagggataga 120
ag 122

<210> 60
<211> 122
<212> DNA
<213> Homo sapiens
<220>
<222> (61)..(62)
<223> CpG ID_cg22964918
<400> 60
cagagggagg aggtgcccct cactagataa ggggccgccg gctggctgcc ggctccatga 60
cgcccgtggg gtcacccccc ggccccggga ctcagccagc ctcgctcctc gctcctcgct 120
cc 122

<210> 61
<211> 122
<212> DNA
<213> Homo sapiens
<220>
<222> (61)..(62)
<223> CpG ID_cg00061551
<400> 61
aaacctcttt cttatgtaaa gtgctcagtc tcgggtatgt ctttatcagc agcatgaaaa 60
cggactaata caggccatcg cagagacaca cattaaactc tcactatggc tactttggga 120

gg 122

<210> 62
<211> 122
<212> DNA
<213> Homo sapiens
<220>
<222> (61)..(62)
<223> CpG ID_cg04610028
<400> 62
cttgccccag ctgggacagc cctgctctga ggaccagaca caggcaggtg ttgtgctatc 60
cgcagtggct gtttctggaa ggcaggagcc tgccttcact tctgcaccac ttagcacagt 120
gc 122

<210> 63
<211> 122
<212> DNA
<213> Homo sapiens
<220>
<222> (61)..(62)
<223> CpG ID_cg20139683
<400> 63
cagcaggggg agccgggatg tggctcacat gcctggggct gctccgtggc catctggatg 60
cgtgcacacg gcagcagggg cagccgggat gtggcttacg tgcctggggc tgctccgtgg 120
cc 122

<210> 64
<211> 122
<212> DNA
<213> Homo sapiens
<220>
<222> (61)..(62)
<223> CpG ID_cg09549987
<400> 64
gtgagcatgg gtgattgggt gggggagttg ggagggtgc tagtgttccg tgtgtgtgca 60
cgtttgtgca catgcgttgt atgcacctat gtgtagagag agaaggtgaa tgaagtgtaa 120
ga 122

<210> 65
<211> 122
<212> DNA
<213> Homo sapiens
<220>
<222> (61)..(62)
<223> CpG ID_cg02299007
<400> 65
acccgtcccg ttcgacgcct ctggccgccc cgtccttgct tctcatctca cagggcactg 60
cgagccgcct gtcgcaatca gcattgagag ccaaaacagc tgtttggtga ctgtgcgagg 120
tt 122

<210> 66
<211> 122
<212> DNA
<213> Homo sapiens
<220>
<222> (61)..(62)
<223> CpG ID_cg17917970
<400> 66
accctgcacc cccaaagtcc tgacaacgca caccccacga agccggcgca cgcgccccta 60
cgacacccat tcggtgctgc tccgcacacc cccgcacgcc gcccgtgcac ctcccgtgtc 120
tc 122

<210> 67

<211> 122
<212> DNA
<213> Homo sapiens
<220>
<222> (61)..(62)
<223> CpG ID_cg10339295
<400> 67
ccccaagcct tgccagatta cattgtcaag gccagcactt tggagatatt tccttggttt 60
cgcaattcac acagtgacta acacatgtta cattttgaaa acttctctgg gtaaaaattt 120
aa 122

<210> 68
<211> 122
<212> DNA
<213> Homo sapiens
<220>
<222> (61)..(62)
<223> CpG ID_cg01736784
<400> 68
taaagcgcgg cggggagtcc ggggggctcc cgcctggagg gctgtgtgag cggcgggccg 60
cggggcggcg cggggggcgc tctccactct gcggaagctg ccccctctgc cctccggtcc 120
gc 122

<210> 69
<211> 122
<212> DNA
<213> Homo sapiens
<220>
<222> (61)..(62)
<223> CpG ID_cg00723994
<400> 69
gcctctgccc gagcgcgccc ttcggcccct gcaattagcg ccgggaggtc agcaggaacc 60
cggacgcctt cacccgcggc tcaaagcaca gcaaaaggcg accccatccc ctcccctccg 120
cg 122

<210> 70
<211> 122
<212> DNA
<213> Homo sapiens
<220>
<222> (61)..(62)
<223> CpG ID_cg26315862
<400> 70
gaaatccccc gcagttagcg gtcaacagaa agggcgacac ggaacggggt tcctggcacc 60
cgagctcgcc gcaccgaagt ctcctggtaa cagcgacacg ggaccgggct atgtgaccac 120
ac 122

<210> 71
<211> 122
<212> DNA
<213> Homo sapiens
<220>
<222> (61)..(62)
<223> CpG ID_cg19937061
<400> 71
cgcgcccgca gggcccgccc accgctttgc ttacgccgct gcccgtgggc caccccggcg 60
cgcagggtcc ccagcccgcg cctccgccac agccggcttt cccgcgcagc cacggactgc 120
ac 122

<210> 72
<211> 122
<212> DNA
<213> Homo sapiens

<220>
<222> (61)..(62)
<223> CpG ID_cg04004787
<400> 72
aaaaggacca gcgggatccg gccgcaagaa ttggaaagcc taggaagtgg cggtggctgg 60
cgcgtttggg gagcaggagt ggggataggg aagcagagct tgagagacct tcctccgggg 120
ca 122

<210> 73
<211> 122
<212> DNA
<213> Homo sapiens
<220>
<222> (61)..(62)
<223> CpG ID_cg03409187
<400> 73
gcgacggaga cactaccgag aaccagatgt tcgccgcccg cgtggtcatc ctgctgctgc 60
cgtttgccgt catcctggcc tcctacggtg ccgtggcccg agctgtctgt tgcatgcggt 120
tc 122

<210> 74
<211> 122
<212> DNA
<213> Homo sapiens
<220>
<222> (61)..(62)
<223> CpG ID_cg00282249
<400> 74
ttccaggagc cccccgtata aggaccccag ggactcctct ccccacgcgg ccgggccgcc 60
cgcccggccc ccagcccgga gagctgccac cgacccccctc aacgtcccaa gccccagctc 120
tg 122

<210> 75
<211> 122
<212> DNA
<213> Homo sapiens
<220>
<222> (61)..(62)
<223> CpG ID_cg20148575
<400> 75
ggggccacca ggtgggccgg gggcgcggtg gaagcggatg gtctgggtcg acgggagaag 60
cgaagcgggc gcgggaggcg ggcgcgggag gcgggcgcgg gaggcgggcg cgggaggcgg 120
gc 122

<210> 76
<211> 122
<212> DNA
<213> Homo sapiens
<220>
<222> (61)..(62)
<223> CpG ID_cg14416371
<400> 76
gagacacgag tccaggggcg cggaggggcg ggcagcgcgc ggagtggtga gactgagccg 60
cgatggaacg cgctggggag acccagcctg ttcggctcca gggttcggag acatcctggg 120
ct 122

<210> 77
<211> 122
<212> DNA
<213> Homo sapiens
<220>
<222> (61)..(62)

```
<223> CpG ID_cg26081900
<400> 77
gcacacacac acacacgtga atatatatat atatatatat atatatatat atatgaaatc        60
cggatggatc aagatgttta tagaaatgca aagctttaaa tctgtggaag aaatgagaga       120
aa                                                                      122

<210> 78
<211> 122
<212> DNA
<213> Homo sapiens
<220>
<222> (61)..(62)
<223> CpG ID_cg10168149
<400> 78
cggagtgcgc attgcgctaa cacgcgcacg ggaattgcac ccttgccgga gcctccgcac        60
cgtgcgccct tcaaagagct ggcgaccccg ctcacgtgta agcaacctcc cactttgaaa       120
ct                                                                      122

<210> 79
<211> 122
<212> DNA
<213> Homo sapiens
<220>
<222> (61)..(62)
<223> CpG ID_cg25366315
<400> 79
ctggttctgg gccttcccag acaaaagcca gagacccgga gcctctttct gagaaggaac        60
cgggcgtccc caagatttcc tctagccgag tcccctgggt cccccgagga ccgggacagc       120
tc                                                                      122

<210> 80
<211> 122
<212> DNA
<213> Homo sapiens
<220>
<222> (61)..(62)
<223> CpG ID_cg19850149
<400> 80
cggcgcgctc tgccagggac cccccccccc caccgccggt gcccgagtgg gccgcgtagg        60
cggggcccag cccataggcc gccagctcca gccgctgcag cgttctacgc ggtccgggac       120
gc                                                                      122
```